(19) Europäisches Patentamt / European Patent Office / Office européen des brevets

(11) **EP 1 773 979 B1**

(12) **EUROPEAN PATENT SPECIFICATION**

(45) Date of publication and mention
of the grant of the patent:
**29.06.2016 Bulletin 2016/26**

(51) Int Cl.:
*C12M 1/34* (2006.01)     *H01M 4/50* (2006.01)
*G01N 27/02* (2006.01)

(21) Application number: **05786773.1**

(22) Date of filing: **04.08.2005**

(86) International application number:
**PCT/US2005/027943**

(87) International publication number:
**WO 2006/017762 (16.02.2006 Gazette 2006/07)**

(54) **DYNAMIC MONITORING OF ACTIVATION OF G-PROTEIN (GPCR) IN LIVING CELLS USING REAL-TIME MICROELECTRONIC CELL SENSING TECHNOLOGY**

DYNAMISCHE ÜBERWACHUNG DER AKTIVIERUNG VON G-PROTEIN (GPCR) IN LEBENDEN ZELLEN UNTER VERWENDUNG VON MIKROELEKTRONISCHER-ECHTZEIT-ZELLMESSTECHNOLOGIE

CONTROLE DYNAMIQUE DE L'ACTIVATION DE LA PROTEINE G (GPCR) DANS DES CELLULES VIVANTES METTANT EN OEUVRE LA TECHNOLOGIE DE DETECTION MICRO-ELECTRONIQUE DE CELLULES EN TEMPS REEL

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.08.2004 US 598609 P**
**04.08.2004 US 598608 P**
**27.09.2004 US 613872 P**
**12.11.2004 US 987732**
**22.11.2004 US 630071 P**
**26.01.2005 US 647189 P**
**26.01.2005 US 647075 P**
**09.02.2005 US 55639**
**10.03.2005 US 660829 P**
**10.03.2005 US 660898 P**
**10.06.2005 US 689422 P**

(43) Date of publication of application:
**18.04.2007 Bulletin 2007/16**

(73) Proprietor: **Acea Biosciences, Inc.**
**San Diego, CA 92121 (US)**

(72) Inventors:
• **Abassi, Yama A.**
**San Diego, CA 92121 (US)**
• **Yu, Naichen**
**San Diego, CA 92121 (US)**
• **Atienza, Josephine**
**San Diego, CA 92121 (US)**
• **Xu, Xiao**
**San Diego, CA 92121 (US)**
• **Wang, Xiaobo**
**San Diego, CA 92121 (US)**

(74) Representative: **Novagraaf Technologies**
**Bâtiment O2**
**2, rue Sarah Bernhardt**
**CS90017**
**92665 Asnières-sur-Seine Cedex (FR)**

(56) References cited:
**WO-A1-2005/005979     WO-A2-00/70343**
**WO-A2-2004/010103     US-B2- 6 596 499**

• **JOACHIM WEGENER ET AL: "Use of electrochemical impedance measurements to monitor Beta-adrenergic stimulation of bovine aortic endothelial cells", PFLÜGERS ARCHIV-EUR. J. PHYSIOL., vol. 437, no. 6, 22 April 1999 (1999-04-22), pages 925-934, XP055026304, ISSN: 0031-6768, DOI: 10.1007/s004240050864**

EP 1 773 979 B1

**Description**

**TECHNICAL FIELD**

This invention relates to the field of cell-based assays. In particular, the invention provides cell-based assays using impedance monitoring devices for identifying a compound capable of interacting with a G-Protein Coupled Receptor (GPCR).

**BACKGROUND OF THE INVENTION**

Bioelectronics is a progressing interdisciplinary research field that involves the integration of biomatereials with electronic devices. Bioelectronic methods have been used for analyzing cells and assaying biological molecules and cells. In one type of application, cells are cultured on microelectrodes and cell-electrode impedance is measured and determined to monitor cellular changes.

In WO 2004/010103, a device for detecting cells and/or molecules on an electrode surface is disclosed. The device detects cells and/or molecules through measurement of impedance changes resulting from the attachment or binding of cells and/or molecules to the electrode surfaces. A number of embodiments of the device is disclosed, together with the apparatuses, system for using such devices to perform certain cell based assays.

In WO 2004/010102, devices, systems and methods for assaying cells using cell-substrate impedance monitoring are disclosed. The cell-substrate monitoring devices comprise electrode arrays on a nonconducting substrate, in which each of the arrays has an approximately uniform electrode resistance across the entire array. The cell-substrate monitoring systems comprise one or more cell-substrate devices comprising multiple wells each having an electrode array, an impedance analyzer, a device station that connects arrays of individual wells to the impedance analyzer, and software for controlling the device station and impedance analyzer. The cellular assays use impedance monitoring to detect changes in cell behavior or state.

In WO 2005/077104, devices, systems and methods for assaying cells using cell-substrate impedance monitoring are disclosed. The disclosed cellular assays use impedance monitoring to detect changes in cell behavior or state. The methods can be used to test the effects of compounds on cells, such as in cytotoxicity assays. Methods of cytotoxicity profiling of compounds were also provided.

Eukaryotic cells from unicellular and multicellular organisms have devised evolutionarily conserved mechanisms for responding to environmental cues by utilizing specific cell surface receptors. These membrane-bound receptors recognize and bind to their cognate ligand and turn on biochemical cascades inside the cell that culminate in a specific cellular response. Members of the G-protein coupled receptor (GPCR) family are one of the main classes of cell surface receptors that participate in a variety of cellular pathways that result in different cellular responses such as proliferation, chemotaxis, and cytoskeletal dynamics. GPCRs have the ability to recognize a wide range of extracellular molecules including amino acids, hormones, growth factors, light, peptide and non-peptide neurotransmitters and a number of odorant molecules. Because GPCRs and their cognate ligands participate in important physiological and pathophysiological settings, including but not limited to inflammation, hypertension, diabetes and autoimmunity they are extremely attractive targets for the pharmaceutical industry. More than 50% of the current therapeutic agents on the market are targeted at GPCRs and as more GPCRs and their ligands are being discovered this number will likely rise even further in the coming years. Therefore, GPCRs occupy a unique position as a pharmaceutical target because it far exceeds any other pharmaceutically relevant target.

As the name implies, GPCRs are associated with a heterotrimeric guanine nucleotide binding-protein complex (G-proteins). Ligand binding to the GPCR induces a conformational change which promotes exchange of GTP for GDP on the G-protein $\alpha$-subunit which allows for dissociation of the G-protein $\alpha$-subunit from G$\beta\gamma$-subunits. Subsequently, the activated G$\alpha$ subunit and G$\beta\gamma$ subunit positively and/or negatively impact the activity of effector enzymes and proteins. Furthermore, in recent years it has come to light that there is a feedback mechanism in place which negatively regulates or modulates GPCR-mediated signaling by a variety of mechanisms such as post-translational modification, protein-protein interaction or receptor endocytosis. Thus, the activity of GPCR is determined by the fine balance of receptor desensitization and resensitization which is dictated by the ligand concentration and numerous other inputs that the cell receives simultaneously.

The human genome project has identified a number of proteins which can be categorized into GPCRs based on sequence. The number of GPCRs encoded by the human genome is estimated to be between 800-1000 and thus far approximately 650 GPCR have been identified from the effort of the human genome project, 200 of which are classified as known GPCR because the activating ligands for these receptors are known (Nambi, P and Aiyar N, G-protein coupled receptors in drug discovery, in Assay and Drug Development Technologies (2003) 1, 305-310). The remaining receptors for which the ligands are not known are considered "orphan receptors" and they are the subject of intense scrutiny as potential medically relevant targets. There are a number of *in vitro* and cell-based assays available which are used to screen for potential agonist or antagonist of GPCRs. The *in vitro* assays are based on binding studies with labeled ligand and receptor. (Nambi, P and Aiyar N, G-protein coupled receptors in drug discovery, in Assay and Drug Development Technologies (2003) 1, 305-310) The cell-based assays are based on engineering cell lines to express exogenous GPCRs alone or together with a reporter plasmid. Calcium sensitive dyes have been used extensively to screen for GPCRs that increase intracellular calcium levels in response to agonists challenge. Alternatively, a fluorescent or luminescent-based reporter assay co-transfected with the appropriate GPCR and G-protein has also been used to identify potential agonists or antagonists of the transfected GPCR (Nambi, P and Aiyar N, 2003, G-protein coupled receptors in drug discovery, in Assay and Drug Development Technologies Vol: 1, pp 305-310). While these assays are extremely useful in high throughput screening to identify potential agonists and antagonists, they do involve pre-labeling the cells with fluorescent dyes in the case of calcium-based assays or lysing the cells to measure the activity of reporter genes.

WO 2005/005979 demonstrates a label-free method for classification and characterization of cellular events including signal transduction pathways from G-protein coupled receptors. Specifically, the method generates parameters and profiles based on activation of secondary messengers and compares the parameters of each profile at a particular time point to classify receptor-ligand interactions into one of four classifications, namely, Gi, Gs, Gq, or PTKR. With respect to impedance, changes in impedance magnitude and phase are determined across a series of frequencies. This impedance spectra is then fitted into a polynomial algorithm, which provides a profile indicative of the second messengers associated with the receptor.

## SUMMARY OF THE INVENTION

The invention provides a method of identifying a compound capable of interacting with a G-Protein Coupled Receptor (GPCR) comprising: a) providing a device capable of measuring cell-substrate impedance; wherein said device is in the format of a multi-well plate, each well comprising a nonconductive substrate over which is fabricated a conductive electrode array that is capable of attachment by a cell, further wherein said device is operably connected to an impedance analyzer; b) adding test cells to at least two wells for attachment to said electrode array, wherein said test cells express a GPCR; c) determining first cell indices from first impedances, wherein said first impedances are cell-substrate impedances measured from said at least two wells immediately preceding step d), wherein the cell indices positively correlate with the number of viable cells in the wells, how well they are attached to the electrode surface, and the cell/electrode contact area due to the cell morphology status; d) adding a compound to at least one well containing said test cells to form at least one compound well and adding a vehicle control to at least another well containing test cells to form at least one control well; e) determining second cell indices from said second impedances, wherein said second impedances are cell-substrate impedances measured from said at least one compound well and said at least one control well after step d), wherein the cell indices positively correlate with the number of viable cells in the wells, how well they are attached to the electrode surface, and the cell/electrode contact area due to the cell morphology status; f) determining the change in cell index for said at least one compound well by comparing said second cell index of said at least one compound well to said first cell index of said at least one compound well, and determining the change in cell index of said at least one control well by comparing said second cell index of said at least one control well to said first cell index of said at least one control well; h) comparing said change in cell index between said at least one compound well and said at least one control well; and g) identifying said compound interacts with said GPCR if said comparison demonstrates a significant difference between said change in cell index of said at least one compound well and said change in cell index of said at least one control well.

According to a particular embodiment of the invention, said compound is added to at least two wells and in at least two different concentrations.

According to another particular embodiment of the invention, said compound is added to at least two wells in at least two different concentrations forming at least two compound wells, further wherein a dose curve is determined by plotting a maximum change in cell index for each concentration of said compound versus the corresponding concentration or determining the area under the cell-index curve for the different concentrations of the compound and plotting the area under the cell-index curve or impedance curve for each of the compound concentrations versus the corresponding concentration.

The invention also provides a method of identifying a compound capable of interacting with a G-Protein Coupled Receptor (GPCR) comprising: a) providing a device capable of measuring cell-substrate impedance; wherein said device is in the format of a multi-well plate, each well comprising a nonconductive substrate over which is fabricated a conductive array that is capable of attachment by a cell, further wherein said device is operably connected to an impedance analyzer; b) adding test cells to at least one of said at least two wells for attachment to said conductive array to form at least one test well, and adding control cells to at least another well for attachment to said conductive array to form at least one control well, wherein said test cells express a GPCR and said control cells do not express said GPCR or express said GPCR at a significantly lesser level that said test cells; c) determining first cell indices from first impedances, wherein said first impedances are cell-substrate impedances measured from said at least one test well and from said at least one control well immediately preceding step d), wherein the cell indices positively correlate with the number of viable cells in the wells, how well they are attached to the electrode surface, and the cell/electrode contact area due to the cell morphology status; d) adding a compound to said at least one test well and to said at least one control well; e) determining second cell indices from said second impedances, wherein said second impedances are cell-substrate impedances measured from said at least one test well and from said at least one control well after step d), wherein the cell indices positively correlate with the number of viable cells in the wells, how well they are attached to the electrode surface, and the cell/electrode contact area due to the cell morphology status; f) determining the change in cell index of said at least one test well by comparing said second cell index of said at least one test well to said first cell index of the said at least one test well, and determining the change in cell index of said at least one control well by comparing said second cell index of said at least one control well to said first cell index of the said at least one control well; g) comparing said changes in cell index between said at least one test well and said at least one control well; and h) identifying said compound interacts with said GPCR if said comparison demonstrates a significant difference between said change cell index for said at least one test well and said change in cell index for said at least one control well.

In some embodiments the multi-well plate is selected from the group consisting of a 16 well plate, a 24 well plate, a 96 well plate, a 384 well plate, and a 1536 well plate. In some embodiments cell substrate impedance is used to measure a characteristic of the cytoskeleton of a cell. In some embodiments the GPCR is selected from the group consisting of a recombinant GPCR, an endogenous GPCR, an orphan GPCR, a constitutively active GPCR, and chimeric GPCR, or other chimeric receptor containing GPCR property.

In some embodiments cell index is a normalized cell index wherein normalization is at a time point a short time before adding said compound or said vehicle control, wherein the said short time is selected from the group consisting of less than 1 minute, less than 2 minutes, less than 5 minutes, less than 10 minutes, less than 30 minutes, less than 1 hour, less than 2 hours, less than 5 hours, less than 10 hours and less than 24 hours. Changes in cell indices can be absolute changes in cell indices or relative changes in cell indices.

Each of said first impedances can be a series of impedance measurements prior to said adding said compound or said vehicle control and each of said first cell indices are a series of cell indices, wherein each of said first cell indices corresponds to a distinct impedance measurement within said series of impedance measurements. Measuring said first impedances can occur at a time selected from the group consisting of less than 1 minute, less than 5 minutes, less than 30 minutes, less than 1 hour, less 2 hours, less than 5 hours, less than 10 hours, and less than 24 hours prior to said adding said compound or said vehicle control. Changes in impedances can be relative changes in impedance. Each of said second impedances can be a series of impedance measurements after said adding said compound or said vehicle control and each of said second cell indices can be a series of cell indices, wherein each of said second cell indices correspond to a distinct impedance measurement within said series of impedance measurements. The second impedances can be measured at a time point selected from the group consisting of more than 1 minute, more than 5 minutes, more than 30 minutes, more than 1 hour, more than 2 hours, more than 5 hours, more than 10 hours, and more than 24 hours after said adding said compound or said vehicle control.

The compound can be obtained from a library of compounds. In some embodiments the compound is selected from a group consisting of a ligand, an agonist, and an inverse agonist. The compound can be identified as an agonist if said comparison indicates a significant change comprising an increase or a decrease cell index for said compound well after said compound is added, or as an inverse agonist if said comparison indicates a significant change in impedance comprising a decrease or increase in cell index for said compound well after said compound is added and the significant change cell index for the compound well is in the opposite direction compared to the change of a known agonist. The method can also include determining an EC50 of said compound, wherein said EC50 is a concentration of a compound capable of inducing 50% of maximum response in cell index or 50% of maximum change in area under the cell-index curve.

## BRIEF DESCRIPTION OF THE DRAWINGS

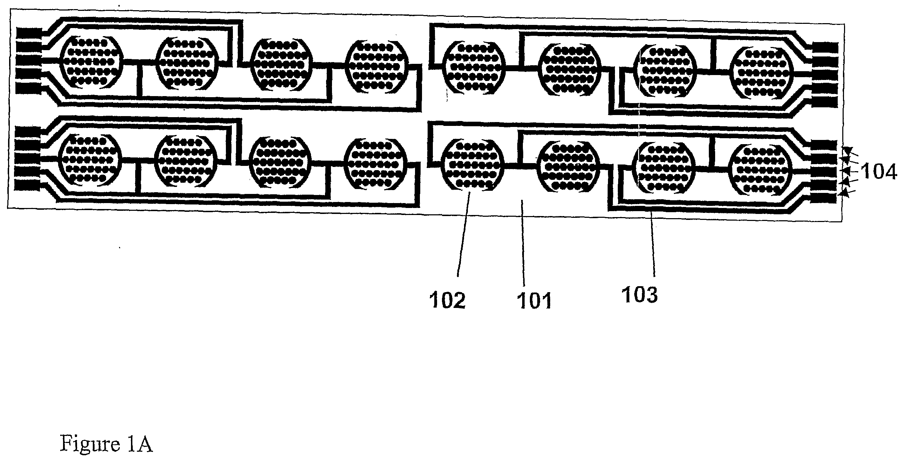

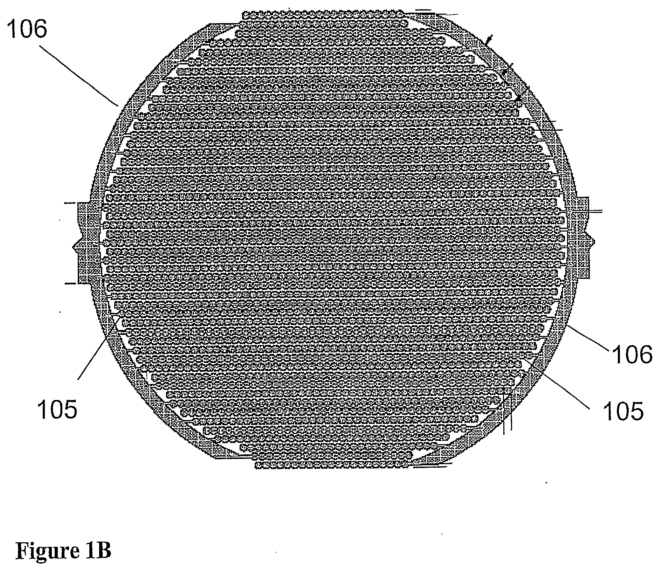

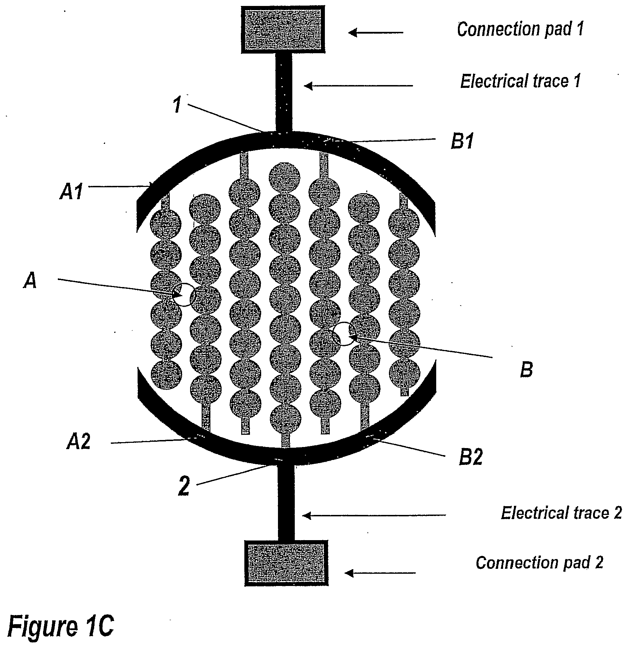

**Figure 1** shows schematic drawings of one design of our cell-substrate impedance measurement device. A) depicts the substrate having 16 electrode arrays (or 16 electrode structure units) that are arranged in a 2-row by 8-column configuration on a substrate. B) depicts a single electrode array of a device. C) shows a schematic drawing of an electrode array, illustrating the requirement of approximately uniform distribution of electrode resistance across the array.

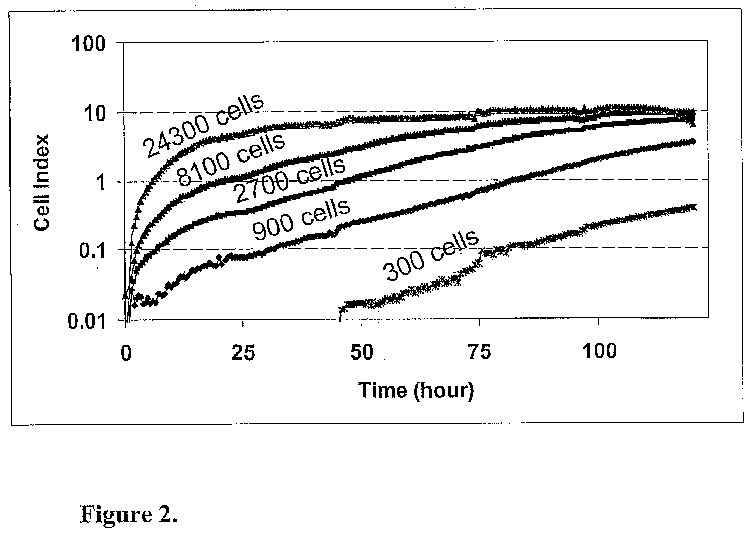

**Figure 2** shows real-time monitoring of proliferation of H460 cells seeded at different initial cell seeding numbers on our cell substrate impedance monitoring system. The cell proliferation was continuously recorded every 15 minutes for over 125 hours. The cell growth curves in the log scale show exponential cell growth or cells in the stationary phase.

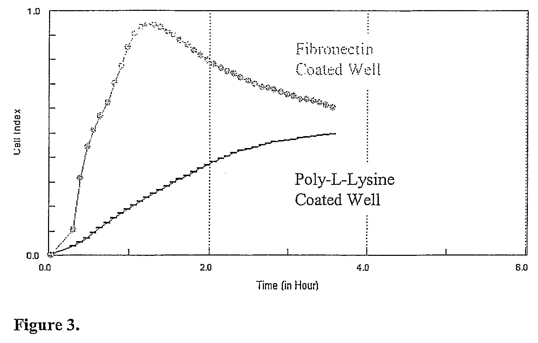

**Figure 3** shows real time monitoring of cell attachment and spreading of NIH3T3 cells using our cell-substrate impedance monitoring system. The cells were seeded onto devices coated with either poly- L-lysine or fibronectin. The cell attachment and cell spreading processes on the different coating surfaces were monitored every 3 minutes for over 3 hours in real time.

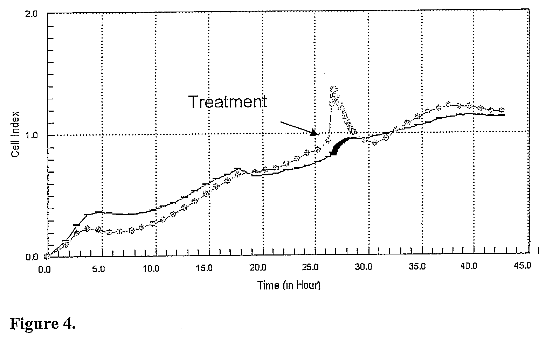

**Figure 4** shows real-time monitoring of morphological changes in Cos-7 cells uisng our cell-substrate impedance monitoring system. The cells were serum starved for 8 hours and stimulated with or without 50 ng/mL EGF. Changes in cell morphology were monitored at 3 min intervals for 2 hours and then 1 hour interval for 14 hours. The initial jump in the signal in EGF-treated cells is due to membrane ruffling and actin dynamics in response to EGF. The arrow indicates the point of EGF stimulation.

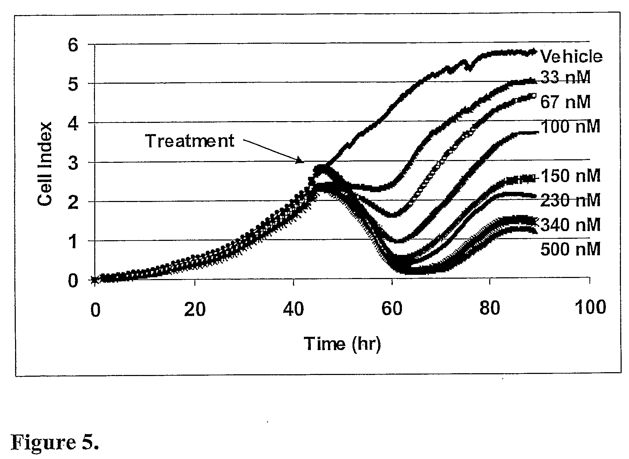

**Figure 5** shows time-dependent cell index for H460 cells treated by anticancer drug paclitaxel. Different wells of cultured H460 cells were treated at their exponential growth phase with different concentrations of Paclitaxel. The dynamic response of the cells to different doses of paclitaxel was monitored in real time every 15 minutes for 50 hours after treatment using our cell-substrate impedance monitoring system. For paclitaxel concentration between 67 nM and 500 nM, H460 cells exhibited a gradual decrease in cell index initially after the compound addition. However, the cell index reached a minimum at a time dependent on the compound concentration between about 15 hours and 20 hours after compound addition. After that point, the cell index exhibited a gradual increase in cell index. The cell index for compound concentration of 33 nM exhibited a near-constant value for time up to about 15 hours after compound addition. After 15 hours following the compound addition, the cell index exhibited a gradual increase in cell index.

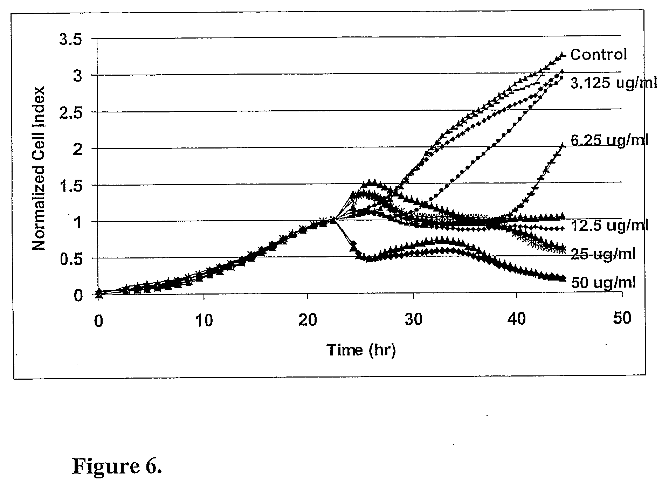

**Figure 6** shows time-dependent cell index for H460 cells treated by anticancer drug AC101103. Different wells of cultured H460 cells were treated at their exponential growth phase with different concentrations of AC101103. The dynamic response of the cells to different doses of AC 101103 was monitored in real time every 30 minutes for about 20 hours on our cell substrate impedance monitoring system. The time-dependent cell index in Figure 6 is significantly different from those shown in Figure 5. For compound concentrations at 3.125 ug/ml, 6.25 ug/ml and 12.5 ug/ml, the cell index exhibited a near-constant value for about 5 hrs, about 15 hrs and > 20 hrs respectively. For compound concentrations at 3.125 ug/ml and 6.25 ug/ml, the cell index started to increase after about 5 hrs and about 15 hrs following compound addition. For the compound concentration of 25 ug/ml, there was a gradual, yet slow decrease in the cell index after compound addition. For the compound concentration of 50 ug/ml, there was an about 10 hr time period over which the cell index remained near-constant, and after that, the cell index decreased steadily.

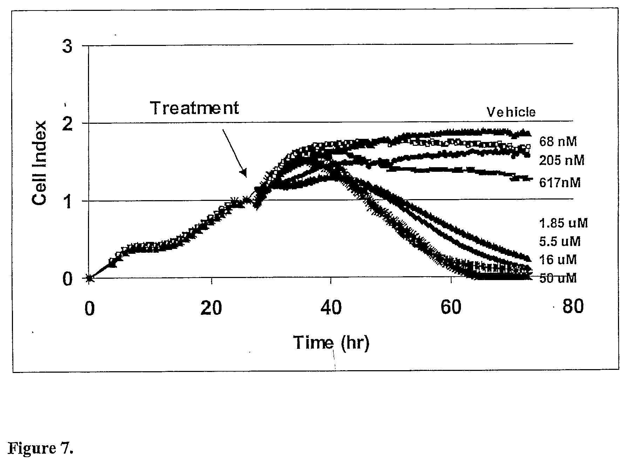

**Figure 7** shows dynamic drug response curves of A549 cells treated with doxombicin. 10,000 A549 cells were seeded in each well of a 16 X device. The cell attachment and cell growth were monitored on our cell-substrate impedance monitoring system in real time before treatment. When the cells were in exponential growth phase, doxorubicin at different concentration was added to the cells. Same volume of a solvent used for dissolve the drug was served as vehicle control. The time, and drug dose dependent cell response to doxorubicin was recorded in real time.

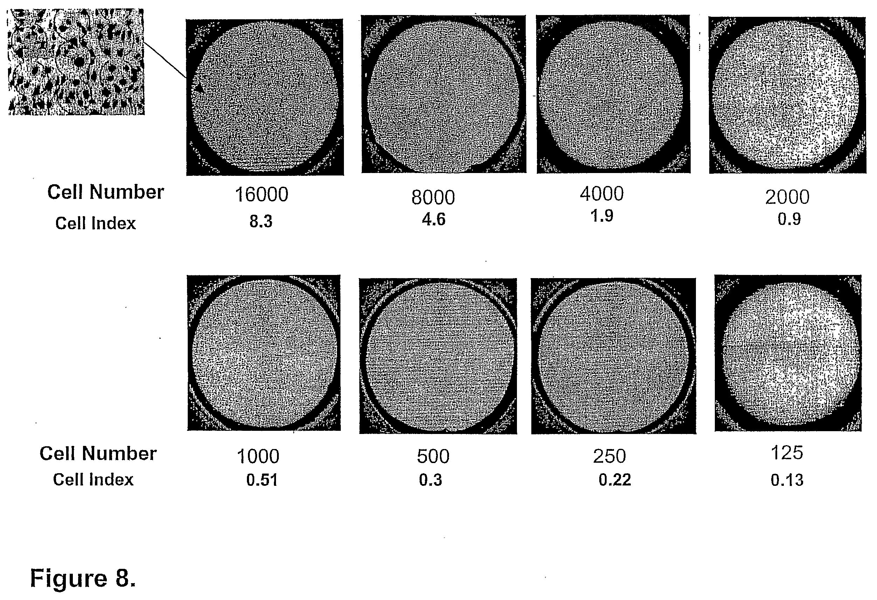

**Figure 8** shows titration of NIH3T3 cells on our cell-substrate impedance device. The indicated cell number of cells were seeded into microtiter devices fabricated with electronic sensor arrays shown in Figure 1B. The electronic sensor arrays were-precoated with fibronectin. Two hours after seeding, the cell index number was determined using our cell-substrate impedance monitoring system.

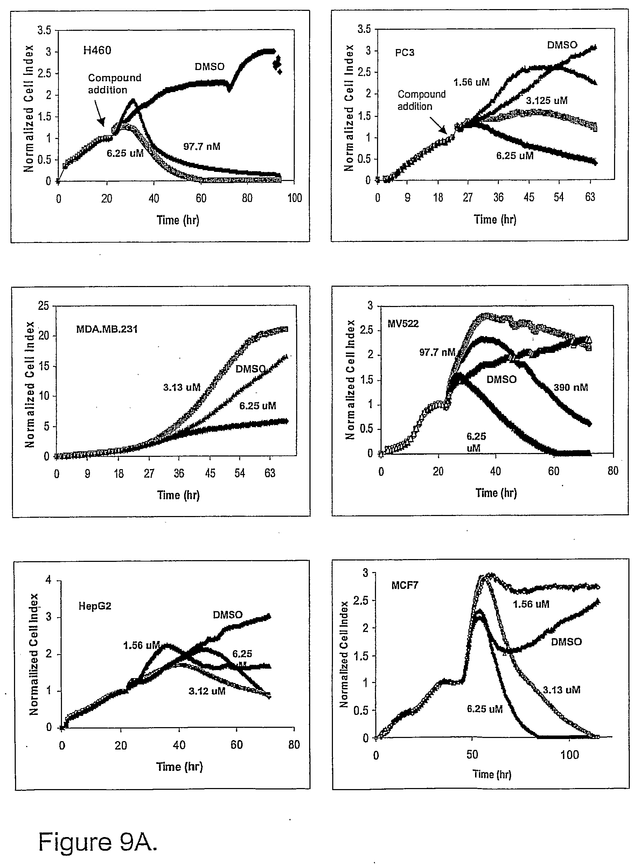

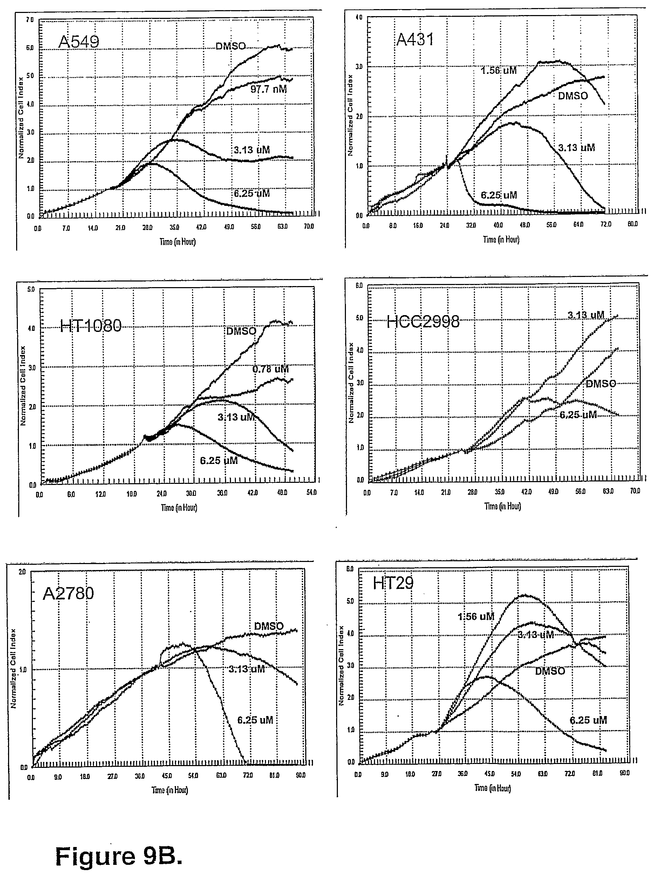

**Figure 9A and B** shows the responses of various cell types (listed in Table 1) to doxorubicin treatment as monitored using our cell-substrate impedance monitoring system. The indicated cell lines were seeded onto microtiter devices fabricated with electronic sensor arrays shown in Figure 1B. The cellular responses were continuously monitored at 15 or 30 or 60 minutes time interval before and after treatment with doxorubicin.

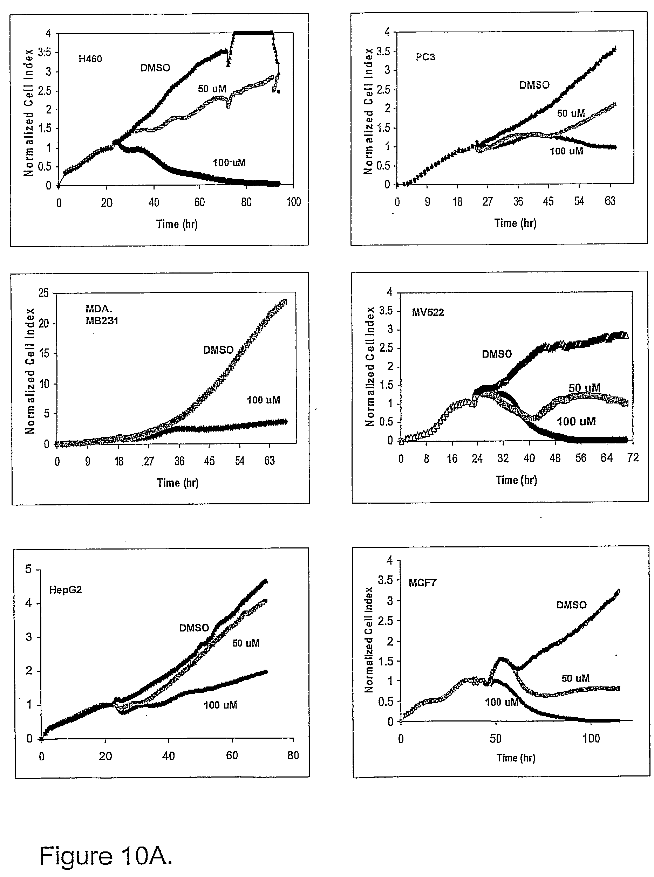

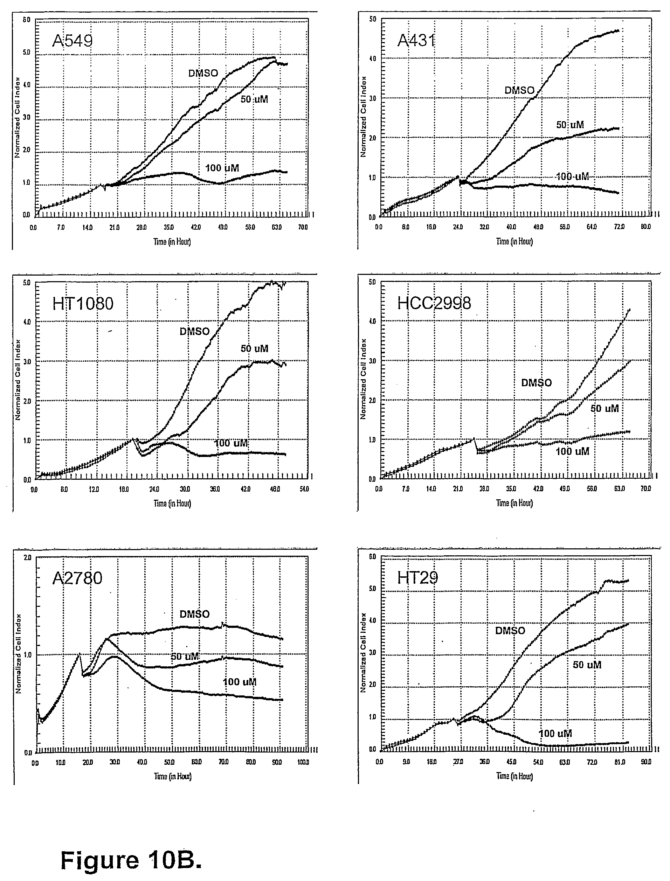

**Figure 10A and B** shows the responses of various cell types (listed in Table 1) to olomoucine treatment as monitored using our cell-substrate impedance monitoring system. The indicated cell lines were seeded onto microtiter devices fabricated with electronic sensor arrays shown in Figure 1B. The cellular responses were continuously monitored at 15 or 30 or 60 minutes time interval before and after treatment with olomoucine.

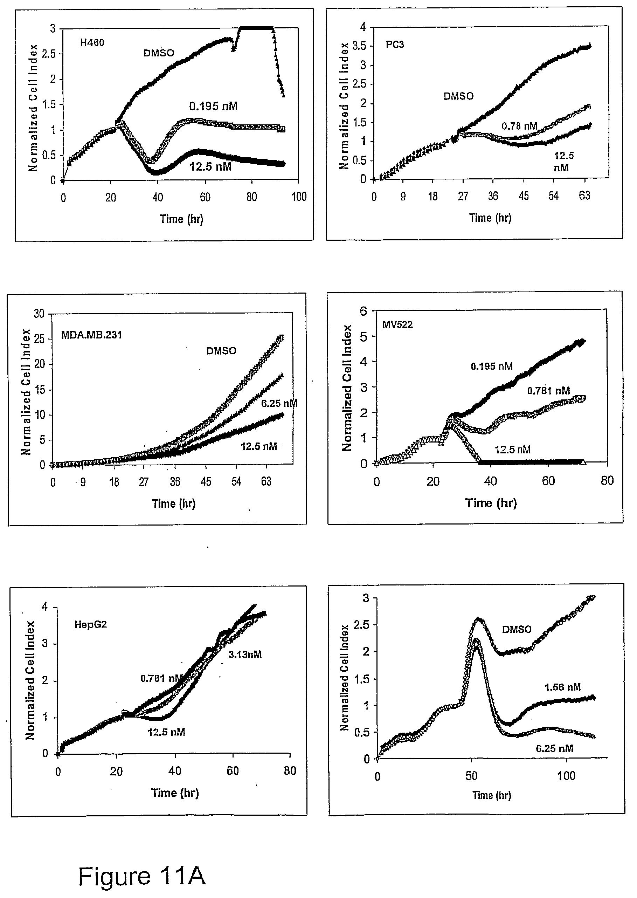

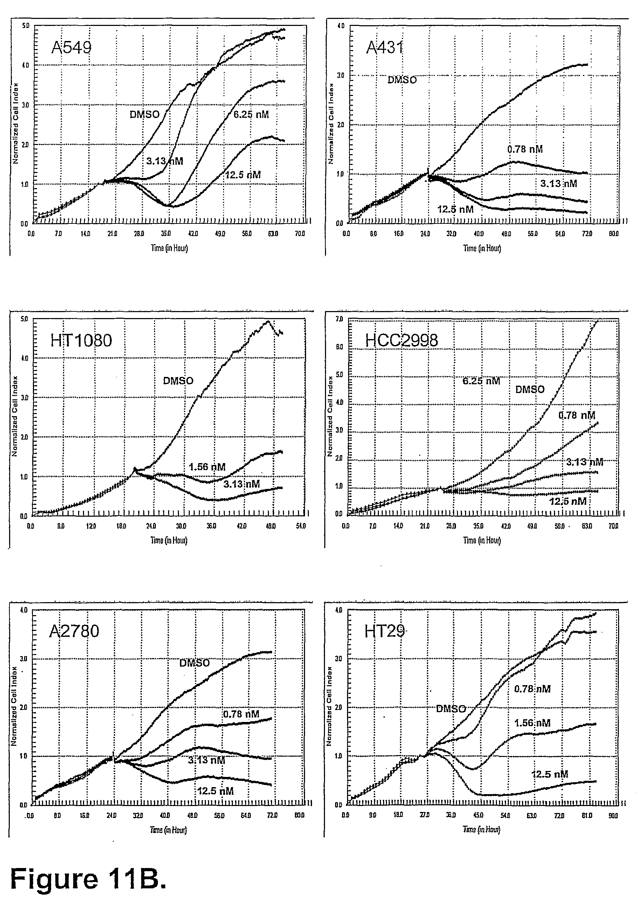

**Figure 11A and 11B** show the responses of various cell types (listed in Table 1) to paclitaxel treatment as monitored using our cell-substrate impedance monitoring system. The indicated cell lines were seeded onto microtiter devices fabricated with electronic sensor arrays shown in Figure 1B. The cellular responses were continuously monitored at 15 or 30 or 60 minutes time interval before and after treatment with paclitaxel.

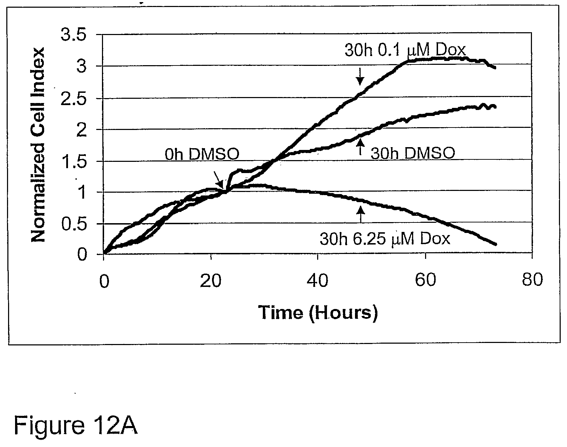

**Figure 12A** shows the response of MV522 cells to doxorubicin treatment as monitored using our cell-substrate impedance monitoring system. MV522 cells were seeded onto microtiter devices fabricated with electronic sensor arrays shown in Figure 1B and were treated with either DMSO or doxorubicin at the indicated time and concentration.

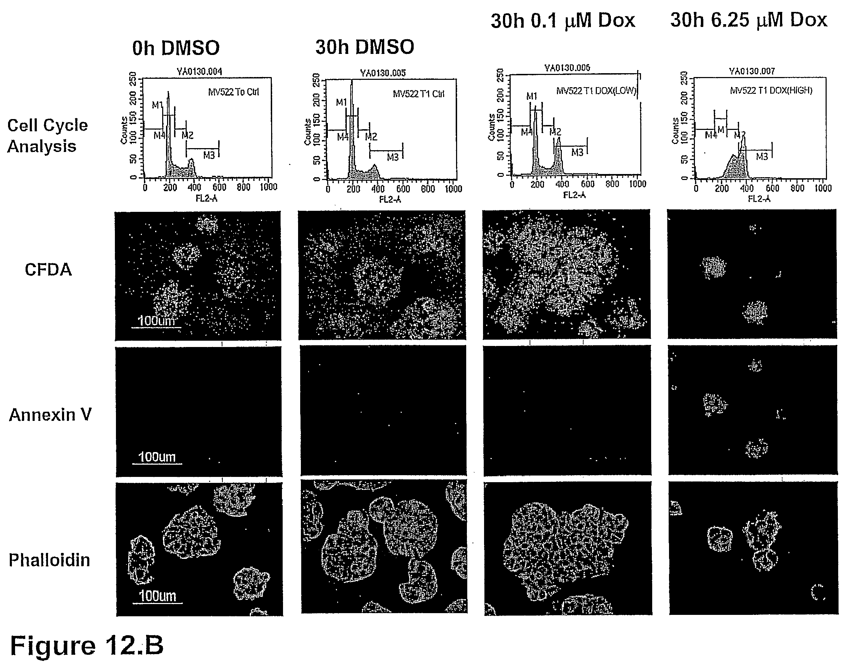

**Figure 12B** shows the characterization of the cell biological effect of doxorubicin treatment on MV522 cells. The cells were either processed for cell cycle analysis using FACS or treated with CFDA and Cy3-Annexin V to assess cell viability. In addition, the cells were fixed and stained with phalloidin to examine cell morphology. For viability and morphology, the cells were visualized and photographed using a fluorescence microscope equipped with CCD camera.

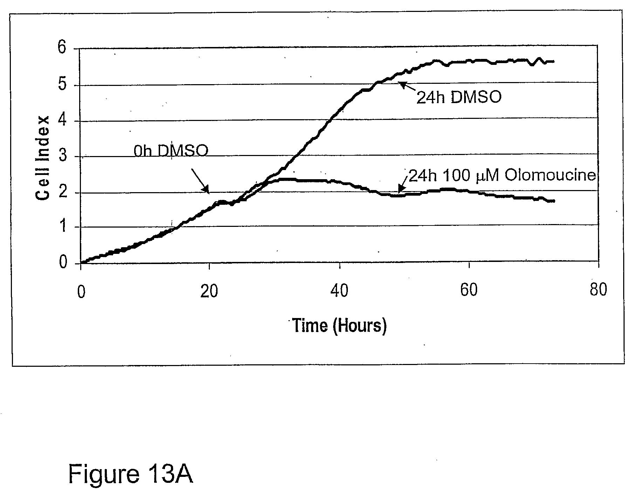

**Figure 13A** shows the response of A549 cells to olomoucine treatment as monitored using our cell-substrate impedance monitoring system. A549 cells were seeded onto microtiter devices fabricated with electronic sensor arrays shown in Figure 1B and were treated with either DMSO or olomoucine at the indicated time and concentration.

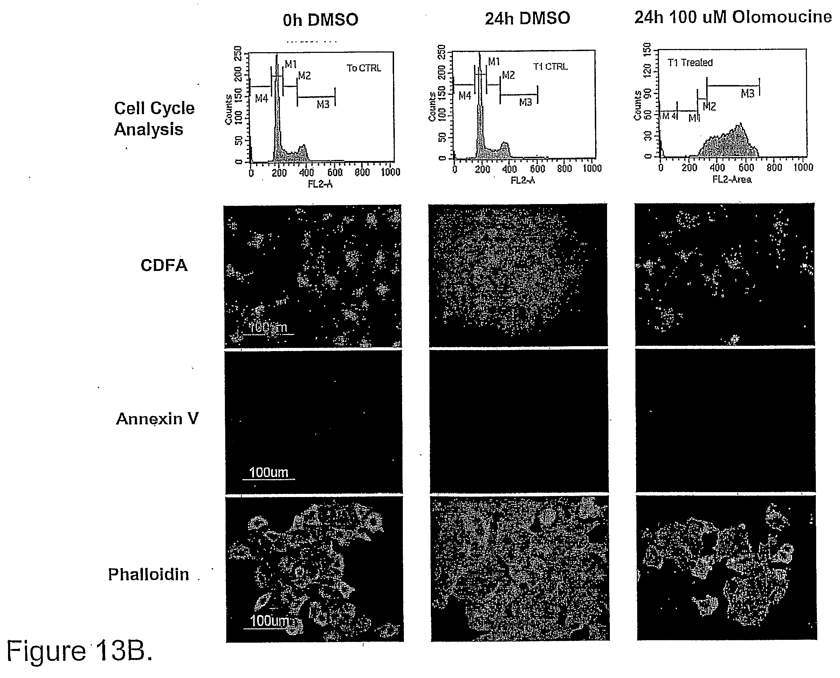

**Figure 13B** shows the characterization of the cell biological effect of olomoucine treatment on MV522 cells. The cells were either processed for cell cycle analysis using FACS or treated with CFDA and Cy3-Annexin V to assess cell viability. In addition, the cells were fixed and stained with phalloidin to examine cell morphology. For viability and morphology, the cells were visualized and photographed using a fluorescence microscope equipped with CCD camera.

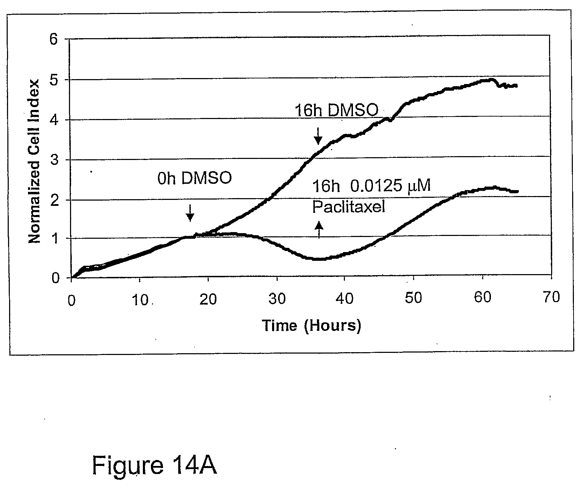

**Figure 14A** shows the response of A549 cells to paclitaxel treatment as monitored using our cell-substrate impedance monitoring system. A549 cells were seeded onto microtiter devices fabricated with electronic sensor arrays shown in Figure 1B and were treated with either DMSO or paclitaxel at the indicated time and concentration.

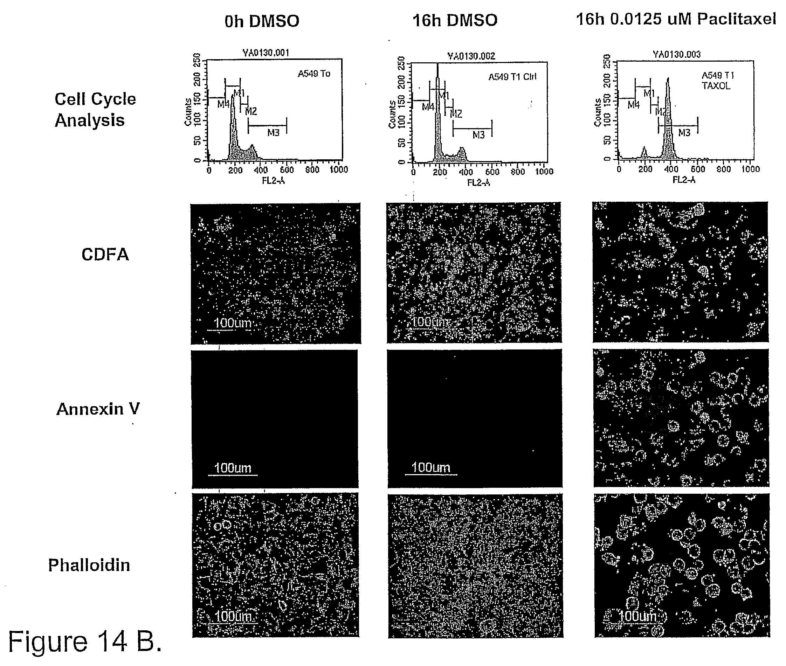

**Figure 14B** shows the characterization of the cell biological effect of paclitaxel n treatment on A549 cells. The cells were either processed for cell cycle analysis using FACS or treated with CFDA and Cy3-Annexin V to assess cell viability. In addition, the cells were fixed and stained with phalloidin to examine cell morphology. For viability and morphology, the cells were visualized and photographed using a fluorescence microscope equipped with CCD camera.

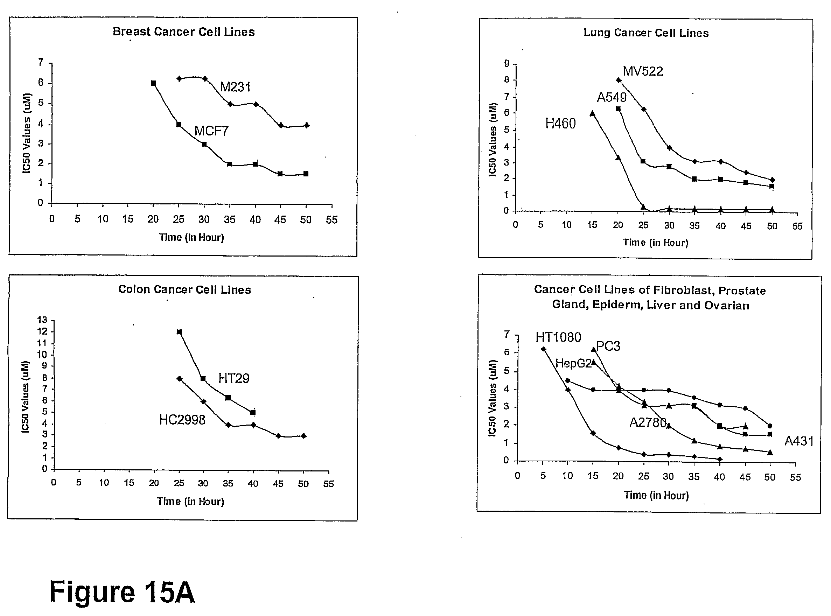

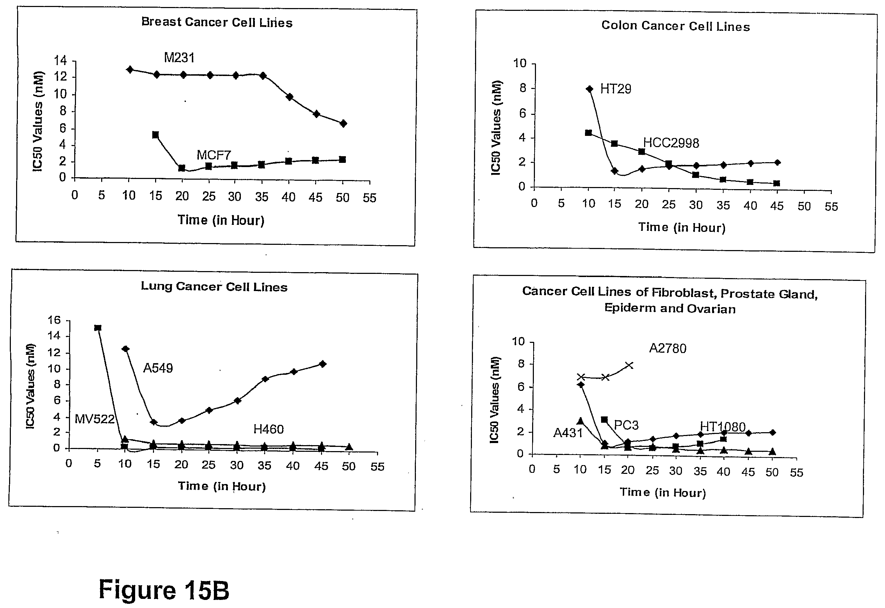

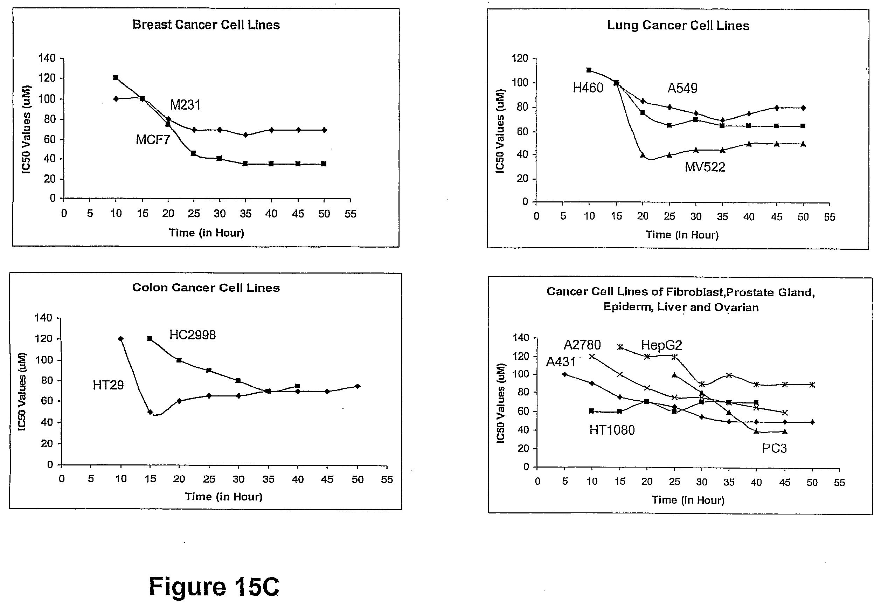

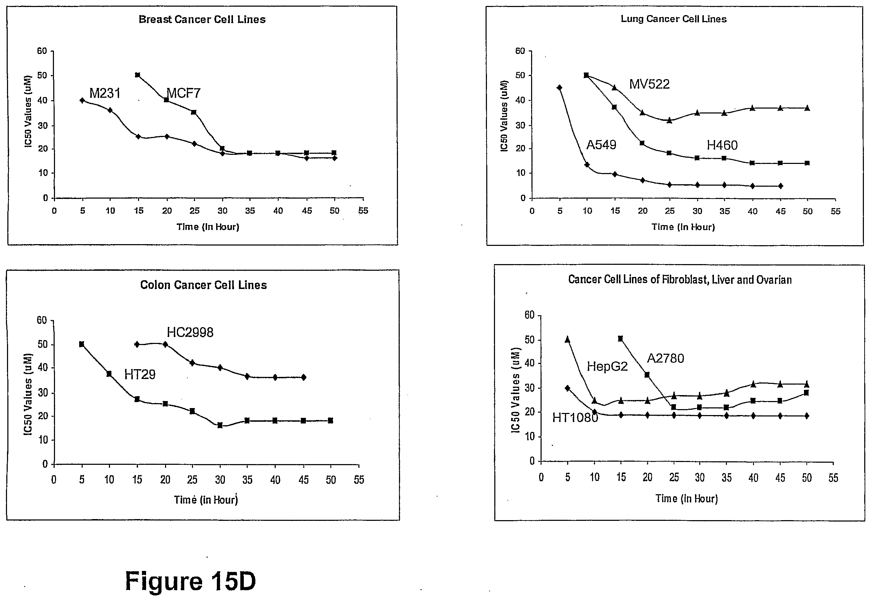

**Figure 15.** The time dependent IC values for each compound (15A: Doxorubicin; 15B: Paclitaxel; 15C: Olomoucine; 15D: Tamoxifan) for the indicated cell lines as estimated at 5 hr intervals from the cell index curves obtained using our cell-substrate impedance monitoring system.

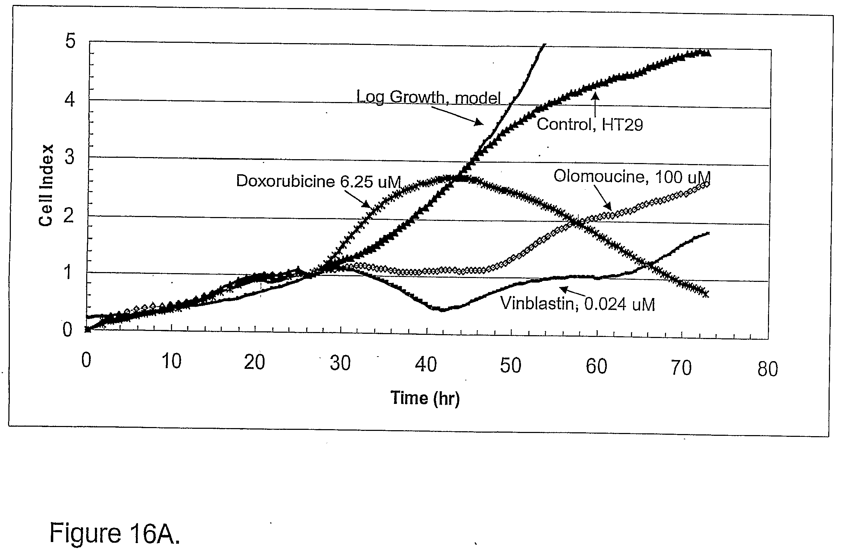

**Figure 16A** shows the cell index curves of HT29 cells before and after treatment with various compounds. Also shown is an theoretical exponential increase of cell index with time (labeled as "Log-growth, model") and cells treated with DMSO vehicle control (labeled as "Control, HT29").

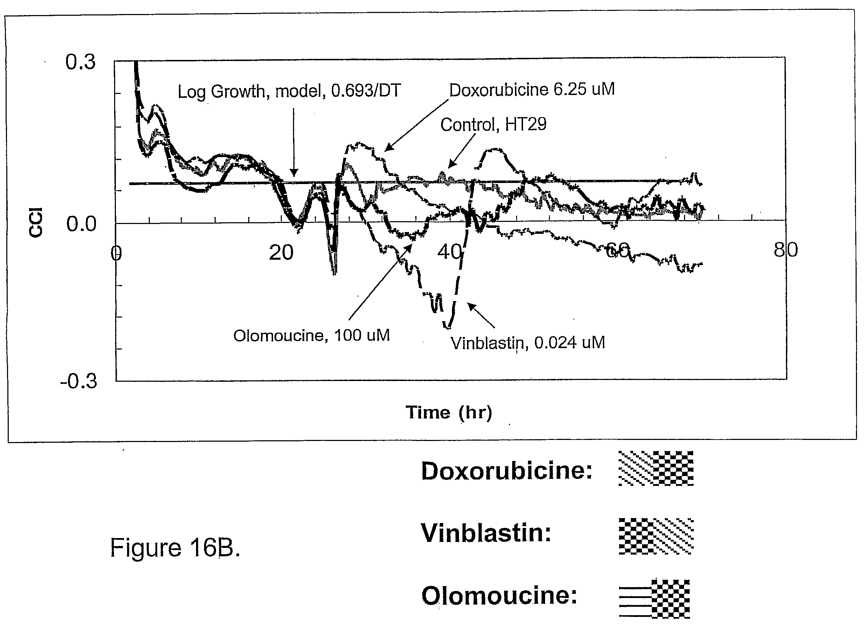

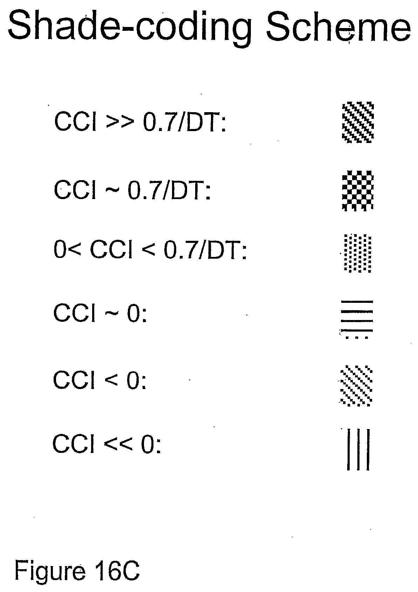

**Figure 16B** shows the derived cell change index (CCI) from the cell index curves shown in Figure 16A. Also shown is the "black-white shading codes" used for different responses based on the convention shown in Figure 16C.

**Figure 16C** shows the color-coding scheme used for representing the CCI curves. If the DT is the doubling time for the cells undergoing exponential growth in the cell culture media used, then CCI having different values relative to 0.7/DT indicates the different cell change status. If CCI >> 0.7/DT, cell index increases faster than that expected for an exponential growth (or log growth) of the cells (such region n the CCI curve is represented as ▨ Rectangle). If CCI is about 0.7/DT, cell index increases in the same rate as that expected for an exponential growth of the cells (such region in the CCI curve is represented as ▦ Rectangle). If CCI is more than zero but somewhat smaller than 0.7/DT, then cell index increases in the rate slowed than that expected for an exponential growth (such region of the CCI curve is represented as ▦ Rectangle). If CCI is about zero, then cell index shows a near constant value (such region of the CCI curve is represented as ☰ Rectangle). If CCI is negative, then the cell index is decreasing with time, showing the cells losing attachment to the electrode surface or changing their morphology (such region of the curve is shown as ▨ Rectangle). If CCI is very negative, then the cell index decreases rapidly with time, showing that either cells lose attachment to the electrode surfaces quickly or cells change their morphology very quickly (such region of the CCI curve is represented as ⦀ Rectangle). The transient, quick noise in the CCI values are removed so that the whole CCI curve is represented after compound addition by one, two or three black/white-shaded rectangles.

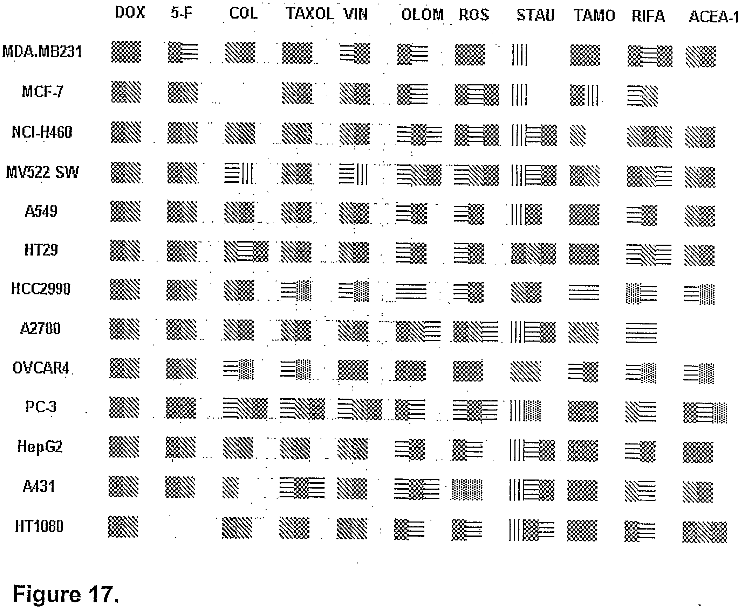

**Figure 17** shows the cell response profile of each cell line tested against the indicated chemotherapeutic agents. For each cell line and compound, the time-dependent cell change index (CCI) was calculated from their corresponding RT-CES responses at an IC50 concentration. (IC 50 is time dependent so that the IC50 concentration at 30h, or the concentration closest to that, after drug addition is used). The specific CCI curves as related to specific cellular responses were coded according to the convention described in Figure 16C and displayed in groups of compounds with similar mechanism of action. DOX: doxorubicin; 5-F: 5-Fluorouracil; COL: Colcemid; TAXOL: paclitaxel; VIN: vinblastin; OLOM: Olomoucine; ROS: Roscovitine; STAU: Staurosporine; TAMO: Tamoxifan; RIFA: Rifampicin; ACEA-1: an ACEA test compound.

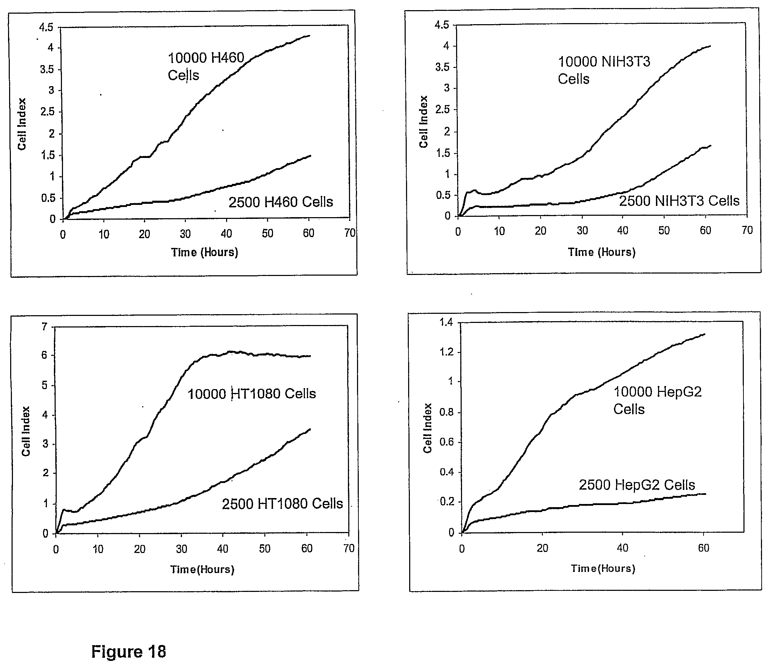

**Figure 18.** Dynamic monitoring of cell proliferation. H1080 fibrosarcoma cells, H460 lung cancer cells, HepG2 hepatosarcoma cancer cells and NIH3T3 mouse fibroblast cell lines were seeded at a density of 2500 and 10,000 cells per well of ACEA 96x e-Plate device. The adhesion, spreading and proliferation of the cells were dynamically monitored every 30 minutes using our cell-substrate impedance monitoring system.

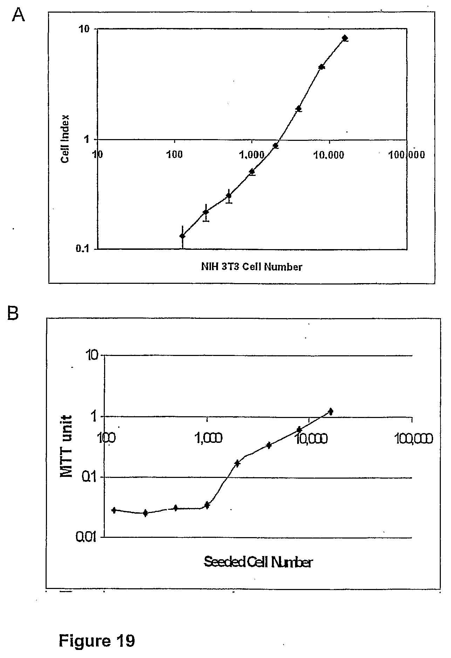

**Figure 19.** Correlation between cell-substrate impedance measurement (as shown here, Cell Index) and number of cells seeded and comparison of Cell Index with MTT. **(A)** Increasing numbers of NIH3T3 ranging from 100 cells all the way up to 10,000 cells were seeded in our device and the cells were monitored for 10 hours at which point the Cell Index was obtained. The Cell Index value was plotted against the corresponding number of cells. **(B)** The cells described in Figure 19A were assayed by MTT assay at the end of the experiment and the optical density at 590 nm was plotted against the number of cells seeded.

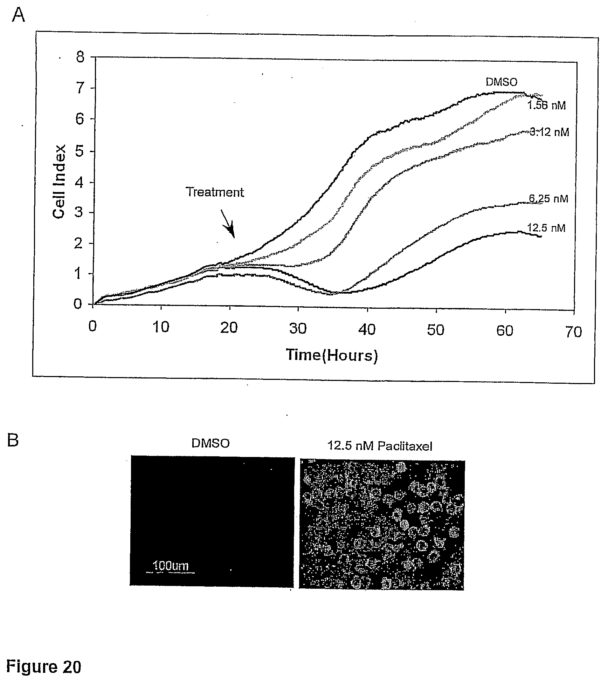

**Figure 20.** Dynamic monitoring of drug interaction with target cells using our cell-substrate impedance monitoring system. **(A)** A549 cells were seeded in a device at a density of 10,000 cells per well and the cells were continuously monitored up to 24 hours at which point paclitaxel was added at the indicated final concentrations. **(B)** Annexin V staining of A549 cells treated with DMSO or 12.5 nM paclitaxel for 20 hours. The cells were observed with fluorescence microscope and images were captured with an attached digital camera.

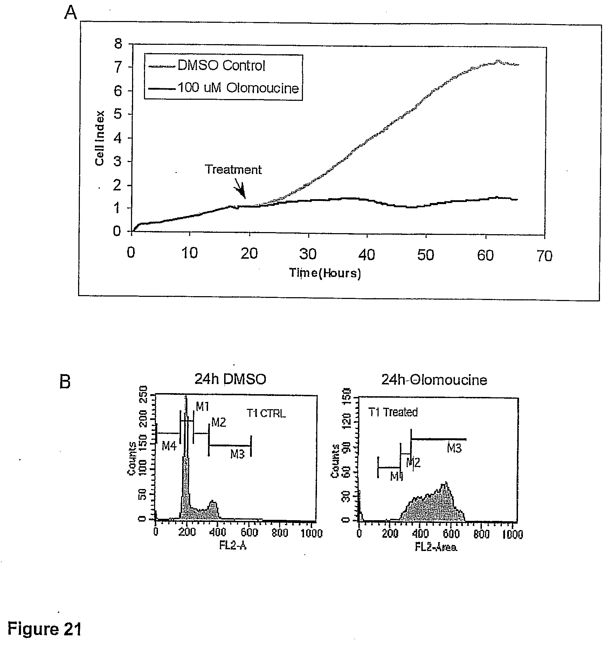

**Figure 21.** Dynamic monitoring of cell cycle arrest using our cell-substrate impedance monitoring system. A549 cells were seeded in a device at 10,000 cells per well and continuously monitored using the RT-CES. The cells were treated with either **(A)** DMSO or 100 $\mu$M Olomoucine **(B)** A549 cells growing on tissue culture dishes for 20 hours were treated with DMSO or 100 $\mu$M Olomoucine. Cell cycle analysis was performed by flow cytometry.

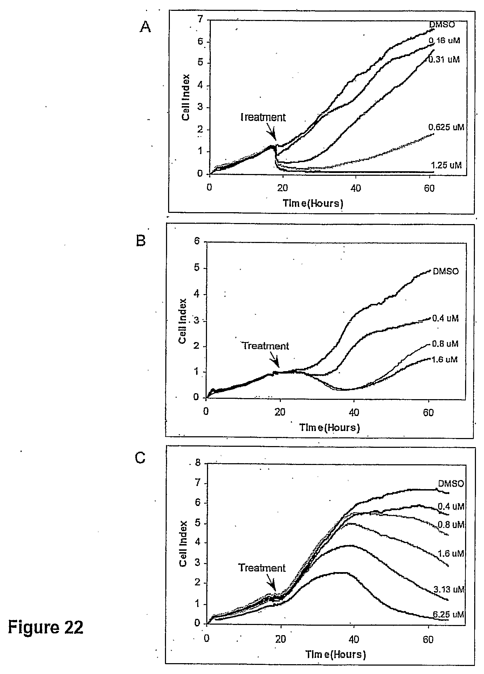

**Figure 22.** Dynamic monitoring of cytotoxic compounds with target cells using our cell-substrate impedance monitoring system. A549 cells were seeded in our device and continuously monitored using the RT-CES system. The cells were treated with the indicated final concentrations of (A) staurosporine, (B) vinblastine and (C) 5-flourouracil.

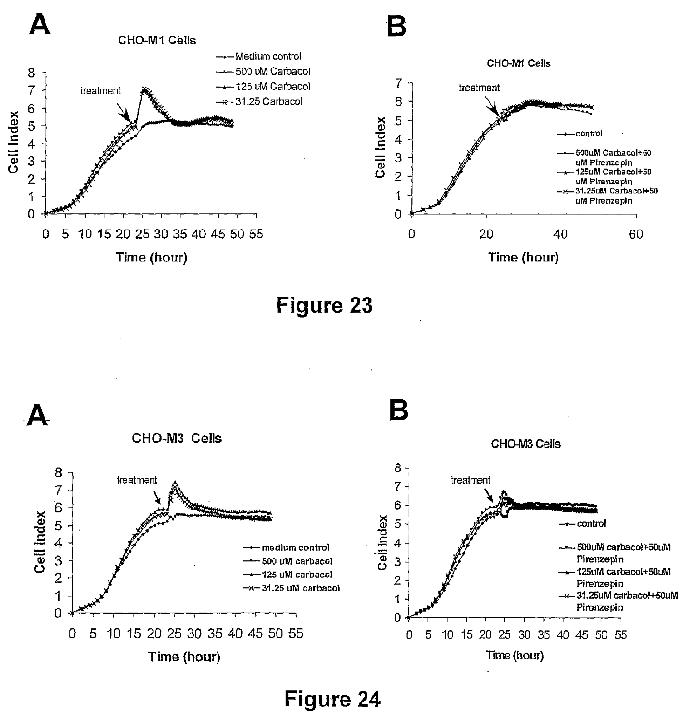

**Figure 23.** (A) CHO-M1 cells expressing the muscarinic M1 receptor were seeded in the wells of ACEA 16X device (16X E-Plate, 16 well microtiter plate that has incorporated microelectrode arrays at the bottom of the wells) and continuously monitored using the RT-CES™ system. At 24 hours post seeding the cells were stimulated with different doses of carbacol between 500 uM and 31.25 uM cabacol, plus media control. (B) The cells were seeded and monitored as described in (A). However, prior to stimulation with indicated doses of carbacol, the cells were incubated with the M1 receptor antagonist, pirenzipine at a final concentration of 50 uM.

**Figure 24.** CHO-M3 cells were seeded on ACEA 16X devices (16X E-Plate), grown and stimulated with the indicated doses of carbacol as described in Figure 23 and monitored by RT- CES™ system.

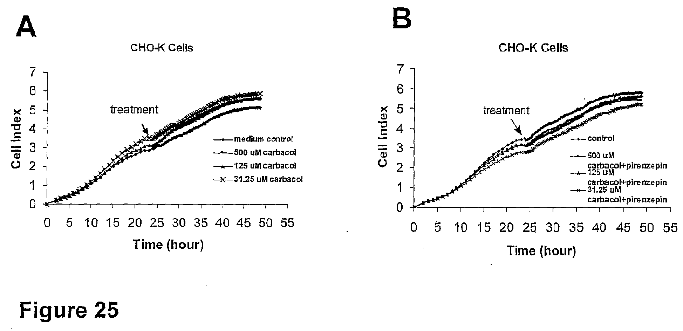

**Figure 25.** CHO cells which do not express the M1 and M3 receptors were seeded on ACEA 16X device (16X E-Plate) and were treated with carbacol and pirenzipine as described in Figures 23 and 24. The cells do not respond to the agonist carbacol (A) or the antagonist pirenzepine (B).

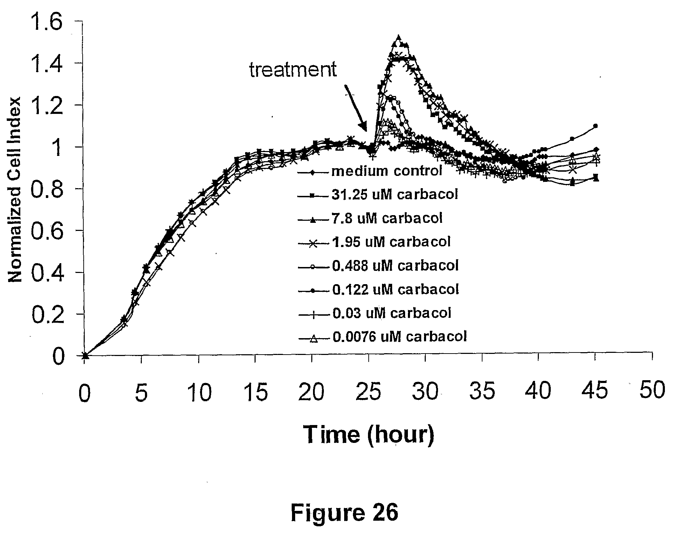

**Figure 26.** CHO-M1 cells growing on ACEA 16X microtiter devices were stimulated with the indicated doses of carbacol and the dose-dependent cell-electrode impedance response was monitored by RT-CES.

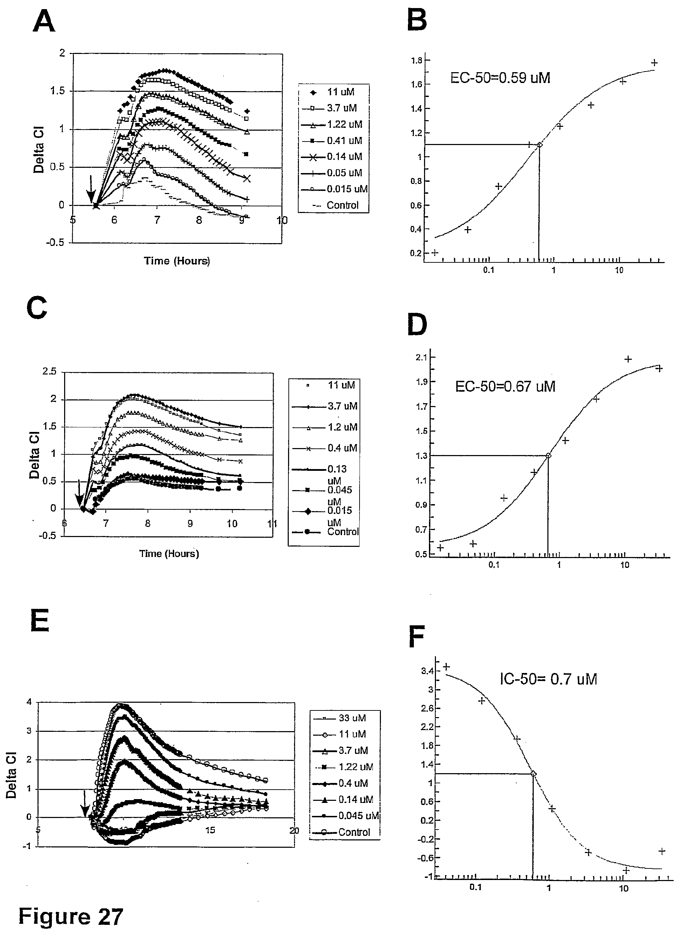

**Figure 27** depicts the pharmacological characterization of carbachol-evoked CI increases in Muscarinic 1 Receptor (M1) And Muscarinic 3 Receptor (M3) expressed in CHO cells: blockage by M1 selective antagonist pirenzepine. M1 and M3 muscarinic receptor cell lines were seeded at 50,000 cells per well of ACEA E-plates (E-plates are microtiter plates that have incorporated microelectrode arrays at the bottom of the wells). The cells were continuously monitored using the RT-CES system. At the indicated time point of treatment, increasing concentrations of carbachol (from 0 to 11 uM) were added to the cells and the cell response was monitored every 3 minutes by the RT-CES system. Carbachol leads to a dose-dependent and transient increase in CI in M1 (A) and M3 (C). Delta cell index shown in (A) and (C) at a given time point was calculated by subtracting the cell index at a standard time point from the cell index at the given time point. Here the standard time point was the last time point of the measurement before adding carbachol to the CHO cells (the first time point on the traces in A and C). Plotting the peak Delta cell index responses versus the corresponding log concentration allows for calculation of the $EC_{50}$ (0.59 uM and 0.67 uM) of carbachol for M1 (B) and M3 (D). For antagonist study, increasing concentrations of pirenzepine (from 0 to 33 uM), a selective M1 antagonist, were added to the cells 10 min prior to the application of a fixed concentration carbachol (10 uM) and the cell response was monitored every 3 minutes by the RT-CES system. Delta cell index shown in (E) at a given time point was calculated by subtracting the cell index at a standard time point from the cell index at the given time point. Here the standard time point was the last time point of the measurement before adding carbachol to the cells (the first time point on the traces in E). Pirenzepine blocked carbachol-induced Delta CI increases in a concentration-dependant manner as shown in traces (E). Plotting the maximal blocking effects on cell index response versus the corresponding log concentration allows for calculation of the $IC_{50}$ (~ 0.6 uM) of pirenzepine (F).

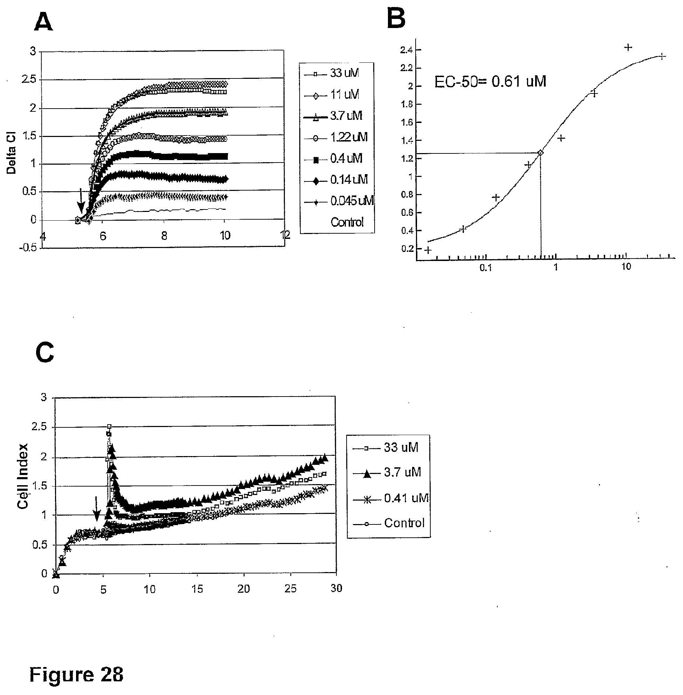

**Figure 28** depicts the carbachol-evoked increases of Cell Index (CI) of rat basophilic leukemia (RBL-2H3) cells expressing M3 and M2 receptors. M3 and M2 muscarinic receptor cell lines were seeded at 50,000 cells per well of ACEA E-plates. The cells were continuously monitored using the RT-CES system. At the indicated time point, increasing concentrations of carbachol (from 0 to 33 uM) were added to the cells and the cell response was monitored every 3 minutes by the RT-CES system. Carbachol leads to a dose-dependent and transient increase in CI in M3 (A) and M2 (C). Plotting the peak cell index response versus the corresponding log concentration allows for calculation of the $EC_{50}$ of carbachol for M3 (B). Delta cell index shown in (A) at a given time point was calculated by subtracting the cell index at a standard time point from the cell index at the given time point. Here the standard time point was the last time point of the measurement before adding carbachol to the cells (the first time point on the traces in A).

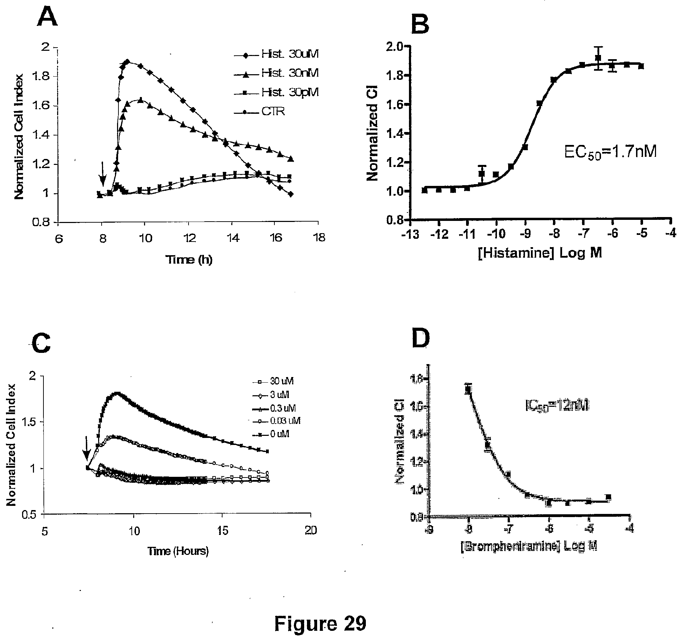

**Figure 29** depicts histamine-evoked increases of Cell Index (CI) of CHO-K1 cells expressing the human recombinant H1 receptor (hH1-C1): pharmacoloy characterization. Cell lines were seeded at 50,000 cells per well of ACEA E-plates. The cells were continuously monitored using the RT-CES system. At the indicated time point, increasing concentrations of histamine (from 0 (CTR) to 30 uM) were added to the cells and the cell response was monitored every 3 minutes by the RT-CES system. Histamine leads to a dose-dependent and transient increase in CI in hH-C1 cells shown in traces (A). Plotting the peak normalized cell index response versus the corresponding log concentration allows for calculation of the $EC_{50}$ of histamine (B). For blocking experiment, various concentrations of brompheniramine (histamine receptor antagonist, between 0 uM and 30 uM) were added to the cells 10 min prior to the addition of a fixed concentration histamine (20 nM)) and the cell response was monitored every 3 minutes by the RT-CES system. Brompheniramine blocked histamine-induced normalized-CI increase in a concentration-dependant manner as shown in traces (C). Plotting the maximal blocking effects on cell index response versus the corresponding log concentration allows for calculation of the $IC_{50}$ of brompheniramine (D). Normalized cell index shown in (A) and (C) at a given time point was calculated by dividing the Cell Index at the time point by the Cell Index at a reference time point. Here the reference time point was the last time point of the measurement before adding histamine to the cells. Note that sometime, we refer the reference time point for calculating normalized cell index as the time point at which the normalization is (i.e., normalization is at the reference time point).

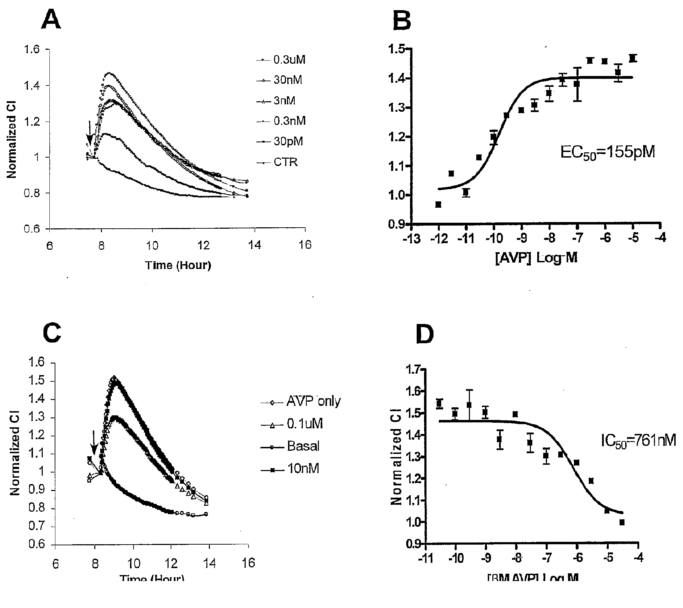

**Figure 30** depicts an example of [Arg[8]]Vasopressin (AVP)-evoked increases of Cell Index (CI) of 1321-N1 cells expressing the human recombinant vasopressin (V1a) receptor (V1a-C1): Blockage by selective non-active analogue [β-Mercapto- β, β - cyclopentamethylene-propionyl1, O-Et-Tyr[2], Val[4], Arg[8]]Vasopressin (βM AVP). V1a-C1 cells were seeded at 50,000 cells per well of ACEA E-plates. The cells were continuously monitored using the RT-CES system. At the indicated time point, increasing concentrations of AVP were added to the cells and the cell response was monitored every 3 minutes by the RT-CES system. AVP leads to a dose-dependent and transient increase in CI in V1a-C1 cells shown in traces (A). Plotting the peak normalized-cell-index response versus the corresponding log concentration allows for calculation of the $EC_{50}$ of AVP (B). For blocking experiment, various concentrations of βM AVP were added to the cells 10 min prior to the addition of a fixed concentration of AVP (1uM) and the cell response was monitored every 3 minutes by the RT-CES system. βM AVP blocked AVP-induced CI increases in a concentration-dependant manner as shown in traces (C). Plotting the maximal blocking effects on cell index response versus the corresponding log concentration allows for calculation of the $IC_{50}$ of βM AVP (D). Normalized cell index shown in (A) and (C) at a given time point was calculated by dividing the Cell Index at the time point by the Cell Index at a reference time point. Here the reference time point was the last time point of the measurement before adding AVP to the cells. Note that sometime, we refer the reference time point for calculating normalized cell index as the time point at which the normalization is (i.e., normalization is at the reference time point).

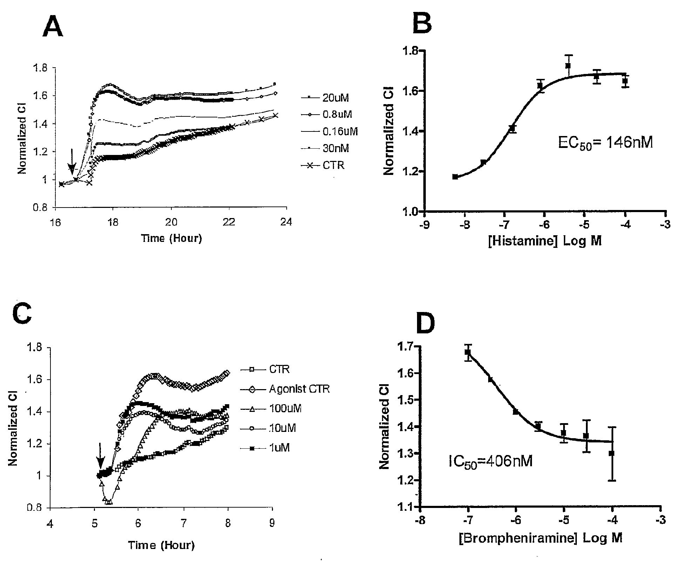

**Figure 31** depicts histamine-evoked increases of Cell Index (CI) of HeLa cells which express the endogenous histamine receptors: Blockage by antagonist brompheniramine. HeLa cells were seeded at 50,000 cells per well of ACEA E-plates. The cells were continuously monitored using the RT-CES system. At the indicated time point, increasing concentrations of histamine were added to the cells and the cell response was monitored every 3 minutes by the RT-CES system. Histamine leads to a dose-dependent and transient increase in CI in HeLa cells shown in traces (A). Plotting the peak cell index response versus the corresponding log concentration allows for calculation of the $EC_{50}$ of histamine (B).For the antagonist study, increasing concentrations of brompheniramine were added to the cells 10 min prior to the application of a fixed concentration histamine (5 uM) and the cell response was monitored every 3 minutes by the RT-CES system. Brompheniramine blocked histamine-induced CI increases in a concentration-dependant manner as shown in traces (C). Plotting the maximal blocking effects on cell index response versus the corresponding log concentration allows for calculation of the $IC_{50}$ of brompheniramine (D). Normalized cell index shown in (A) and (C) at a given time point was calculated by dividing the Cell Index at the time point by the Cell Index at a reference time point. Here the reference time point was the last time point of the measurement before adding histamine to the cells. Note that sometime, we refer the reference time point for calculating normalized cell index as the time point at which the normalization is (i.e., normalization is at the reference time point).

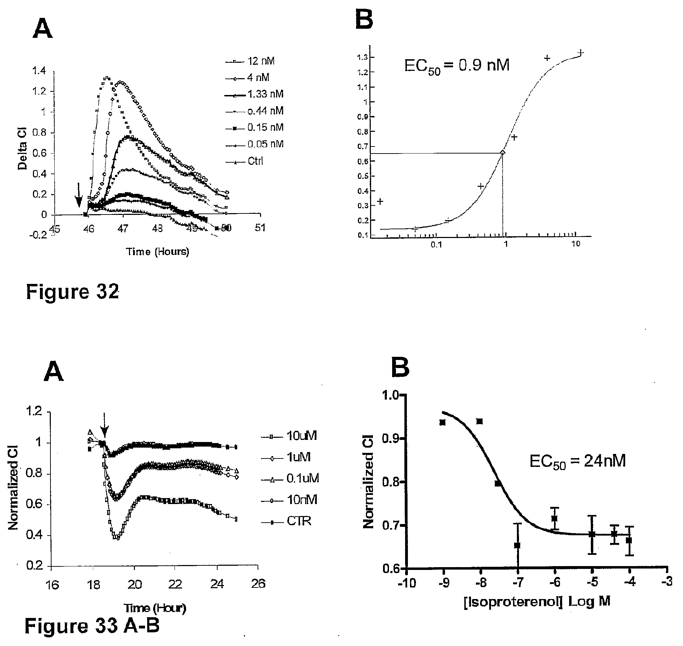

**Figure 32** depicts Endothelin 1-evoked increases of Cell Index (CI) of HeLa cells which express the endogenous endothelin receptors. HeLa cells were seeded at 50,000 cells per well of ACEA E-plates. The cells were continuously monitored using the RT-CES system. At the indicated time point, increasing concentrations of histamine were added to the cells and the cell response was monitored every 3 minutes by the RT-CES system. Endothelin 1 leads to a dose-dependent and transient increase in CI in HeLa cells shown in traces (A). Plotting the peak cell index response versus the corresponding log concentration allows for calculation of the $EC_{50}$ of endothelin 1 (B). Delta cell index shown in (A) at a given time point was calculated by subtracting the cell index at a standard time point from the cell index at the given time point. Here the standard time point was the last time point of the measurement before adding Endothelin to the cells (the first time point on the traces in A).

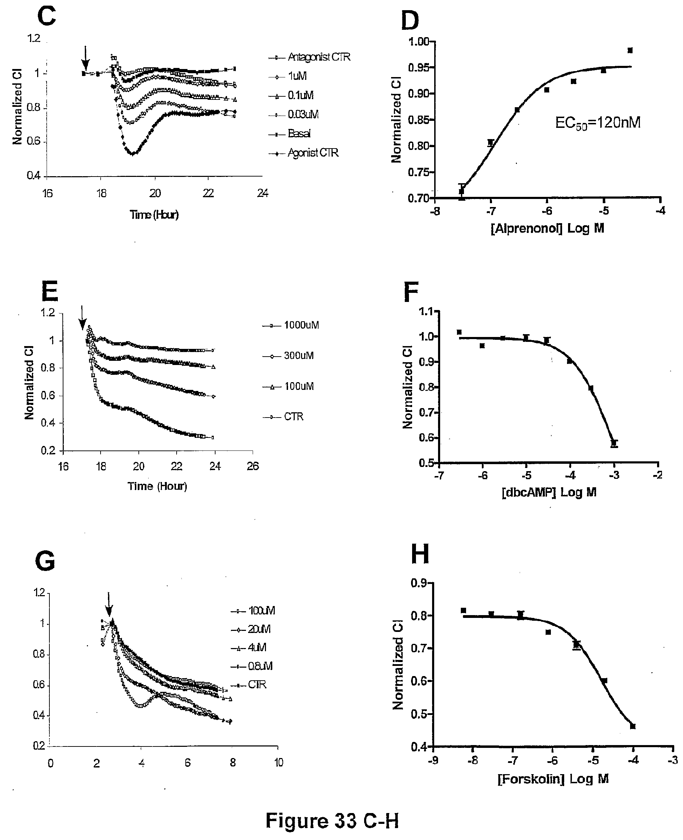

**Figure 33** depicts isoproterenol-evoked decreases of cell index (CI) Of C6 glioma cells which express the endogenous B-adrenergic receptors: Demonstration Of The Involvement Gs And Adenylate Cyclase. C6 cells were seeded at 50,000 cells per well of ACEA E-plates. The cells were continuously monitored using the RT-CES system. At the indicated time point, increasing concentrations of isoproterenol were added to the cells and the cell response was monitored every 3 minutes by the RT-CES system. Isoproterenol leads to a dose-dependent and transient decrease in CI in C6 cells shown in traces (A). Plotting the peak cell index response versus the corresponding log concentration allows for calculation of the $EC_{50}$ of isoproterenol (B). For the antagonist study, increasing concentrations of alprenolol were added to the cells 10 min prior to the application of a fixed concentration isoproterenol (10 uM) and the cell response was monitored every 3 minutes by the RT-CES system. alprenolol blocked isoproterenol-induced CI decreases in a concentration-dependant manner as shown in traces (C). Plotting the maximal blocking effects on cell index response versus the corresponding log concentration allows for calculation of the $IC_{50}$ of alprenolol (D). The effects of isoproterenol can be mimicked by dibutyl-cAMP, a cell membrane permeable analogue of cAMP, and forskolin, an adenylate cyclase activator, shown in traces (E,G) and curves (F, H), respectively. Normalized cell index shown in (A, C, E and G) at a given time point was calculated by dividing the Cell Index at the time point by the Cell Index at a reference time point. Here the reference time point was the last time point of the measurement before adding Isoproterenol to the cells.

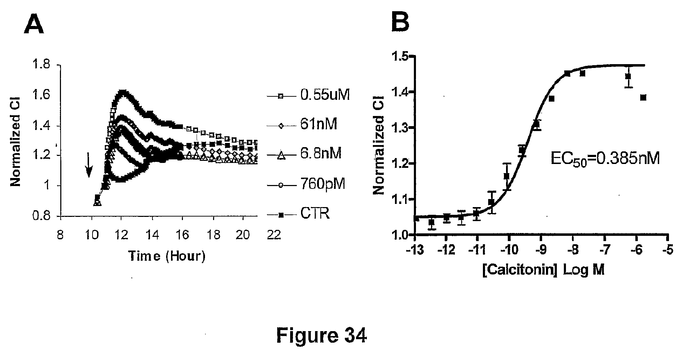

**Figure 34.** depicts Calcitonin-evoked increases of Cell Index (CI) of CHO cells which express the endogenous calcitonin receptors. CHO cells were seeded at 50,000 cells per well of ACEA E-plates. The cells were continuously monitored using the RT-CES system. At the indicated time point, increasing concentrations of calcitonin were added to the cells and the cell response was monitored every 3 minutes by the RT-CES system. Calcitonin leads to a dose-dependent and transient decrease in CI in CHO cells shown in traces (A). Plotting the peak cell index response versus the corresponding log concentration allows for calculation of the $EC_{50}$ (0.385 nM) of calcitonin (B). Normalized cell index shown in (A) at a given time point was calculated by dividing the Cell Index at the time point by the Cell Index at a reference time point. Here the reference time point was the last time point of the measurement before adding Calcitonin to the cells.

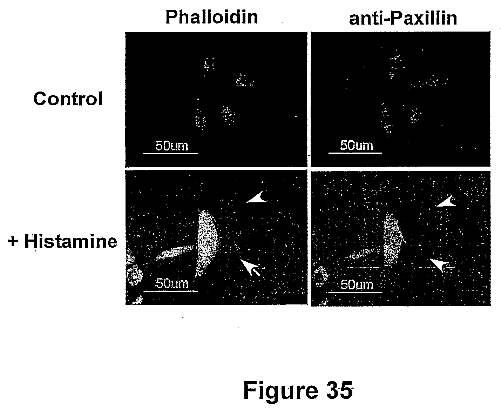

**Figure 35** depicts an example where Histamine induces focal adhesion assembly and membrane ruffling in hH1-C1 cells. The hH1-C1 cells were seeded on eight-well chamber slides with the density of 10,000 cells per well for one day. After an overnight incubation in serum-free medium, the cells were treated with 100 nM Histamine for 5 minutes. The cells were fixed, stained with FITC-Phalloidin and anti-Paxillin antibody and visualized by using an immunofluorescence microscope. Membrane ruffling can be observed (arrows).

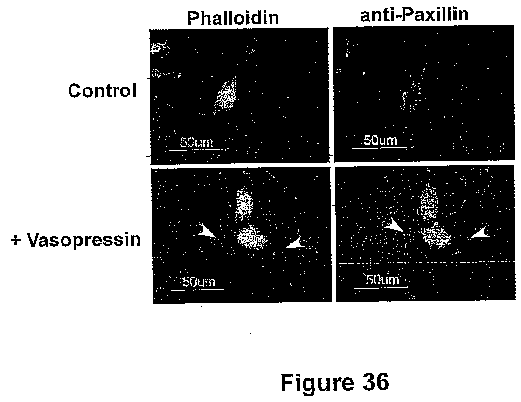

**Figure 36.** depicts an example where AVP induces focal adhesion assembly and membrane ruffling in V1a-C1 cells. The V1a-C1 cells were seeded on eight-well chamber slides with the density of 10,000 cells per well for one day. After an overnight incubation in serum-free medium, the cells were treated with 1 uM AVP for 5 minutes. The cells were fixed, stained with FITC-Phalloidin and anti-Paxillin antibody and visualized by using an immunofluorescence microscope. Membrane ruffling can be observed (arrows).

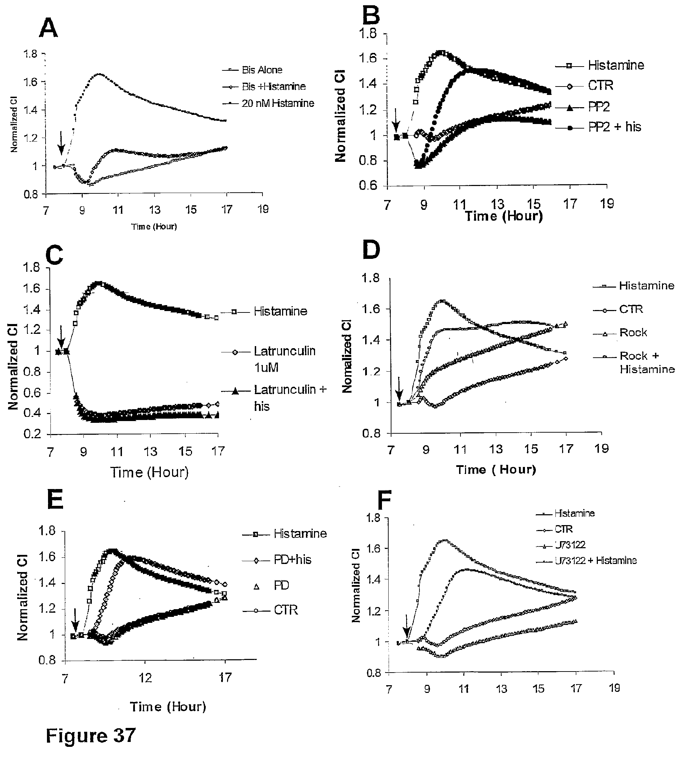

**Figure 37.** Mechanistic studies of Histamine-induced increases of CI in hH1-C1 cells by various reagents ranging from pathway blockers and toxins. Cell lines were seeded at 50,000 cells per well of ACEA E-plates. The cells were continuously monitored using the RT-CES system. Bisindolylmalemide (Bis, 10 uM), a PKC inhibitor (A); PP2 (10 uM), a Src inhibitor (B); latrunculin (1uM), a cell adhesion blocker (C); ROCK (10 uM), a Rho kinase inhibitor (D); PD98059 (PD, 10 uM), a MEK inhibitor (E) and U73122 (10uM), a PLC inhibitor (F) were added alone or 10 min before the addition of histamine (20nM).The cells were continuously monitored by the RT-CES system to the end of the experiment. Normalized CI traces are shown respectively. Normalized cell index shown in (A-F) at a given time point was calculated by dividing the Cell Index at the time point by the Cell Index at a reference time point. Here the reference time point was the last time point of the measurement before adding histamine (20 nM) to the cells.

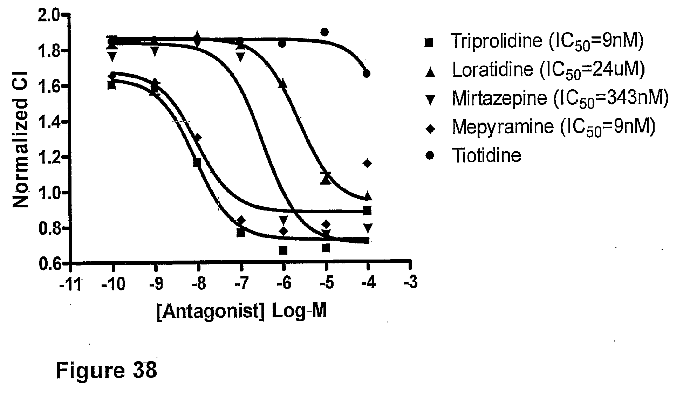

**Figure 38.** depicts an application of RT-CES System to rank the potency of selective H1 antagonists on hH1-C1 cells. A panel of selective H1 antagonists and H2 antagonist (for negative control purpose) were chosen to test on hH1-C1 cells by using RT-CES system. The cell lines were seeded at 50,000 cells per well of ACEA E-plates. The cells were continuously monitored using the RT-CES system. At the indicated time point, increasing concentrations of a given antagonists were added to the cells 10 min prior to the addition of a fixed concentration of histamine (20 nM)and the cell response was monitored every 3 minutes by the RT-CES system. Four chosen H1 antagonists (triprolidine, loratidine, mirtazepine and mepyramine) blocked histamine-induced CI increases in a concentration-dependant manner with different potencies as reflected by $IC_{50}$s indicated. H2 selective antagonist tiotidine did not block histamine-induced CI responses at the highest concentration (10 uM) tested.

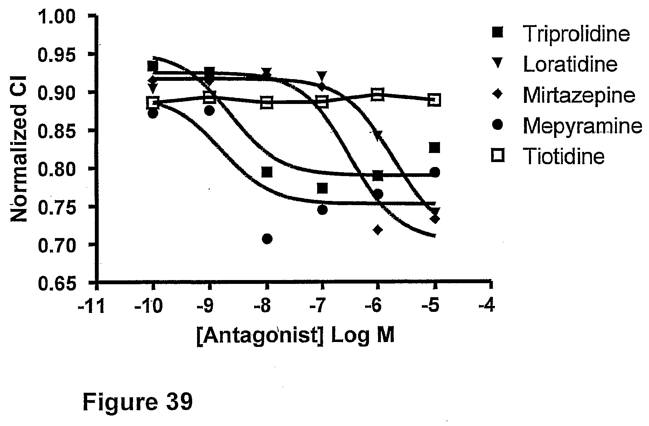

**Figure 39.** depicts an application Of RT-CES System to rank the potency of selective H1 antagonists as inverse agonists. The cell lines were seeded at 50,000 cells per well of ACEA E-plates. The cells were continuously monitored using the RT-CES system. Increasing concentrations of a given antagonist were added to the cells and the cell response was monitored every 3 minutes by the RT-CES system. Four chosen H1 antagonists (triprolidine, loratidine, mirtazepine and mepyramine) alone causes a concentration-dependent decreases of CI with different potencies as indicated $EC_{50s}$ and while H2 antagonist (tiotidine) did not induce any changes in CI at the highest concentration (10 uM) tested.

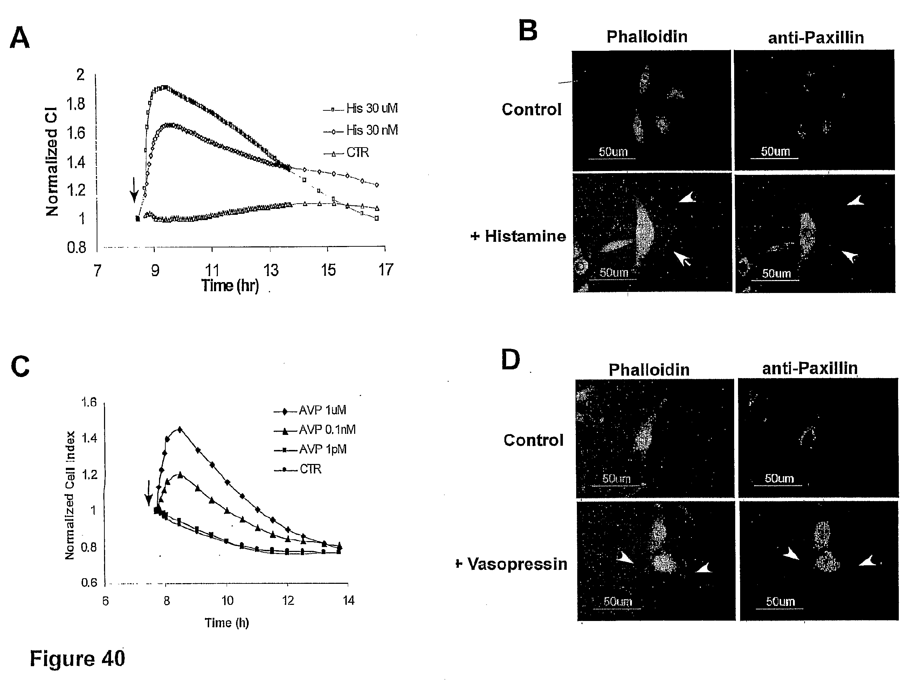

**Figure 40** depicts the characterization of activation of overexpressed GPCRs by RT-CES system. Human recombinant histamine 1 receptor (H1) and human recombinant vasopressin 1a (V1a) receptor (panel A and panel C, respectively) were seeded at 50,000 cells per well of ACEA E-plates. The cells were continuously monitored using the RT-CES system. At the indicated time point, increasing concentrations of histamine were added to the cells and the cell response was monitored every 3 min. A typical set of CI traces by H1 cell line and V1a cell line were shown. Arrows indicate the addition of the ligand. Plotting the peak cell index response versus the corresponding log concentration allows for calculation of the $EC_{50}$. Panel B demonstrates cell ruffling and cell adhesion enhancement after 5 min application of respective ligand. Histamine increase cell ruffling (c, arrow head) and cell adhesion assembly (d, arrow head) in H1 cells and AVP increase cell ruffling (g, arrow head) and cell adhesion assembly (h, arrow head) in V1a cell.

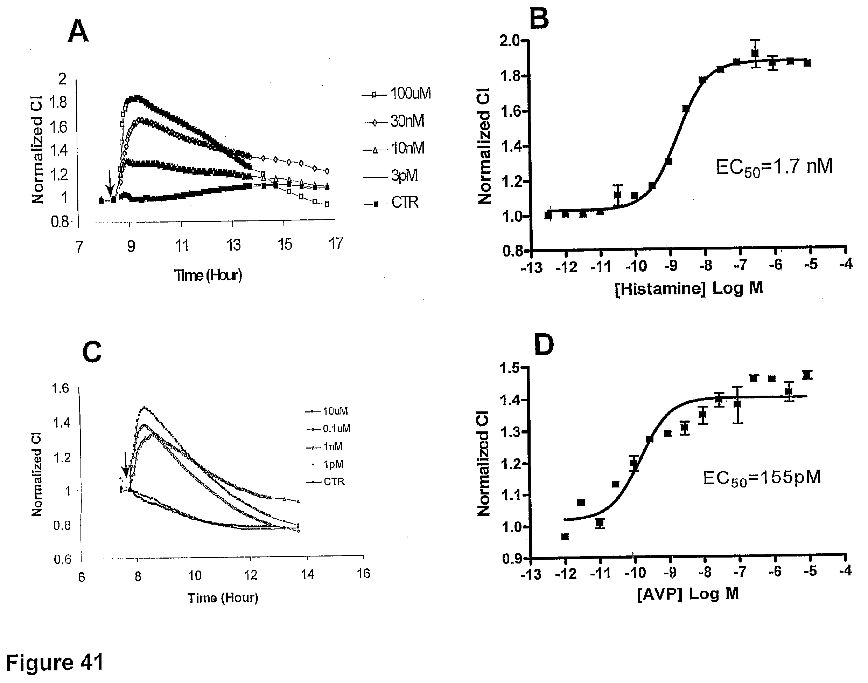

Figure 41 depicts a pharmacological study of H1 cell line and V1a cell line coupled to Gq: Generation of $EC_{50s}$. H1 or V1a cell lines were seeded at 50,000 cells per well of ACEA E-plates. The cells were continuously monitored using the RT-CES system. At the indicated time point (arrows), increasing concentrations of histamine (A) or AVP (C) were added to the cells and the cell response was monitored every 3 min by the RT-CES system. A typical set of CI traces were shown. Plotting the peak cell index responses versus the corresponding log concentrations allows for calculation of the $EC_{50}$ of histamine (B) and AVP (D). Normalized cell index shown in (A and C) at a given time point was calculated by dividing the Cell Index at the time point by the Cell Index at a reference time point. Here the reference time point was the last time point of the measurement before adding histamine (A) or AVP (C) to the cells.

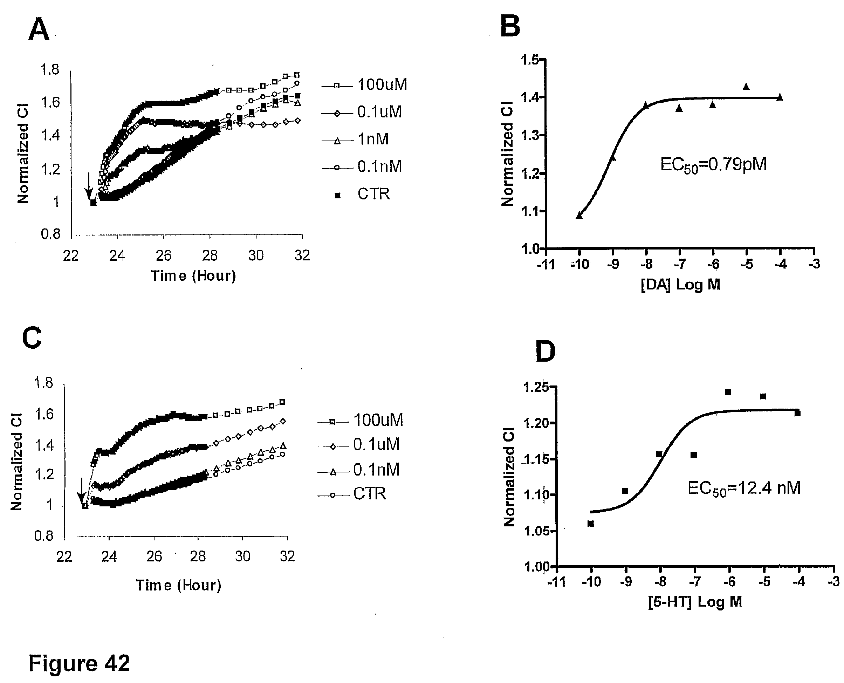

**Figure 42** depicts a pharmacological study of D1 cell line (Gs) and 5-HT1A cell line (Gi): Generation of $EC_{50s}$. D1 or 5-HT1A cell lines were seeded at 25,000 or 12,000cells per well of ACEA E-plates. The cells were continuously monitored using the RT-CES system. At the indicated time point (arrows), increasing concentrations of dopamine (A) or 5-HT (C) were added to the cells and the cell response was monitored every 3 min by the RT-CES system. CI traces were shown. Plotting the peak cell index responses versus the corresponding log concentrations allows for calculation of the $EC_{50s}$ of DA (B) and 5-HT (D). Normalized cell index shown in (A and C) at a given time point was calculated by dividing the Cell Index at the time point by the Cell Index at a reference time point. Here the reference time point was the last time point of the measurement before adding dopamine (A) or 5-HT (C) to the cells.

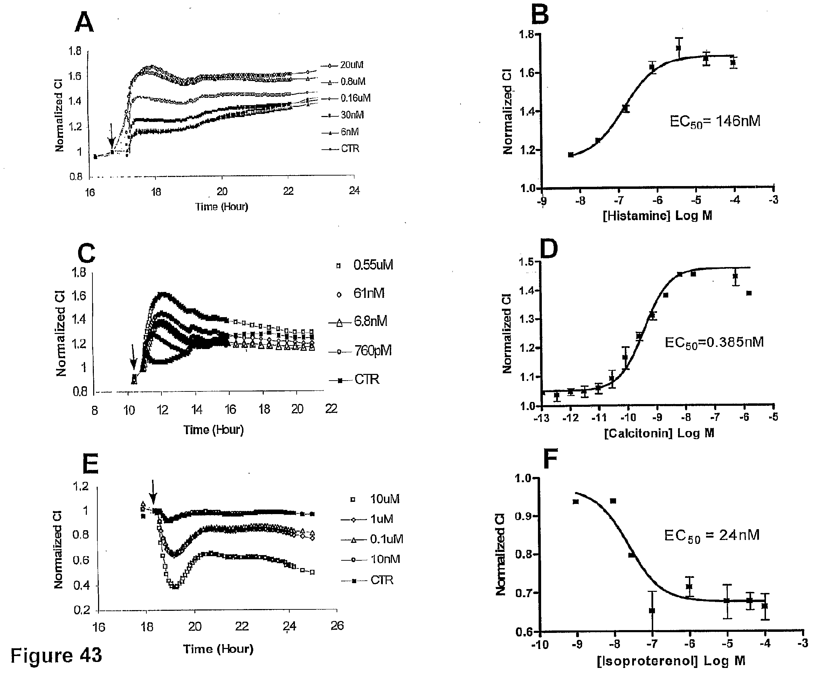

**Figure 43** depicts a pharmacological study of endogenous GPCRs by various ligands: Generation of $EC_{50s}$. HeLa cells (A) CHO cells (C) and C6 cells (E) were seeded at 50,000 cells per well of ACEA E-plates. The cells were continuously monitored using the RT-CES system. At the indicated time point (arrows), increasing concentrations of histamine (A) calcitonin (C) or isoproterenol (E) were added to the cells and the cell response was monitored every 3 min by the RT-CES system. Plotting the peak cell index responses versus the corresponding log concentrations allows for calculation of the $EC_{50s}$ of histamine (B) calcitonin (D) and isoproterenol (F) respectively. Normalized cell index shown in (A, C and E) at a given time point was calculated by dividing the Cell Index at the time point by the Cell Index at a reference time point. Here the reference time point was the last time point of the measurement before adding histamine (A) or calcitonin (C) or isoproterenol (E) to the cells.

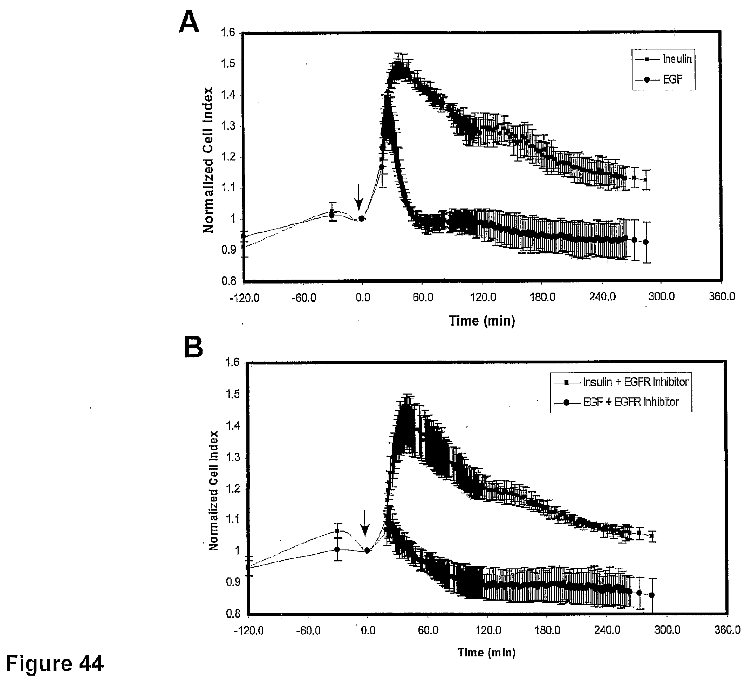

**Figure 44** depicts an assessment of specificity of cellular response to EGF and insulin treatments. COS7 cells were pretreated for 1 hour with either a specific EGFR inhibitor or vehicle. Cells were then stimulated with insulin or EGF. (A) Cells treated with insulin or EGF showed a characteristic rise in cell index. (B) When pretreated with 10 uM EGFR inhibitor, 4557W, the EGF response is inhibited while the insulin response remains intact. Normalized cell index shown in (A and B) at a given time point was calculated by dividing the Cell Index at the time point by the Cell Index at a reference time point. Here the reference time point was the last time point of measurement before treatment of the cells with EGF or insulin.

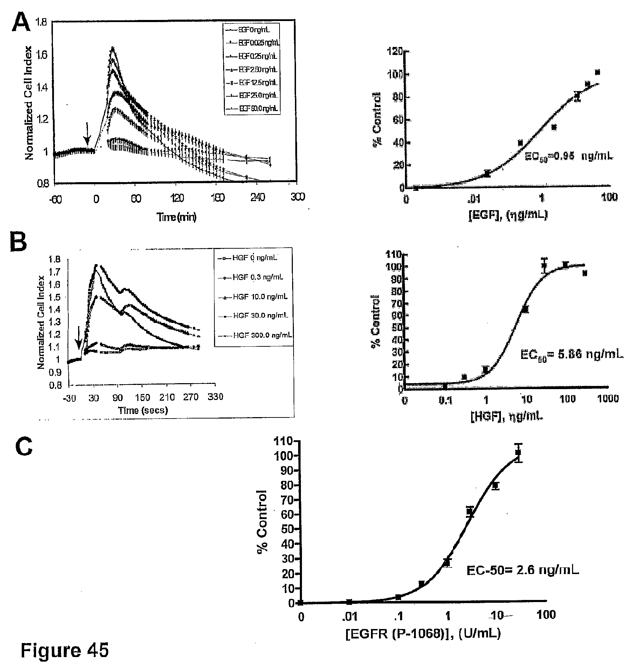

**Figure 45** depicts a characterization of COS7 cellular response to EGF and HGF treatments. Time-dependent normalized-cell-index traces are shown for COS7 cells treated with EGF (A) and HGF (B). Maximum cell indices (i.e., cell indexes) were determined from each trace corresponding to treatment of cells with one concentration of EGF or HGF and dose response curves were generated by plotting % control (relative to the normalized-cell-index response of the sample treated with maximum ligand concentration) versus ligand concentration. (C) ELISA assay of phosphorylated EGFR were performed on COS7 cells treated with varying concentrations of EGF. Dose response curves were generated by plotting % control (relative to the response of the sample treated with maximum ligand concentration) of absorbance readings versus ligand concentration. Normalized cell index shown in (A and B) at a given time point was calculated by dividing the Cell Index at the time point by the Cell Index at a reference time point. Here the reference time point was the last time point before adding EGF (A) or HGF (B).

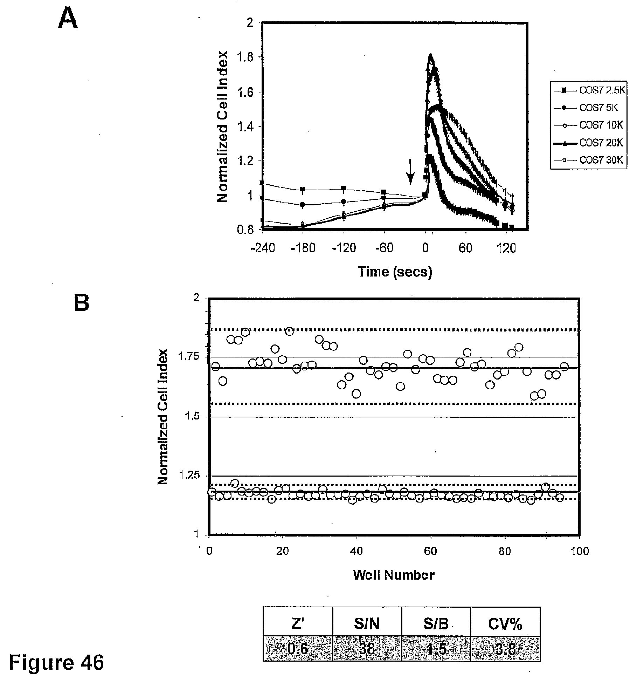

**Figure 46** depicts an optimization of assay conditions for screening of inhibitors against RTKs. (A) Increasing number of COS7 cells were plated, treated with EGF and cell indexes measured every minute over several hours. B) Statistical evaluation of label-free EGFR inhibitor screening assay. Z', S/N, S/B and % CV were determined to assess quality of assay.

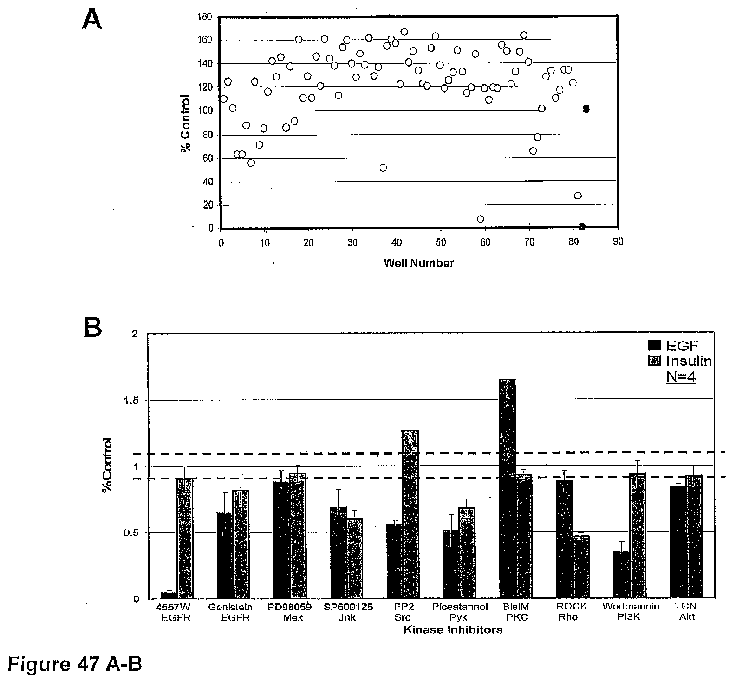

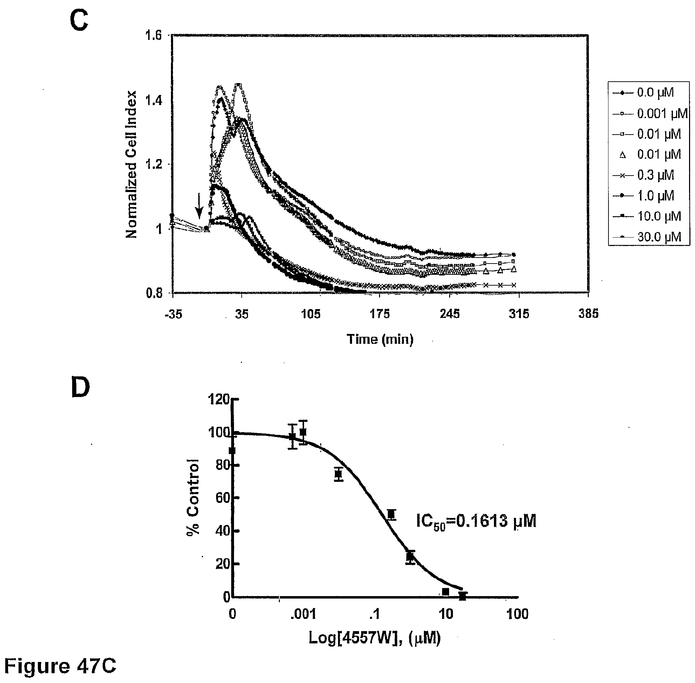

**Figure 47:** depicts (A) Graphical representation of a screen of 81 compounds mostly from Sigma's enzyme inhibitor Ligand Set. Compounds were screened in singlets at 5-10 uM concentrations. Red circle represents negative control and blue circle represents positive control. (B) A collection of kinase inhibitors were screened for inhibition of EGFR activity. (C) EGFRI, 4557W, was identified as potent inhibitor of EGFR signaling from both screens. Cellular response to EGF after pretreatment with varying concentration of inhibitor were measured and dose response curves generated by plotting % control of maximum cell index versus ligand concentration.

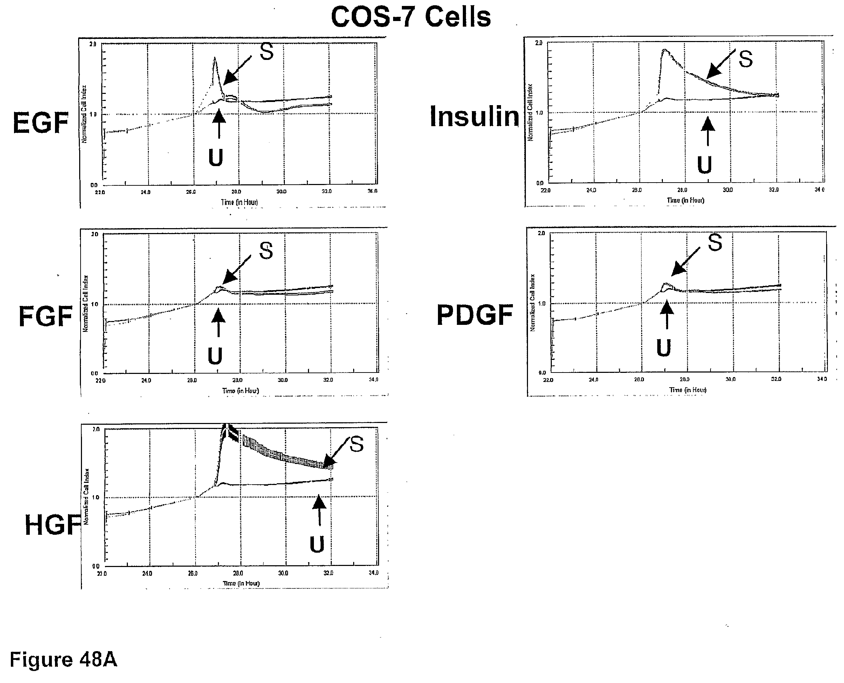

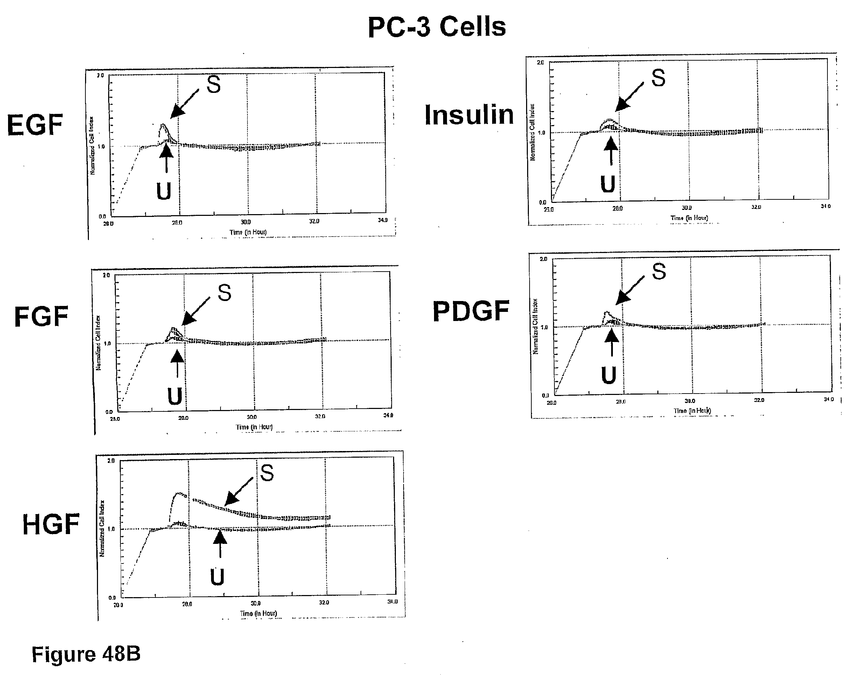

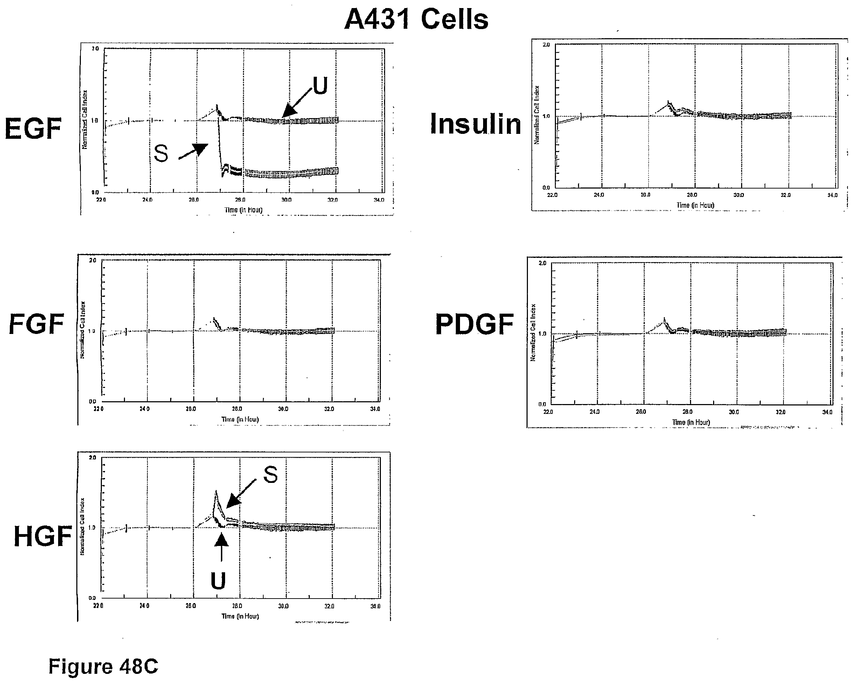

**Figure 48** depicts a comparison of unique signaling patterns of selected immortalized cell lines after treatment with various ligands for RTK. Cells were plated, serum starved and treated with ligands. Traces indicated with "S" represent cells treated or stimulated with ligand, and traces indicated with "U" represent cells treated with vehicle control (i.e., unstimulated). Response was measured every minute and data normalized to time point just before ligand addition. Error bars represent standard deviation of n=4. EGF =Epidermal Growth Factor; FGF = Fibroblast Growth Factor; HGF = Hepatocyte Growth Factor; PDGF = Platelet Derived Growth Factor.

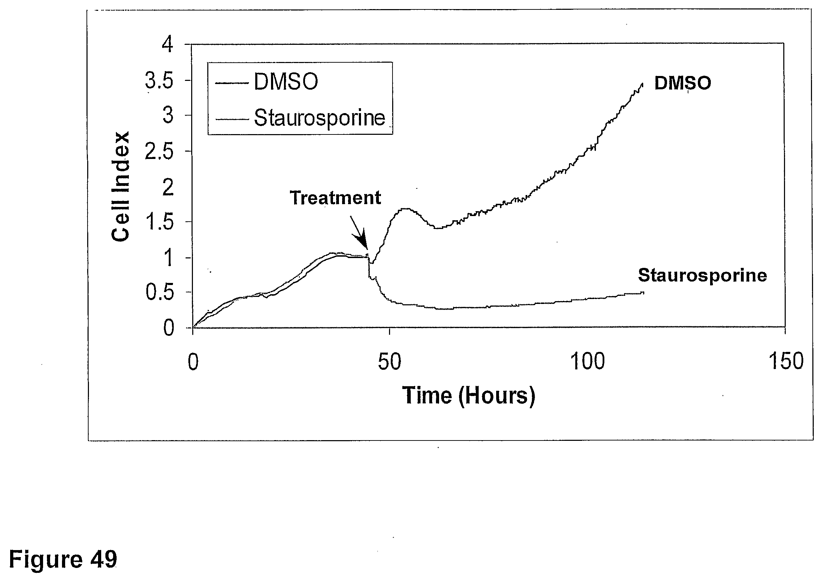

**Figure 49** depicts the monitoring of cancer cell proliferation and its inhibition by inhibitors of tyrosine kinases using the RT-CES system. MCF-7 breast cancer cells were seeded at a density of 8000 cells per well in ACEA e-plates. The cells were continuously monitored using the RT-CES system for about 48 hours at which time they were treated with staurosporine or DMSO as a control. Cellular proliferation was continually monitored by the RT-CES.

## DETAILED DESCRIPTION OF THE INVENTION

### A. Definitions

Unless defined otherwise, all technical and scientific terms used herein have the same meaning as is commonly understood by one of ordinary skill in the art to which this invention belongs.
As used herein, "a" or "an" means "at least one" or "one or more."
As used herein, "membrane" is a sheet of material.
As used herein, "biocompatible membrane" means a membrane that does not have deleterious effects on cells, including the viability, attachment, spreading, motility, growth, or cell division.
When a suspension of viable, unimpaired, epithelial or endothelial cells is added to a vessel, a surface of the vessel "is suitable for cell attachment" when a significant percentage of the cells are adhering to the surface of the vessel within twelve hours. Preferably, at least 50% of the cells are adhering to the surface of the vessel within twelve hours. More preferably, a surface that is suitable for cell attachment has surface properties so that at least 70% of the cells are adhering to the surface within twelve hours of plating (i.e., adding cells to the vessel). Even more preferably, the surface properties of a surface that is suitable for cell attachment results in at least 90% of the cells adhering to the surface within twelve hours of plating. Most preferably, the surface properties of a surface that is suitable for cell attachment results in at least 90% of the cells adhering to the surface within eight, six, four, two hours of plating. To have desired surface properties for cell attachment, the surface may need to chemically-treated (e.g. treatment with an acid and/or with a base), and/or physically treated (e.g. treatment with plasma), and/or biochemically treated (e.g. coated with one or more molecules or biomolecules that promotes cell attachment). A biocompatible surface (such as a membrane) preferably is suitable for the attachment of cells of the type that are to be used in an assay that uses the biocompatible surface (e.g., membrane), and most preferably, allows the attachment of at least 90% of the cells that contact the biocompatible surface during the assay.
A "biomolecular coating" is a coating on a surface that comprises a molecule that is a naturally occurring biomolecule or biochemical, or a biochemical derived from or based on one or more naturally occurring biomolecules or biochemicals. For example, a biomolecular coating can comprise an extracellular matrix component (e.g., fibronectin, collagens), or a derivative thereof, or can comprise a biochemical such as polylysine or polyornithine, which are polymeric molecules based on the naturally occurring biochemicals lysine and ornithine. Polymeric molecules based on naturally occurring biochemicals such as amino acids can use isomers or enantiomers of the naturally-occuring biochemicals.
An "extracellular matrix component" is a molecule that occurs in the extracellular matrix of an animal. It can be a component of an extracellular matrix from any species and from any tissue type. Nonlimiting examples of extracellular matrix components include laminins, collagens fibronectins, other glycoproteins, peptides, glycosaminoglycans, proteoglycans, etc. Extracellular matrix components can also include growth factors.
An "electrode" is a structure having a high electrical conductivity, that is, an electrical conductivity much higher than the electrical conductivity of the surrounding materials.
As used herein, an "electrode structure" refers to a single electrode, particularly one with a complex structure (as, for example, a spiral electrode structure), or a collection of at least two electrode elements that are electrically connected together. All the electrode elements within an "electrode structure" are electrically connected.
As used herein, "electrode element" refers to a single structural feature of an electrode structure, such as, for example, a fingerlike projection of an interdigitated electrode structure.
As used herein, an "electrode array" or "electrode structure unit" is two or more electrode structures that are constructed to have dimensions and spacing such that they can, when connected to a signal source, operate as a unit to generate an electrical field in the region of spaces around the electrode structures. Preferred electrode structure units measure impedance changes due to cell attachment to an electrode surface. Non-limiting examples of electrode structure units are interdigitated electrode structure units and concentric electrode structure units.
An "electrode bus" is a portion of an electrode that connects individual electrode elements or substructures. An electrode bus provides a common conduction path from individual electrode elements or individual electrode substructures to another electrical connection. In our devices an electrode bus can contact each electrode element of an electrode structure and provide an electrical connection path to electrical traces that lead to a connection pad.

"Electrode traces" or "electrically conductive traces" or "electrical traces", are electrically conductive paths that extend from electrodes or electrode elements or electrode structures toward one end or boundary of a device or apparatus for connecting the electrodes or electrode elements or electrode structures to an impedance analyzer. The end or boundary of a device may correspond to the connection pads on the device or apparatus.

A "connection pad" is an area on an apparatus or a device which is electrically connected to at least one electrode or all electrode elements within at least one electrode structure on an apparatus or a device and which can be operatively connected to external electrical circuits (e.g., an impedance measurement circuit or a signal source). The electrical connection between a connection pad and an impedance measurement circuit or a signal source can be direct or indirect, through any appropriate electrical conduction means such as leads or wires. Such electrical conduction means may also go through electrode or electrical conduction paths located on other regions of the apparatus or device.

"Interdigitated" means having projections coming one direction that interlace with projections coming from a different direction in the manner of the fingers of folded hands (with the caveat that interdigitated electrode elements preferably do not contact one another).

As used herein, a "high probability of contacting an electrode element" means that, if a cell is randomly positioned within the sensor area of a device or apparatus, the probability of a cell (or particle) contacting on an electrode element, calculated from the average diameter of a cell used on or in a device or apparatus, the sizes of the electrode elements, and the size of the gaps between electrode elements, is greater than about 50%, more preferably greater than about 60%, yet more preferably greater than about 70%, and even more preferably greater than about 80%, greater than about 90%, or greater than about 95%.

As used herein, "at least two electrodes fabricated on said substrate" means that the at least two electrodes are fabricated or made or produced on the substrate. The at least two electrodes can be on the same side of the substrate or on the different side of the substrate. The substrate may have multiple layers, the at least two electrodes can be either on the same or on the different layers of the substrate.

As used herein, "at least two electrodes fabricated to a same side of said substrate" means that the at least two electrodes are fabricated on the same side of the substrate.

As used herein, "at least two electrodes fabricated to a same plane of said substrate" means that, if the non-conducting substrate has multiple layers, the at least two electrodes are fabricated to the same layer of the substrate.

As used herein, "said... electrodes [or electrode structures] have substantially the same surface area" means that the surface areas of the electrodes referred to are not substantially different from each other, so that the impedance change due to cell attachment or growth on any one of the electrodes (or electrode structures) referred to will contribute to the overall detectable change in impedance to a same or similar degree as the impedance change due to cell attachment or growth on any other of the electrodes (or electrode structures) referred to. In other words, where electrodes (or electrode structures) have substantially the same surface area, any one of the electrodes can contribute to overall change in impedance upon cell attachment or growth on the electrode. In most cases, the ratio of surface area between the largest electrode and the smallest electrode that have "substantially the same surface area" is less than 10. Preferably, the ratio of surface area between the largest electrode and the smallest electrode of an electrode array is less than 5, 4, 3, 2, 1.5, 1.2 or 1.1. More preferably, the at least two electrodes of an electrode structure have nearly identical or identical surface area.

As used herein, "said device has a surface suitable for cell attachment or growth" means that the electrode and/or non-electrode area of the apparatus has appropriate physical, chemical or biological properties such that cells of interest can viably attach on the surface and new cells can continue to attach, while the cell culture grows, on the surface of the apparatus. However, it is not necessary that the device, or the surface thereof, contain substances necessary for cell viability or growth. These necessary substances, e.g., nutrients or growth factors, can be supplied in a medium. Preferably, when a suspension of viable, unimpaired, epithelial or endothelial cells is added to the "surface suitable for cell attachment" when at least 50% of the cells are adhering to the surface within twelve hours. More preferably, a surface that is suitable for cell attachment has surface properties so that at least 70% of the cells are adhering to the surface within twelve hours of plating (i.e., adding cells to the chamber or well that comprises the said device). Even more preferably, the surface properties of a surface that is suitable for cell attachment results in at least 90% of the cells adhering to the surface within twelve hours of plating. Most preferably, the surface properties of a surface that is suitable for cell attachment results in at least 90% of the cells adhering to the surface within eight, six, four, two hours of plating.

As used herein, "detectable change in impedance between or among said electrodes" (or "detectable change in impedance between or among said electrode structures") means that the impedance between or among said electrodes (or electrode structures) would have a significant change that can be detected by an impedance analyzer or impedance measurement circuit when molecule binding reaction occurs on the electrode surfaces. The impedance change refers to the difference in impedance values when molecule binding reaction occurs on the electrode surface of the apparatus and when no molecular reaction occurs on the electrode surface. Alternatively, the impedance change refers to the difference in impedance values when cells are attached to the electrode surface and when cells are not attached to the electrode surface, or when the number, type, activity, adhesiveness, or morphology of cells attached to the electrode-comprising surface of the apparatus changes. In most cases, the change in impedance is larger than 0.1% to be detectable. Preferably, the detectable change in impedance is larger than 1%, 2%, 5%, or 8%. More preferably, the detectable change in impedance is larger than 10%. Impedance between or among electrodes is typically a function of the frequency of the applied electric field for measurement. "Detectable change in impedance between or among said electrodes" does not require the impedance change at all frequencies being detectable. "Detectable change in impedance between or among said electrodes" only requires a detectable change in impedance at any single frequency (or multiple frequencies). In addition, impedance has two components, resistance and reactance (reactance can be divided into two categories, capacitive reactance and inductive reactance). "Detectable change in impedance between or among said electrodes" requires only that either one of resistance and reactance has a detectable change at any single frequency or multiple frequencies. Impedance refers to the electrical or electronic impedance. The method for the measurement of such impedance is achieved by, (1) applying a voltage between or among said electrodes at a given frequency (or multiple frequencies, or having specific voltage waveform) and monitoring the electrical current through said electrodes at the frequency (or multiple frequencies, or having specific waveform), dividing the voltage amplitude value by the current amplitude value to derive the impedance value; (2) applying an electric current of a single frequency component (or multiple frequencies or having specific current wave form) through said electrodes and monitoring the voltage resulted between or among said electrodes at the frequency (or multiple frequencies, or having specific waveform), dividing the voltage amplitude value by the current amplitude value to derive the impedance value; (3) other methods that can measure or determine electric impedance. Note that in the description above of "dividing the voltage amplitude value by the current amplitude value to derive the impedance value", the "division" is done for the values of current amplitude and voltage amplitude at same frequencies. Measurement of such electric impedance is an electronic or electrical process that does not involve the use of any reagents.

As used herein, "said at least two electrodes have substantially different surface area" means that the surface areas of any electrodes are not similar to each other so that the impedance change due to cell attachment or growth on the larger electrode will not contribute to the overall detectable impedance to a same or similar degree as the impedance change due to cell attachment or growth on the smaller electrodes.

Preferably, any impedance change due to cell attachment or growth on the larger electrode is significantly smaller than the impedance change due to cell attachment or growth on the smaller electrode. Ordinarily, the ratio of surface area between the largest electrode and the smallest electrode is more than 10. Preferably, the ratio of surface area between the largest electrode and the smallest electrode is more than 20, 30, 40, 50 or 100.

As used herein, "multiple pairs of electrodes or electrode structures spatially arranged according to wells of a multi-well microplate" means that the multiple pairs of electrodes or electrode structures of a device or apparatus are spatially arranged to match the spatial configuration of wells of a multi-well microplate so that, when desirable, the device can be inserted into, joined with, or attached to a multiwell plate (for example, a bottomless multiwell plate) such that multiple wells of the multi-well microplate will comprise electrodes or electrode structures.

As used herein, "arranged in a row-column configuration" means that, in terms of electric connection, the position of an electrode, an electrode array or a switching circuit is identified by both a row position number and a column position number.

As used herein, "each well contains substantially same number ... of cells" means that the lowest number of cells in a well is at least 50% of the highest number of cells in a well. Preferably, the lowest number of cells in a well is at least 60%, 70%, 80%, 90%, 95% or 99% of the highest number of cells in a well. More preferably, each well contains an identical number of cells.

As used herein, "each well contains ...same type of cells" means that, for the intended purpose, each well contains same type of cells; it is not necessary that each well contains exactly identical type of cells. For example, if the intended purpose is that each well contains mammalian cells, it is permissible if each well contains same type of mammalian cells, *e.g.,* human cells, or different mammalian cells, *e.g.,* human cells as well as other non-human mammalian cells such as mice, goat or monkey cells, etc.

As used herein, "each well contains ... serially different concentration of a test compound" means that each well contains a test compound with a serially diluted concentrations, e.g., an one-tenth serially diluted concentrations of 1 M, 0.1 M, 0.01 M, etc.

As used herein, "dose-response curve" means the dependent relationship of response of cells on the dose concentration of a test compound. The response of cells can be measured by many different parameters. For example, a test compound is suspected to have cytotoxicity and cause cell death. Then the response of cells can be measured by percentage of non-viable (or viable) cells after the cells are treated by the test compound. Plotting this percentage of non-viable (or viable) cells as a function of the dose concentration of the test compound constructs a dose response curve. The percentage of non-viable (or viable) cells can be expressed in terms of cell index derived from impedance measurement, or in terms of cell change indexes. For example, for a give cell type and under specific cellular physiological condition (e.g., a particular cell culture medium), cell index can be shown to have a linear correlation or positive correlation with the number of viable cells in a well from which cell index was derived from the impedance measurement. Thus, one can plot cell index as a function of the dose concentration of the test compound to construct a "dose-response curve". Note that, generally, cell index not only correlate with the number of viable cells in the wells but also relate to the cell morphology and cell attachment. Thus plotting cell index versus dose concentration provides information not only about number of cells but also about their physiological status (e.g. cell morphology and cell adhesion). Furthermore, an important advantage offered by our system and devices is that in a single experiment, one can obtain "dose-response curves" at multiple time points since the system allows for the continuous monitoring of cells and provides impedance measurement at many time points over a time range as short as a few minutes to as long as days or weeks. In another example, a test compound may result in a change in cell morphology, which can be monitored or measured by cell-substrate impedance. Thus, cell-substrate impedance and cell index may follow a dose-dependent relationship on the concentration of the test compound. One may construct dose-response by plotting the maximum change in cell index after adding the test compound at each compound concentration to the cells with respect to the cell index prior to the addition of the test compound. From such dose-response curve, one may derive important parameters such as EC50 or IC50 of the test compound.

As used herein, "the electrodes have, along the length of the microchannel, a length that is substantially less than the largest single-dimension of a particle to be analyzed" means that the electrodes have, along the length of the microchannel, a length that is at least less than 90% of the largest single-dimension of a particle to be analyzed. Preferably, the electrodes have, along the length of the microchannel, a length that is at least less than 80%, 70%, 60%, 50%, 40%, 30%, 20%, 10%, 5% of the largest single-dimension of a particle to be analyzed.

As used herein, "the microelectrodes span the entire height of the microchannel" means that the microelectrodes span at least 70% of the entire height of the microchannel. Preferably, microelectrodes span at least 80%, 90%, 95% of the entire height of the microchannel. More preferably, microelectrodes span at least 100% of the entire height of the microchannel.

As used herein, "an aperture having a pore size that equals to or is slightly larger than size of said particle" means that aperture has a pore size that at least equals to the particle size but less than 300% of the particle size. Here both pore size and particle size are measured in terms of single dimension value.

As used herein, "microelectrode strip or electrode strip" means that a non-conducting substrate strip on which electrodes or electrode structure units are fabricated or incorporated. The non-limiting examples of the non-conducting substrate strips include polymer membrane, glass, plastic sheets, ceramics, insulator-on-semiconductor, fiber glass (like those for manufacturing printed-circuits-board). Electrode structure units having different geometries can be fabricated or made on the substrate strip by any suitable microfabrication, micromachining, or other methods. Non-limiting examples of electrode geometries include interdigitated electrodes, circle-on-line electrodes, diamond-on-line electrodes, castellated electrodes, or sinusoidal electrodes. Characteristic dimensions of these electrode geometries may vary from as small as less than 5 micron, or less than 10 micron, to as large as over 200 micron, over 500 micron, over 1 mm. The characteristic dimensions of the electrode geometries refer to the smallest width of the electrode elements, or smallest gaps between the adjacent electrode elements, or size of a repeating feature on the electrode geometries. The microelectrode strip can be of any geometry. One exemplary geometry for the microelectrode strips is rectangular shape-having the width of the strip between less than 50 micron to over 10 mm, and having the length of the strip between less than 60 micron to over 15 mm. An exemplary geometry of the microelectrode strips may have a geometry having a width of 200 micron and a length of 20 mm. A single microelectrode strip may have two electrodes serving as a measurement unit, or multiple such two-electrodes serving as multiple measurement units, or a single electrode structure unit as a measurement unit, or multiple electrode structure units serving as multiple electrode structure units. In one exemplary embodiment, when multiple electrode structure units are fabricated on a single microelectrode strip, these electrode structure units are positioned along the length direction of the strip. The electrode structure units may be of squared-shape, or rectangular-shape, or circle shapes. Each of electrode structure units may occupy size from less than 50 micron by 50 micron, to larger than 2 mm x 2mm.

As used herein, "sample" refers to anything which may contain a moiety to be isolated, manipulated, measured, quantified, detected or analyzed using apparatuses, microplates or methods in the present application. The sample may be a biological sample, such as a biological fluid or a biological tissue. Examples of biological fluids include suspension of cells in a medium such as cell culture medium, urine, blood, plasma, serum, saliva, semen, stool, sputum, cerebral spinal fluid, tears, mucus, amniotic fluid or the like. Biological tissues are aggregates of cells, usually of a particular kind together with their intercellular substance that form one of the structural materials of a human, animal, plant, bacterial, fungal or viral structure, including connective, epithelium, muscle and nerve tissues. Examples of biological tissues also include organs, tumors, lymph nodes, arteries and individual cell(s). The biological samples may further include cell suspensions, solutions containing biological molecules (e.g. proteins, enzymes, nucleic acids, carbohydrates, chemical molecules binding to biological molecules).

As used herein, a "liquid (fluid) sample" refers to a sample that naturally exists as a liquid or fluid, e.g., a biological fluid. A "liquid sample" also refers to a sample that naturally exists in a non-liquid status, *e.g.,* solid or gas, but is prepared as a liquid, fluid, solution or suspension containing the solid or gas sample material. For example, a liquid sample can encompass a liquid, fluid, solution or suspension containing a biological tissue.

A "compound" or "test compound" is any compound whose activity or direct or indirect effect or effects on cells is investigated in any assay. A test compound can be any compound, including, but not limited to, a small molecule, a large molecule, a molecular complex, an organic molecule, an inorganic molecule, a biomolecule such as but not limited to a lipid, a steroid, a carbohydrate, a fatty acid, an amino acid, a peptide, a protein, an antibody, a nucleic acid, or any combination of these. A test compound can be a synthetic compound, a naturally occurring compound, a derivative of a naturally-occurring compound, etc. The structure of a test compound can be known or unknown. In one application, a compound is capable of, or is suspected of, inducing cytotoxicity. In another application, a compound is capable of, or is suspected of, stimulating effector cells. In still another application, a compound is capable of, or is suspected of, interacting with cells (for example, binding to cell surface receptor, or inhibiting certain intracellular signal transduction pathway, or activating cells).

A "known compound" is a compound for which at least one activity is known. A known compound preferably is a compound for which one or more direct or indirect effects on cells is known. Preferably, the structure of a known compound is known, but this need not be the case. Preferably, the mechanism of action of a known compound on cells is known, for example, the effect or effects of a known compound on cells can be, as nonlimiting examples, effects on cell viability, cell adhesion, apoptosis, cell differentiation, cell proliferation, cell morphology, cell cycle, IgE-mediated cell activation or stimulation, receptor-ligand binding, cell number, cell quality, cell cycling, etc.

An "impedance value" is the impedance measured for electrodes in a well with or without cell present. Impedance is generally a function of the frequency, i.e., impedance values depend on frequencies at which the measurement was conducted. For the present application, impedance value refers to impedance measured at either single frequency or multiple frequencies. Furthermore, impedance has two components, one resistance component and one reactance component. Impedance value refers to resistance component, or reactance component, or both resistance and reactance component. Thus, when "impedance value" was measured or monitored, we are referring to that, resistance, or reactance, or both resistance and reactance were measured or monitored. In many embodiments of the methods of the present application, impedance values also refer to parameter values that are derived from raw, measured impedance data. For example, cell index, or normalized cell index, or delta cell index could be used to represent impedance values.

A "Cell Index" or "CI" is a parameter that can derived from measured impedance values and that can be used to reflect the change in impedance values. There are a number of methods to derive or calculate Cell Index.

A "Normalized Cell Index" at a given time point is calculated by dividing the Cell Index at the time point by the Cell Index at a reference time point. Thus, the Normalized Cell Index is 1 at the reference time point.

A "delta cell index" at a given time point is calculated by subtracting the cell index at a standard time point from the cell index at the given time point. Thus, the delta cell index is the absolute change in the cell index from an initial time (the standard time point) to the measurement time.

A "Cell Change Index" or "CCI" is a parameter derived from Cell Index and "CCI" at a time point is equal to the $1^{st}$ order derive of the Cell Index with respect to time, divided by the Cell Index at the time point. In other words, CCI is calculated as

$$CCI\ (t) = \frac{dCI\ (t)}{CI\ (t) \cdot dt}.$$

## B. Devices and systems for monitoring cell-substrate impedance

<u>Devices for Measuring Cell-Substrate Impedance</u>

Our devices for measuring cell-substrate impedance comprise a nonconducting substrate; two or more electrode arrays fabricated on the substrate, where each of the two or more electrode arrays comprises two electrode structures; and at least two connection pads, each of which is located on an edge of the substrate. Each electrode array of the device has approximately uniform electrode resistance across the entire array. The substrate of the device has a surface suitable for cell attachment or growth; where cell attachment or growth on said substrate can result in a detectable change in impedance between or among the electrode structures within each electrode array.

An electrode array is two or more electrode structures that are constructed to have dimensions and spacing such that they can, when connected to a signal source, operate as a unit to generate an electrical field in the region of spaces around the electrode structures. An electrode structure refers to a single electrode, particularly one with a complex structure. (For example, an electrode structure can comprise two or more electrode elements that are electrically connected together.) In our devices an electrode array comprises two electrode structures, each of which comprises multiple electrode elements, or substructures. In preferred embodiments, the electrode structures of each of the two or more electrode arrays of a device have substantially the same surface area. In preferred embodiments of our device each of the two or more electrode arrays of a device comprise two electrode structures, and each electrode structure comprises multiple electrode elements. Each of the two electrode structures of an electrode array is connected to a separate connection pad that is located at the edge of the substrate.

Thus, in our devices for each of the two or more electrode arrays of the device, the first of the two electrode structures is connected to one of the two or more connection pads, and the second of the two electrode structures is connected to another of the two or more connection pads. Preferably, each array of a device is individually addressed, meaning that the electrical traces and connection pads of the arrays are configured such that an array can be connected to an impedance analyzer in such a way that a measuring voltage can be applied across a single array at a given time by using switches (such as electronic switches).

Each electrode array of the device has an approximately uniform electrode resistance distribution across the entire array. By "uniform resistance distribution across the array" is meant that when a measurement voltage is applied across the electrode structures of the array, the electrode resistance at any given location of the array is approximately equal to the electrode resistance at any other location on the array. Preferably, the electrode resistance at a first location on an array of the device and the electrode resistance at a second location on the same array does not differ by more than 30%. More preferably, the electrode resistance at a first location on an array of the device and the electrode resistance at a second location on the same array does not differ by more than 15%. Even more preferably, the electrode resistance at a first location on an array of the device and a second location on the same array does not differ by more than 5%. More preferably yet, the electrode resistance at a first location on an array of the device and a second location on the same array does not differ by more than 2%.

Preferred arrangements for the electrode elements, gaps between the electrodes and electrode buses in a given electrode array are used to allow all cells, no matter where they land and attach to the electrode surfaces, to contribute similarly to the total impedance change measured for the electrode array. Thus, it is desirable to have similar electric field strengths at any two locations within any given array of the device when a measurement voltage is applied to the electrode array. At any given location of the array, the field strength is related to the potential difference between the nearest point on a first electrode structure of the array and the nearest point on a second electrode structure of the array. It is therefore desirable to have similar electric potential drops across the electrode elements and across the electrode buses of a given array. Based on this requirement, it is preferred to have an approximately uniform electrode resistance distribution across the whole array where the electrode resistance at a location of interest is equal to the sum of the electrode resistance between the nearest point on a first electrode structure (that is the point on the first electrode structure nearest the location of interest) and a first connection pad connected to the first electrode structure and the electrode resistance between the nearest point on a second electrode structure (that is the point on the first electrode structure nearest the location of interest) and a second connection pad connected to the second electrode structure.

Our devices are designed such that the arrays of the device have an approximately uniform distribution across the whole array. This can be achieved, for example, by having electrode structures and electrode buses of particular spacing and dimensions (lengths, widths, thicknesses and geometrical shapes) such that the resistance at any single location on the array is approximately equal to the resistance at any single other location on the array. In most embodiments, the electrode elements (or electrode structures) of a given array will have even spacing and be of similar thicknesses and widths, the electrode buses of a given array will be of similar thicknesses and widths, and the electrode traces leading from a given array to a connection pad will be of closely similar thicknesses and widths. Thus, in these preferred embodiments, an array is designed such that the lengths and geometrical shapes of electrode elements or structures, the lengths and geometrical shapes of electrode traces, and the lengths and geometrical shapes of buses allow for approximately uniform electrode resistance distribution across the array.

Electrode structures preferably comprise multiple electrode elements, and each electrode element connects directly to an electrode bus. Electrode elements of a first electrode structure connect to a first electrode bus, and electrode elements of a second electrode structure connect to a second electrode bus. In these embodiments, each of the two electrode buses connects to a separate connection pad via an electrical trace. Although the resistances of the traces contribute to the resistance at a location on the array, for any two locations on the array the trace connections from the first bus to a first connection pad and from the second bus to a second connection pad are identical. Thus, in these preferred embodiments trace resistances do not need to be taken into account in designing the geometry of the array to provide for uniform resistances across the array.

In preferred embodiments the device for monitoring cell-substrate impedance has two or more electrode arrays that share a connection pad. Preferably one of the electrode structures of at least one of the electrode arrays of the device is connected to a connection pad that also connects to an electrode structure of at least one other of the electrode arrays of the device. Preferably for at least two arrays of the device, each of the two or more arrays has a first electrode structure connected to a connection pad that connects with an electrode structure of at least one other electrode array, and each of the two or more arrays has a second electrode structure that connects to a connection pad that does not connect with any other electrode structures or arrays of the device. Thus, in preferred designs there are at least two electrode arrays each of which has a first electrode structure that is connected to a common connection pad and a second electrode structure that is connected to an independent connection pad.

In some preferred embodiments each of the electrode structures of an array is connected to an electrode bus that is connected to one of the two or more connection pads of the device via an electrically conductive trace. In preferred embodiments, each of the two electrode structures is connected to a single bus, such that each array connects to two buses, one for each electrode structures. In this arrangement, each of the two buses connects to a separate connection pad of the substrate.

The electrically conductive traces that connect a bus with a connection can be fabricated of any electrically conductive material. The traces can be localized to the surface of the substrate, and can be optionally covered with an insulating layer. Alternatively the traces can be disposed in a second plane of the substrate. Description of arrangements and design of electrically conductive traces on impedance measurement devices can be found in U.S. Patent 7,470,533.

Appropriate electronic connection means such as metal clips engaged onto the connection pads on the substrate and connected printed-circuit-boards can be used for leading the electronic connections from the connection pads on the devices to external electronic circuitry (e.g. an impedance analyzer). Description of the design of cell-substrate impedance devices and their manufacture can be found in U.S. Patent 7,470,533.

Preferably the nonconducting substrate is planar, and is flat or approximately flat. Exemplary substrates can comprise many materials, including, but not limited to, silicon dioxide on silicon, silicon-on-insulator (SOI) wafer, glass (e.g., quartz glass, lead glass or borosilicate glass), sapphire, ceramics, polymer, fiber glass, plastics, e.g., polyimide (e.g. Kapton, polyimide film supplied by DuPont), polystyrene, polycarbonate, polyvinyl chloride, polyester, polypropylene and urea resin. Preferably, the substrate and the surface of the substrate are not going to interfere with molecular binding reactions that will occur at the substrate surface. For cell-substrate impedance monitoring, any surface of the nonconducting substrate that can be exposed to cells during the use of a device is preferably biocompatible. Substrate materials that are not biocompatible can be made biocompatible by coating with another material, such as polymer or biomolecular coating.

All or a portion of the surface of a substrate can be chemically treated, including but not limited to, modifying the surface such as by addition of functional groups, or addition of charged or hydrophobic groups.

Descriptions of electrode arrays used for impedance measurement that apply to our devices are described in U.S. Patent 7,470,533.

Preferred electrode arrays for devices include arrays comprising two electrode structures, such as, for example, spiral electrode arrays and interdigitated arrays. In some preferred devices, electrode arrays are fabricated on a substrate, in which the arrays comprises two electrode structures, each of which comprises multiple circle-on-line electrode elements, in which the electrode elements of one structure alternate with the electrode elements of the opposite electrode structure.

Preferably, the electrode elements (or electrode structures) of an array of the device are of approximately equal widths. Preferably the electrode elements (or electrode structures) of an array of the device are greater than 30 microns in width, more preferably from about 50 to about 300 microns in width, and more preferably yet about 90 microns in width.

Preferably, the electrode elements (or electrode structures) of an array of the device are approximately evenly spaced. Preferably, the gap between electrode elements (or electrode structures) of an array of the device is less than 50 microns in width, more preferably from about 5 to about 30 microns in width, and more preferably yet about 20 microns in width.

Our device can include one or more fluid-impermeable receptacles which serve as fluid containers. Such receptacles may be reversibly or irreversibly attached to or formed within the substrate or portions thereof (such as, for example, wells formed as in a microtiter plate). In another example, the device includes microelectrode strips reversibly or irreversibly attached to plastic housings that have openings that correspond to electrode structure units located on the microelectrode strips. Suitable fluid container materials comprise plastics, glass, or plastic coated materials such as ceramics, glass, metal, etc. Descriptions and disclosure of devices that comprise fluid containers can be found in U.S. Patent 7,470,533.

In preferred embodiments, each electrode array on the substrate of our device is associated with a fluid-impermeable container or receptacle, such as, for example, a well. Preferably, the device is assembled to a bottomless, multiwell plastic plate or strip with a fluid tight seal. The device is assembled such that a single array of the substrate is at the bottom of a receptacle or well. Preferably, each array of a device is associated with a well of a multiwell plate.

The design and assembly of multiwell impedance measurement devices is described in U.S. Patent 7,470,533, and also in U.S. Patent 7,195,752. Our device preferably has between 2 and 1,536 wells, more preferably between 4 and 384 wells, and even more preferably, between 16 and 96 wells, all or less than all or which are associated with electrode arrays.

Commercial tissue culture plates can be adapted to fit our device. Bottomless plates may also be custom-made to preferred dimensions. Preferably, well diameters are from about 1 millimeter to about 20 millimeters, more preferably from about 2 millimeters to about 8 millimeters at the bottom of the well (the end disposed on the substrate). The wells can have a uniform diameter or can taper toward the bottom so that the diameter of the container at the end in contact with the substrate is smaller than the diameter of the opposing end.

Cell-Substrate Impedance Measurement Systems

Our cell-substrate impedance measurement system comprises a) at least one multiple-well cell-substrate impedance measuring device, in which at least two of the multiple wells comprise an electrode array at the bottom of the well; b) an impedance analyzer electronically connected to the multiple-well cell-substrate impedance measuring device; c) a device station capable of engaging the one or more multiple-well devices and comprising electronic circuitry capable of selecting and connecting electrode arrays within any of the multiple wells to the impedance analyzer; and d) a software program connected to the device station and impedance analyzer to control the device station and perform data acquisition and data analysis from the impedance analyzer.

In our cell-substrate impedance measurement system, the impedance analyzer engages connection pads of one or more multi-well devices to measure impedance. The impedance analyzer is capable of measuring impedance between 0.1 ohm and $10^5$ ohm in frequency range of 1Hz to 1 MHz. The impedance analyzer is preferably capable of measuring both resistance and reactance (capacitive reactance and inductive reactance) components of the impedance. The impedance analyzer is capable of measuring impedance between 0.1 ohm and $10^3$ ohm in frequency range of 100 Hz to 100 kHz.

A cell-substrate measurement system can be used to efficiently and simultaneously perform multiple assays by using circuitry of the device station to digitally switch from recording from measuring impedance over an array in one well to measuring impedance over an array in another well. Our system under software control is capable of completing an impedance measurement for an individual well at a single frequency within less than ten seconds. In another embodiment, the averaged time used by the system to complete an impedance measurement for an individual well at a single frequency is less than one second.

A multiple-well cell-substrate impedance measuring device in our system can be any multiple-well cell-substrate impedance measuring device in which at least two of the multiple wells comprise an electrode array at the bottom of the well, and in which at least two of the multiple wells comprise an electrode array are individually addressed. In one embodiment of the above system, the multi-well device takes the form of a specialized microtiter plate which has microelectronic sensor arrays integrated into the bottom of the wells.

A device used in our system, when connected to an impedance analyzer, can measure differences in impedance values that relate to cell behavior. For example, a cell-substrate impedance measuring device used in our system can measure differences in impedance values when cells are attached to the electrode array and when cells are not attached to the electrode array, or can detect differences in impedance values when the number, type, activity, adhesiveness, or morphology of cells attached to the electrode-comprising surface of the apparatus changes.

Preferred devices that can be part of a cell-substrate impedance monitoring system can be those described in U.S. Patent 7,470,533 and in U.S. Patent 7,192,752. Preferred devices that can be part of a cell-substrate impedance monitoring system can also be those described herein.

Preferably a multi-well device of our system comprises between 4 and 1,536 wells, some or all of which can comprise electrode arrays. In some embodiments, a device station can comprise one or more platforms or one or more slots for positioning one or more multi-well devices. The one or more platforms or one or more slots can comprise sockets, pins or other devices for electrically connecting the device to the device station. The device station preferably can be positioned in a tissue culture incubator during cell impedance measurement assays. It can be electrically connected to an impedance analyzer and computer that are preferably located outside the tissue culture incubator.

The device station comprises electronic circuitry that can connect to an impedance monitoring device and an impedance analyzer and electronic switches that can switch on and off connections to each of the two or more electrode arrays of the multiwell devices used in the system. The switches of the device station are controlled by a software program. The software program directs the device station to connect arrays of the device to an impedance analyzer and monitor impedance from one or more of the electrode arrays. During impedance monitoring, the impedance analyzer can monitor impedance at one frequency or at more than one frequency. Preferably, impedance monitoring is performed at more than one time point for a given assay, and preferably, impedance is monitored at at least three time points. The device station can connect individual arrays of a device to an impedance analyzer to monitor one, some, or all of the arrays of a device for a measurement time point. The switches of the device station allow the selected individual arrays to be monitored in rapid succession for each desired monitoring time point. Each monitoring time point is in fact a narrow time frame (for example from less than one second to minutes) of measurement in the assay during which impedance monitoring is performed. In some preferred embodiments the device station software is programmable to direct impedance monitoring of any of the wells of the device that comprise arrays at chosen time intervals.

The software of the impedance monitoring system can also store and display data. Data can be displayed on a screen, as printed data, or both. Preferably the software can allow entry and display of experimental parameters, such as descriptive information including cells types, compound concentrations, time intervals monitored, etc.

Preferably, the software can also analyze impedance data. In preferred embodiments, the software can calculate a cell index (CI) for one or more time points for one or more wells of the multiwell device. In some preferred embodiments, the software can calculate a cell change index (CCI) from impedance measurements of one or more wells of the multiwell device. The software can preferably generate plots of impedance data and impedance values, such as but not limited to CI or CCI, with respect to time. The software may perform other analysis as well, such as calculate cell number from CI, generate dose-response curves based on impedance data, calculate IC values based on impedance values, and calculate kinetic parameters of cell growth or behavior based on impedance values and impedance value curves. The software of the impedance monitoring system can also store and display analyses of the data, such as calculated impedance values and kinetic parameters derived therefrom, Data can be displayed on a screen, as printed data, or both.

**C. Methods for Calculating Cell Index (CI) and Cell Change Index (CCI)**

*Cell Index*

Based on the dependent relationship between the measured impedance, cell number (more accurately, the viable cell number, or attached cell number) and cell attachment status, it is possible to derive a so-called "cell number index" or "cell index" from the measured impedance frequency spectra that provides a useful index for quantitating and comparing cell behavior in the impedance-based assays. In some applications, "cell index" is the same as "cell number index" in PCT WO 2004/010103 and in United States patent7,470,533, and U.S. Patent 7.192.752.

Various methods for calculating such a cell number index can be used, some of which are novel methods disclosed herein.

We provide several methods of calculating cell index numbers for cells attached to two or more essentially identical arrays of a cell-substrate impedance device, where the cells are monitored for impedance changes. In preferred embodiments, the methods calculate cell index number with better accuracy than previous methods of calculating cell index for cells on two or more arrays of a cell-substrate monitoring device. In some preferred, methods of calculating a cell index rely on novel methods for calculating the resistances of electrical traces leading to two or more essentially identical arrays. The methods therefore also includes methods of calculating resistances of electrical traces leading to two or more essentially identical arrays on a substrate.

By "essentially identical electrode arrays" or "essentially identical arrays" is meant that the dimensions and arrangement of electrodes, electrode structures, and electrode elements is the same for the referenced arrays. Thus, two essentially identical electrode arrays will have electrode structures of the same dimensions (length, width, thickness), where the electrode structures have the same number of electrode elements, and the arrangement of electrode structures and electrode elements in each array are the same. By arrangement is meant the distance between structures or elements (gap width), their physical position with respect to one another, and their geometry (angles, degree of curvature, circle-on-line or castellated geometries, etc.), including the same features of any electrode buses that may be connected to electrode structures or electrode elements. Electrodes of essentially identical arrays also comprise the same materials. For the purposes of calculating trace resistances and cell index number, a substrate can have any number of essentially identical arrays.

The following discussion provides novel methods of calculating cell index of cells adhered to arrays of a cell-substrate impedance monitoring device and novel methods for the calculation of the resistances of the electrical connection traces leading to two or more electrode arrays of a cell-substrate impedance monitoring device.

Impedance (Z) has two components, namely the resistance Rs and reactance Xs. Mathematically, the impedance Z is expressed as follows,

$$Z = Rs + j\, Xs, \hspace{3cm} \underline{(2)}$$

where $j = \sqrt{-1}$ , depicting that for the (serial) reactance component Xs, the voltage applied over it is 90 degree phased-out from the current going through it. For the (serial) resistance, the voltage applied over it is in phase with the current going through it. As it is well-known in electronic and electrical engineering, the impedance can also be expressed in terms of parallel resistance Rp and parallel reactance Xp, as follows,

$$Z = Rp*(j\, Xp)/(Rp + j\, Xp), \hspace{3cm} \underline{(3)}$$

where $j = \sqrt{-1}$. Nevertheless, these expressions (serial resistance and serial reactance, or parallel resistance and parallel reactance) are equivalent. Those who are skilled in electrical and electronic engineering can readily derive one form of expression from the parameter values in the other expression. For the sake of clarity and consistency, the description and discussion utilizes the expression of serial resistance and serial reactance. For simplicity, serial resistance and serial reactance are simply called resistance and reactance.

As described in US patent 7,470,533and WO 2004/010103, monitoring cell-substrate impedance for detection or measurement of change in impedance can be done by measuring impedance in any suitable range of frequencies. For example, the impedance can be measured in a frequency range from about 1 Hz to about 100 MHz. In another example, the impedance can be measured in a frequency range from about 100 Hz to about 2 MHz. The impedance is typically a function of the frequency, i.e., the impedance values change as frequency changes. Monitoring cell-substrate impedance can be done either in a single frequency or multiple frequencies. If the impedance measurement is performed at multiple frequencies, then a frequency-dependent impedance spectrum is obtained - i.e., there is an impedance value at each measured frequency. As mentioned above, the impedance has two components - a resistance component and a reactance component. A change in either resistance component or reactance component or both components can constitute a change in impedance.

As described in US patent 7,470,533 WO 2004/010103, the method for the measurement of electrical (or electronic) impedance is achieved by , (1) applying a voltage between or among said electrodes at a given frequency (or multiple frequencies, or having specific voltage waveform) and monitoring the electrical current through said electrodes at the frequency (or multiple frequencies, or having specific waveform), dividing the voltage amplitude value by the current amplitude value to derive the impedance value; (2) applying an electric current of a single frequency component (or multiple frequencies or having specific current wave form) through said electrodes and monitoring the voltage resulted between or among said electrodes at the frequency (or multiple frequencies, or having specific waveform), dividing the voltage amplitude value by the current amplitude value to derive the impedance value; (3) other methods that can measure or determine electric impedance. Note that in the description above of "dividing the voltage amplitude value by the current amplitude value to derive the impedance value", the "division" is done for the values of current amplitude and voltage amplitude at same frequencies. As it is well-known in electrical and electronic engineering, in such calculations (e.g. divisions mentioned above), the current amplitude and voltage amplitude are expressed in the form of complex numbers, which take into account of how big the current and the voltage are and what the phase difference between the sinusoidal waves of the current and the voltage is. Similarly, the impedance value is also expressed in a complex form, having both resistance and reactance component, as shown in equations above.

As described in US patent 7,470,533and WO 2004/010103, the measured cell-substrate impedance can be used to calculate a parameter termed Cell Index or Cell Number Index. Various methods for calculating such a cell number index can be used based on the changes in resistance or reactance when cells are attached to the electrode structures with respect to the cases no cells are attached to the electrode structures. The impedance (resistance and reactance) of the electrode structures with no cell attached but with same cell culture medium over the electrode structures is sometimes referred as baseline impedance. The baseline impedance may be obtained by one or more of the following ways: (1) the impedance measured for the electrode structures with a cell-free culture medium introduced into the well containing the electrode structures, wherein the culture medium is the same as that used for the impedance measurements for the condition where the cell attachment is monitored; (2) the impedance measured shortly (e.g. 10 minutes) after the cell-containing medium was applied to the wells comprising the electrode structures on the well bottom (during the short period after cell-containing medium addition, cells do not have enough time to attach to the electrode surfaces. The length of this short-period may depend on cell type and/or surface treatment or modification on the electrode surfaces); (3) the impedance measured for the electrode structures when all the cells in the well were killed by certain treatment (e.g. high-temperature treatment) and/or reagents (e.g. detergent) (for this method to be used, the treatment and/or reagents should not affect the dielectric property of the medium which is over the electrodes).

In one example (**A**), the cell index or cell number index can be calculated by: (**A1**) at each measured frequency, calculating the resistance ratio by dividing the resistance of the electrode arrays when cells are present and/or attached to the electrodes by the baseline resistance,

(**A2**) finding or determining the maximum value in the resistance ratio over the frequency spectrum,

(**A3**) and subtracting one from the maximum value in the resistance ratio.

Using a mathematically formula, Cell Index is derived as

$$\text{Cell Index} = \max_{i=1,2,\ldots N}\left( \frac{R_{cell}(f_i)}{R_b(f_i)} - 1 \right) \tag{4}$$

Where N is the number of the frequency points at which the impedance is measured. For example, if the frequencies used for the measurements are at 10 kHz, 25 kHz and 50 kHz, then N=3, $f_1$= 10 kHz, $f_2$ = 25 kHz, $f_3$ = 50 kHz. $R_{cell}(f_i)$ is the resistance (cell-substrate resistance) of the electrode arrays or electrode structures when the cells are present on the electrodes at the frequency $f_i$ and $R_b(f_i)$ is the baseline resistance of the electrode array or structures at the frequency $f_i$.

The cell index obtained for a given well reflects: 1) how many cells are attached to the electrode surfaces in this well, 2) how well cells are attached to the electrode surfaces in the well. In this case, a zero or near-zero "cell index or cell number index" indicates that no cells or very small number of cells are present on or attached to the electrode surfaces. In other words, if no cells are present on the electrodes, or if the cells are not well-attached onto the electrodes, $R_{cell}(f_i)$ is about the same as $R_b(f_i)$, leading to Cell Index =0. A higher value of "cell number index" indicates that, for same type of the cells and cells under similar physiological conditions, more cells are attached to the electrode surfaces. In other words, under same physiological conditions, more cells attached on the electrodes, the larger the values $R_{cell}(f_i)$ is, leading to a large value for Cell Index. Thus Cell Index is a quantitative measure of cell number present in a well. A higher value of "cell index" may also indicate that, for same type of the cells and same number of the cells, cells are attached better (for example, cells spread out more, or cell adhesion to the electrode surfaces is stronger) on the electrode surfaces.

Thus, for same number of the cells present in the well, change in a cell status will lead to a change in cell index. For example, an increase in cell adhesion or a cell spread leading to large cell/electrode contact area will result in an increase in $R_{cell}(f)$ and a larger Cell Index. On the other hand, a cell death or toxicity induced cell detachment, cell rounding up, will lead to smaller $R_{cell}(f)$ and thus smaller Cell Index.

In another example **(B),** the cell number index can be calculated by:

(**B1**) at each measured frequency, calculating the reactance ratio by dividing the reactance of the electrode arrays when cells are present on and/or attached to the electrodes by the baseline reactance,
(**B2**) finding or determining the maximum value in the reactance ratio over the frequency spectrum,
(**B3**) and subtracting one from the maximum value in the resistance ratio.

In this case, a zero or near-zero "cell number index" indicates that no cells or very small number of cells are present on or attached to the electrode surfaces. A higher value of "cell number index" indicates that, for same type of the cells and cells under similar physiological conditions, more cells are attached to the electrode surfaces.

In yet another example **(C),** the cell index can be calculated by:

(**C1**) at each measured frequency, subtracting the baseline resistance from the resistance of the electrode arrays when cells are present or attached to the electrodes to determine the change in the resistance with the cells present relative to the baseline resistance;
(**C2**) then finding or determining the maximum value in the change of the resistance.

In this case, "cell-number index" is derived based on the maximum change in the resistance across the measured frequency range with the cells present relative to the baseline resistance. This cell index would have a dimension of ohm.

In yet another example **(D),** the cell index can be calculated by:

(**D1**) at each measured frequency, calculating the magnitude of the impedance (equaling to $\sqrt{R_s^2 + X_s^2}$, where $R_s$ and $X_s$ are the serial resistance and reactance, respectively).
(**D2**) subtracting the magnitude of the baseline impedance from the magnitude of the impedance of the electrode arrays when cells are present or attached to the electrodes to determine the change in the magnitude of the impedance with the cells present relative to the baseline impedance;
(**D3**) then finding or determining the maximum value in the change of the magnitude of the impedance.

In this case, "cell-number index" is derived based on the maximum change in the magnitude of the impedance across the measured frequency range with the cells present relative to the baseline impedance. This cell index would have a dimension of ohm.

In yet another example **(E),** the index can be calculated by:

(**E1**) at each measured frequency, calculating the resistance ratio by dividing the resistance of electrode arrays when cells are present or attached to the electrodes by the baseline resistance,
(**E2**) then calculating the relative change in resistance in each measured frequency by subtracting one from the resistance ratio,
(**E3**) then integrating all the relative-change value (i.e., summing together all the relative-change values at different frequencies).

In this case, "cell-number index" is derived based on multiple-frequency points, instead of single peak-frequency like above examples. Again, a zero or near-zero "cell number index" indicates that on cells are present on the electrodes. A higher value of "cell number index" indicates that, for same type of the cells and cells under similar physiological conditions, more cells are attached to the electrodes.

In yet another example **(F),** the cell index can be calculated by:

**(F1)** at each measured frequency, subtracting the baseline resistance from the resistance of the electrode arrays when cells are attached to the electrodes to determine the change in the resistance with the cells present relative to the baseline impedance; (here the change in the resistance is given by $\Delta R(f_1) = R_{s\text{-}cell}(f_i) - R_{s\text{-}baseline}(f_i)$ for the frequency $f_i$, $R_{s\text{-}cell}$ and $R_{s\text{-}baseline}$ are the serial resistances with the cells present on the electrode array and the baseline serial resistances, respectively);

**(F3)** analyzing the frequency dependency of the change of the resistance to derive certain parameters that can quantify such dependency. In one example, such parameters can be calculated as $\sqrt{\sum_i \left[\Delta R(f_i)\right]^2}$ . In another example, such parameter can be calculated as The parameter(s) are used as cell index or cell number index.

In this case, "cell-number index" is derived based on the analysis of the frequency spectrum of the change in the resistance. Depending how the parameters are calculated, the cell index may have a dimension of ohm.

In yet another example (**G**), the cell index can be calculated by:

**(G1)** at each measured frequency, calculating the magnitude of the impedance (equaling to where $R_s$ and $X_s$ are the serial resistance and reactance, respectively).

**(G2)** subtracting the magnitude of the baseline impedance from the magnitude of the impedance of the electrode arrays when cells are attached to the electrodes to determine the change in the magnitude of the impedance with the cells present relative to the baseline impedance; (here, the change in the magnitude of the impedance is given by $\Delta Z(f_i) = |Z_{cell}(f_i)| - |Z_{baseline}(f_i)|$ for the frequency $f_i$,

$R_{s\text{-}cell}$ and $X_{s\text{-}cell}$ being the serial resistance and reactance with the cells present on the electrode arrays, respectively, $|Z_{cell}(f_i)|$ is the magnitude of the impedance of the electrode array with cells present on the electrode arrays, $|Z_{baseline}(f_i)|$ is the magnitude of the baseline impedance of the electrode array);

**(G3)** analyzing the frequency dependency of the change of the magnitude of the impedance to derive certain parameters that can quantify such dependency. In one example, such parameters can be calculated as In another example, such parameter can be calculated as The parameter(s) are used as cell index or cell number index.

In this case, "cell-number index" is derived based on the analysis of the frequency spectrum of the change in the magnitude of the impedance. Depending how the parameters are calculated, the cell index may have a dimension of ohm.

As described in US patent 7,470,533and WO 2004/010103, there are different methods for calculating the parameter termed Cell Index or Cell Number Index from the measured cell-substrate impedance (resistance or reactance). Cell Index or Cell Number Index is a quantitative measure of cells in the wells under cell-substrate impedance measurement.

It is worthwhile to point out that it is not necessary to derive such a "cell number index" for utilizing the impedance information for monitoring cell conditions over the electrodes. Actually, one may choose to directly use measured impedance (e.g., at a single fixed frequency; or at a maximum relative-change frequency, or at multiple frequencies) as an indicator of cell conditions. If measured impedance values are directly used for monitoring cell conditions, then resistance, or reactance or both resistance and reactance can be used.

Still, deriving "cell index" or "cell number index" and using such index to monitor cell conditions may have advantages. There are several advantages of using "cell number index" to monitor cell growth and/or attachment and/or viability conditions.

First, one can compare the performance of different electrode geometries by utilizing such cell number index.

Secondly, for a given electrode geometry, it is possible to construct "calibration curve" for depicting the relationship between the cell number and the cell number index by performing impedance measurements for different number of cells added to the electrodes (in such an experiment, it is important to make sure that the seeded cells have well-attached to the electrode surfaces). With such a calibration curve, when a new impedance measurement is performed, it is then possible to estimate cell number from the newly-measured cell number index.

Thirdly, cell number index can also be used to compare different surface conditions. For the same electrode geometry and same number of cells, a surface treatment given a larger cell number index indicates a better attachment for the cells to the electrode surface and/or better surface for cell attachment.

As shown above, for some methods of calculating cell index or cell number index, it is important to know the impedance (resistance and/or reactance) of the electrode structures with and without cells present on them. Based on the equation (1), the impedance of the electrode array (with or without cells present on the electrodes) is given by

$$Z_{electrode\text{-}array} = Z_{total} - Z_{trace} - Z_{switch} \qquad (5)$$

Where $Z_{switch}$ is the impedance of electronic switch at its on stage, $Z_{trace}$ is the impedance of the electrical connection traces (or electrical conductive traces) on the substrate between the connection pads and the electrode buses, $Z_{total}$ is the total impedance measured at the impedance analyzer. By choosing electronic switches with good quality, it is possible to have all the electronic switches have a consistent on-impedance (mainly resistance). For example, the on-resistance of electronic switches can be about 3 ohm (+/- 10%) with the on reactance being negligible (for example, less than 0.2 ohm in the frequency range of interest). Thus, if the trace impedance is determined or calculated, then formula (5) can be used to calculate the impedance of the electrode arrays with or without cells present.

A method is invented in the present application to determine the impedance of electrical conductive (electrical connection) traces (mainly trace resistance, trace reactance is very small for the thin conductive film trace) based on the relationships among two or more essentially identical arrays on a cell-substrate impedance monitoring device. In the following, the four electrode arrays A, B, C and D as indicated in Figure 1, are used to illustrate this method. The electrical reactance (serial reactance) of the electronic switches and the electrical reactance (serial reactance) of the electrical connection traces are small as compared with the corresponding electrical resistances (serial resistances). Thus, we focus on the analysis of the resistance of the electrical connection traces. The impedance determined from the impedance analyzer does contain both resistance (serial resistance, $R_{total}$) and reactance (serial reactance). For the electrode arrays A - D, the measured total resistance $R_{total}$, the resistance ($R_{trace}$) of electrical conductive (connection) trace, the switch resistance ($R_{switch}$) and the resistance ($R_{e\text{-}array}$) of the electrode array satisfy the following equations:

$$R_{e-array\,-A} = R_{total\,-A} - R_{trace\,-A} - R_{switch\,-A} \qquad (6A)$$

$$R_{e-array\,-B} = R_{total\,-B} - R_{trace\,-B} - R_{switch\,-B} \qquad (6B)$$

$$R_{e-array\,-C} = R_{total\,-C} - R_{trace\,-C} - R_{switch\,-C} \qquad (6C)$$

$$R_{e-array\,-D} = R_{total\,-D} - R_{trace\,-D} - R_{switch\,-D} \qquad (6D)$$

With chosen electronic switches having consistent switch-on resistance, $R_{switch\text{-}A}$, $R_{switch\text{-}B}$, $R_{switch\,\text{-}C}$ and $R_{switch\,\text{-}D}$ have very similar values and can be assumed to be the same, $R_{switch}$. Thus, in above equations, the known parameters are $R_{total\,\text{-}A}$, $R_{total\text{-}B}$, $R_{total\,\text{-}C}$, and $R_{total\,\text{-}D}$, and $R_{switch\,\text{-}A}$, $R_{switch\,\text{-}B}$, $R_{switch\,\text{-}C}$ and $R_{switch\,\text{-}D}$, and there are eight unknown parameters $R_{e\text{-}array\,\text{-}A}$, $R_{e\text{-}array\,\text{-}B}$, $R_{e\text{-}array\,\text{-}C}$, and $R_{e\text{-}array\,\text{-}D}$, and $R_{trace\,\text{-}A}$, $R_{trace\,\text{-}B}$, $R_{trace\,\text{-}C}$ and $R_{trace\,\text{-}D}$. It is impossible to solve these equations for the eight unknown variables from these four equations directly. Additional relationships between these variables are needed to solve for them. Each trace resistance $R_{trace\,\text{-}A}$, $R_{trace\,\text{-}B}$, $R_{trace\,\text{-}C}$ and $R_{trace\,\text{-}D}$) depends on the metal film type used, and the geometry of the trace such as the how many rectangular segments the trace has, the film thickness(es) of the segments, the width(s) of the segments, the length(s) of the segment(s). For example,

$$R_{trace-A} = \sum_{i=1}^{N} \rho \frac{L_{A-i}}{t_{A-i} * d_{A-i}} \tag{7}$$

where N is the number of the segments of the trace-A, $t_{A-i}$, $d_{A-i}$ and $L_{A-i}$ is the thickness, width and length of the i-th segment of the traces for the electrode array A, and p is the resistivity of the thin film. The equation here applies to the film comprising a single type of metal. The equation can be readily modified to be applicable to the film comprising two or more metal types (e.g. gold film over chromium adhesion layer).

If the film thickness is reasonably uniform (for example, less than 10% in thickness variation) across the substrate, then the relationship among the trace resistances is simply determined by the pre-determined geometrical shapes (e.g. the length, width of the segments). For example, it would be straightforward to calculate the ratio $\alpha_{A-D}$ between the resistance of the electrically conductive traces for the electrode array A to the resistance of the electrically conductive traces for the electrode array D as below, where the film thickness is assumed to be the same everywhere on these traces and the resistivity is also the same everywhere on these traces,

$$\alpha_{A-D} = \frac{R_{trace-A}}{R_{trace-D}} = \frac{\sum_{i=1}^{N} \rho \frac{L_{A-i}}{t_{A-i} * d_{A-i}}}{\sum_{i=1}^{M} \rho \frac{L_{D-i}}{t_{D-i} * d_{D-i}}} = \frac{\sum_{i=1}^{N} \frac{L_{A-i}}{d_{A-i}}}{\sum_{i=1}^{M} \frac{L_{D-i}}{d_{D-i}}} . \tag{8}$$

Similarly, one can determine the ratio $\alpha_{B-D}$ and $\alpha_{C-D}$ based on the pre-determined geometrical relationships for the traces of the electrode arrays B, C and D. Note that above equations can be similarly derived for the cases where the thin film in these traces comprises more than one metal type. Thus, based on the equalities

$$R_{switch-A} = R_{switch-B} = R_{switch-C} = R_{switch-D} = R_{switch} , \tag{9A}$$

$$R_{trace-A} = \alpha_{A-D} \bullet R_{trace-D} , \tag{9B}$$

$$R_{trace-B} = \alpha_{B-D} \bullet R_{trace-D} , \tag{9C}$$

and

$$R_{trace-C} = \alpha_{C-D} \bullet R_{trace-D} \tag{9D}$$

equations (6A)-(6D) can be re-written in the following format:

$$R_{e-array-A} = R_{total-A} - \alpha_{A-D} \bullet R_{trace-D} - R_{switch} \tag{10A}$$

$$R_{e-array-B} = R_{total-B} - \alpha_{B-D} \bullet R_{trace-D} - R_{switch} \tag{10B}$$

$$R_{e-array-C} = R_{total-C} - \alpha_{C-D} \bullet R_{trace-D} - R_{switch} \tag{10C}$$

$$R_{e-array-D} = R_{total-D} - R_{trace-D} - R_{switch-D} \tag{10D}$$

For equations (10A) through (10D), there are five unknown variables, $R_{e-array-A}$, $R_{e-array-B}$, $R_{e-array-C}$, and $R_{e-array-D}$ and $R_{trace-D}$. Mathematically, these unknown variables cannot be determined from these equations. Additional information is needed to solve for these variables $R_{e-array-A}$, $R_{e-array-B}$, $R_{e-array-C}$, and $R_{e-array-D}$ and $R_{trace-D}$.

One approach is invented and described herein. In this approach, same biological or chemical solutions or suspensions are applied to the electrode-arrays A through D. Because the electrode arrays A through D have essentially identical electrode structures, the electrode array resistances $R_{e\text{-}array\text{-}A}$, $R_{e\text{-}array\text{-}B}$, $R_{e\text{-}array\text{-}C}$ and $R_{e\text{-}array\text{-}D}$ should be of same, or very similar value for such a condition when all the electrode arrays are exposed to the same biological or chemical solutions or suspensions, i.e.: $R_{e\text{-}array\text{-}A} \approx R_{e\text{-}array\text{-}B} \approx R_{e\text{-}array\text{-}C} \approx R_{e\text{-}array\text{-}D}$. If we assume the averaged electrode array resistance is $R_{e\text{-}array}$, then these approximate relationship exists $R_{e\text{-}array\text{-}A} \approx R_{e\text{-}array\text{-}B} \approx R_{e\text{-}array\text{-}C} \approx R_{e\text{-}array\text{-}D} \approx R_{e\text{-}array}$. Thus, equations (10A- 10D) can be changed to the following:

$$R_{e-array} \approx R_{total-A} - \alpha_{A-D} \bullet R_{trace-D} - R_{switch} \qquad (11A)$$

$$R_{e-array} \approx R_{total-B} - \alpha_{B-D} \bullet R_{trace-D} - R_{switch} \qquad (11B)$$

$$R_{e-array} \approx R_{total-C} - \alpha_{C-D} \bullet R_{trace-D} - R_{switch} \qquad (11C)$$

$$R_{e-array} \approx R_{total-D} - R_{trace-D} - R_{switch-D} \qquad (11D)$$

Thus, we would need to find $R_{trace\text{-}D}$ and $R_{e\text{-}array}$ that satisfy the above approximate equality as close as possible. One mathematical approach is to find $R_{trace\text{-}D}$ and $R_{e\text{-}array}$ that would result in the minimum value for the following expression - an expression that quantifies the differences between the two sides of the approximate equality in (11A, 11B, 11C and 11D),

$$F(R_{trace-D}, R_{e-array}) = \left[R_{e-array} - (R_{total-A} - \alpha_{A-D} R_{trace-D} - R_{switch})\right]^2 +$$

$$\left[R_{e-array} - (R_{total-B} - \alpha_{B-D} R_{trace-D} - R_{switch})\right]^2 +$$

$$\left[R_{e-array} - (R_{total-C} - \alpha_{C-D} R_{trace-D} - R_{switch})\right]^2 +$$

$$\left[R_{e-array} - (R_{total-D} - R_{trace-D} - R_{switch})\right]^2 \qquad (12)$$

The *expression F* ($R_{trace\text{-}D}$, $R_{e\text{-}array}$) is the sum of the squared-differences between the two-sides of the approximate equality in (11A, 11B, 11C and 11D). The smaller $F(R_{trace\text{-}D}, R_{e\text{-}array})$, the closer the two sides of the approximate equality (11A, 11B, 11C and 11D). Thus, values of $R_{trace\text{-}D}$ and $R_{e\text{-}array}$ that result in the minimum value of $F(R_{trace\text{-}D}, R_{e\text{-}array})$ should be determined. Mathematical approach involves in the calculation of the first order derivative of $F(R_{trace\text{-}D}, R_{e\text{-}array})$ to $R_{trace\text{-}D}$ and to $R_{e\text{-}array}$ and let such first order derivatives equal to zero. The values of $R_{trace\text{-}D}$ and $R_{e\text{-}array}$ that result in zero for these first-order-derivatives are those that result in the minimum value of $F(R_{trace\text{-}D}, R_{e\text{-}array})$. The first order derivatives are as follows:

$$\frac{\partial\left[F(R_{trace-D}, R_{e-aaray})\right]}{\partial R_{trace-D}} = 2 \bullet \alpha_{A-D} \bullet \left[R_{e-array} - (R_{total-A} - \alpha_{A-D} R_{trace-D} - R_{switch})\right] +$$

$$2 \bullet \alpha_{B-D} \bullet \left[R_{e-array} - (R_{total-B} - \alpha_{B-D} R_{trace-D} - R_{switch})\right] +$$

$$2 \bullet \alpha_{C-D} \bullet \left[R_{e-array} - (R_{total-C} - \alpha_{C-D} R_{trace-D} - R_{switch})\right] +$$

$$2 \bullet \left[R_{e-array} - (R_{total-D} - R_{trace-D} - R_{switch})\right]$$

$$=0; \qquad (13A)$$

$$\frac{\partial\left[F\left(R_{trace-D},R_{e-aaray}\right)\right]}{\partial R_{e-array}} = 2 \bullet\left[R_{e-array} - \left(R_{total-A} - \alpha_{A-D}R_{trace-D} - R_{switch}\right)\right]+$$

$$2 \bullet\left[R_{e-array} - \left(R_{total-B} - \alpha_{B-D}R_{trace-D} - R_{switch}\right)\right]+$$

$$2 \bullet\left[R_{e-array} - \left(R_{total-C} - \alpha_{C-D}R_{trace-D} - R_{switch}\right)\right]+$$

$$2 \bullet\left[R_{e-array} - \left(R_{total-D} - R_{trace-D} - R_{switch}\right)\right]$$

$$=0. \tag{13B}$$

Equations (13A) and (13B) can be re-written as

$$R_{e-array} \bullet\left[\alpha_{A-D} + \alpha_{B-D} + \alpha_{C-D} + 1\right]+ R_{trace-D} \bullet\left[\alpha_{A-D}^2 + \alpha_{B-D}^2 + \alpha_{C-D}^2 + 1\right]=$$

$$\alpha_{A-D} \bullet\left[R_{total-A} - R_{switch}\right]+ \alpha_{B-D} \bullet\left[R_{total-B} - R_{switch}\right]+$$

$$\alpha_{C-D} \bullet\left[R_{total-C} - R_{switch}\right]+ \left[R_{total-D} - R_{switch}\right] \tag{14A}$$

$$4 \bullet R_{e-array} + R_{trace-D} \bullet\left[\alpha_{A-D} + \alpha_{B-D} + \alpha_{C-D} + 1\right]=$$

$$\left[R_{total-A} - R_{switch}\right]+ \left[R_{total-B} - R_{switch}\right]+ \left[R_{total-C} - R_{switch}\right]+ \left[R_{total-D} - R_{switch}\right] \tag{14B}$$

Thus, we can solve for $R_{trace-D}$ as follows:

$$R_{trace-D} = \frac{4 \bullet S_1 - A_{11} \bullet S_2}{4 \bullet A_{12} - A_{11} \bullet B_{12}} \tag{15}$$

where

$$A_{11} = \left[\alpha_{A-D} + \alpha_{B-D} + \alpha_{C-D} + 1\right];$$

$$A_{12} = \left[\alpha_{A-D}^2 + \alpha_{B-D}^2 + \alpha_{C-D}^2 + 1\right];$$

$$S_1 = \alpha_{A-D} \bullet\left[R_{total-A} - R_{switch}\right]+ \alpha_{B-D} \bullet\left[R_{total-B} - R_{switch}\right]+$$

$$\alpha_{C-D} \bullet\left[R_{total-C} - R_{switch}\right]+ \left[R_{total-D} - R_{switch}\right];$$

$$B_{12} = \left[\alpha_{A-D} + \alpha_{B-D} + \alpha_{C-D} + 1\right];$$

$$S_2 = \left[R_{total-A} - R_{switch}\right]+ \left[R_{total-B} - R_{switch}\right]+ \left[R_{total-C} - R_{switch}\right]+ \left[R_{total-D} - R_{switch}\right].$$

Thus, with the determined $R_{trace-D}$, the trace resistances of $R_{trace-A}$, $R_{trace-B}$ and $R_{trace-C}$ can be calculated using equations (9B), (9C) and (9D). Furthermore, the electrode array resistance $R_{e-array-A}$, $R_{e-array-B}$, $R_{e-array-C}$ and

$R_{e\text{-}array\text{-}D}$ can be calculated from the measured resistance $R_{total\text{-}A}$, $R_{total\text{-}B}$, $R_{total\text{-}C}$ and $R_{total\text{-}D}$ respectively using equations (10A), (10B), (10C) and (10D).

Thus, calculating resistances of the electrical connection traces s from the measured, total resistances for two or more essentially identical electrode arrays (such as, for example arrays A-D in Figure 1), can include the following steps:

(1) exposing the electrode arrays to the solutions having same or similar solutions or suspensions;
(2) with an impedance analyzer or impedance measurement circuit, measuring the resistance (serial resistance) for each electrode array, such resistance being the sum of the resistance of electronic switches, the resistance of the electrical connection traces between the connection pads and the electrode structures (for example, between the connection pads and the electrode buses, for the electrode structures in Figure 1), and the resistance of the electrode array with the solutions or suspensions present;
(3) solving for the resistances of electrical connection traces using equation (15) and equations (9B), (9C) and (9D), noting in the calculation with equation (15), the geometrical relationships between the electrode arrays are used to determine the factor $\alpha_{A\text{-}D}$, $\alpha_{B\text{-}D}$ and $\alpha_{C\text{-}D}$.

Another method of calculating the resistance of the electrode arrays from the measured, total electrode resistances for two or more essentially identical electrode arrays (such as, for example arrays A-D in Figure 1) if the same or similar solutions or suspensions are added to be in contact with the electrode assays, includes the following steps:

(1) exposing the electrode arrays to the solutions having same or similar solutions or suspensions;
(2) with an impedance analyzer or impedance measurement circuit, measuring the resistance (serial resistance) for each electrode array, such resistance being the sum of the resistance of electronic switches, the resistance of the electrical connection traces between the connection pads and the electrode structures (for example, between the connection pads and the electrode buses, for the electrode structures in Figure 1) and the resistance of the electrode arrays with the solutions or suspensions present;
(3) solving for the resistances of electrical connection traces using equation (15) and equations (9B), (9C) and (9D), noting in the calculation with equation (15), the geometrical relationships between the electrode arrays are used to determine the factor $\alpha_{A\text{-}D}$, $\alpha_{B\text{-}D}$ and $\alpha_{C\text{-}D}$;
(4) calculating the resistances of the electrode arrays using equations (10A, 10B, 10C and 10D)).

In many applications, the solutions or suspensions (for example, cell suspension) applied to each electrode array may have different compositions. For example, cell suspensions of different cell numbers may be used so that the suspensions applied to each electrode array are quite different. Under such cases, the determination of the resistance of the electrode arrays with the cells present would require the determination of the resistance of the electrical connection traces by performing a "reference run" or "calibration run" in which the electrode arrays are exposed to a same, reference solution. From the "reference run", the resistances of the electrical connection traces can be determined. In a separate test, the electrode arrays are exposed to the solutions or cell suspensions of interest and the resistances for the electrode arrays under such conditions are measured with an impedance analyzer or impedance measuring circuit. The resistance of the electrode arrays with such cell suspensions present can be determined (or continuously determined) from the measured resistance by subtracting the sum of the resistance of the electronic switches and the resistance of the electrical connection traces for corresponding electrode arrays from the measured resistances.

Another method of calculating the resistance of the electrode arrays from the total electrical resistances measured at an impedance analyzer for essentially identical electrode arrays (such as electrode arrays A-D in Figure 1 used as an example) if different solutions or suspensions of interest are applied to the electrode assays, includes the following steps:

(1) exposing the electrode arrays to the solutions having same or similar solutions or suspensions (reference solutions);
(2) with an impedance analyzer or impedance measurement circuit, measuring the resistance (serial resistance) for each electrode array, such resistance being the sum of the resistance of electronic switches, the resistance of the electrical connection traces between the connection pads and the electrode structures (for example, between the connection pads and the electrode buses, for the electrode structures in Figure 1) and the resistance of the electrode arrays with the reference solutions present;
(3) solving for the resistances of electrical connection traces using equation (15) and equations (9B), (9C) and (9D), noting in the calculation with equation (15), the geometrical relationships between the electrode arrays of Figure 1 are used to determine the factor $\alpha_{A\text{-}D}$, $\alpha_{B\text{-}D}$ and $\alpha_{C\text{-}D}$;
(4) applying the solutions or suspensions of interest to each electrode array; and with an impedance analyzer or impedance measurement circuit, measuring the resistance (serial resistance) of each electrode array, such resistance being the sum of the resistance of electronic switches, the resistance of the electrical connection traces between the connection pads and the electrode structures, the resistance of the electrode arrays with the solutions or suspensions of the interest present,

(5) Calculating the resistance of the electrode arrays using equations (10A), (10B), (10C) and (10D) by subtracting the electronic switch resistances and the resistances of electrical connection traces from the measured resistances in the step (4).

Note that in above method, the steps of exposing the electrode arrays to reference solutions for the determination of the resistances of electrically conductive traces (step (1), (2) and (3)) may be performed before or after the steps of applying the solutions or suspensions of interest to the electrode arrays and measuring the total electrical resistance (step (4)). For example, step (4) may be performed first. After that, the solutions or suspensions of the interest may be removed from the electrode array. The reference solutions can then be added to the electrode arrays (step (1)). Step (2) and step (3) can be then performed to determine the resistances of electrical connection traces. Finally, Step (5) can be done.

In another approach, step (1) and (2) can be performed ahead of step (4).

Another method of determining the resistance of the electrode arrays with the cells present for a cell-based assay based on the total electrical resistance measured at an impedance analyzer for essentially identical electrode arrays. In this method, the electrode arrays are exposed to a same, reference solution (for example, a same cell culture medium that does not contain any cells) and electrical measurement is conducted to determine the resistance of electrical connection traces. With the resistances of the electrical connection traces determined, electrical resistances of the electrode arrays with cell suspensions added to electrode arrays can be calculated from the total electrical resistances measured at an impedance analyzer. Such total electrical resistance would include the resistance of the electrode arrays with cells present, the resistance of electronic switches and the resistance of electrical connection traces. The method comprises following steps

(1) exposing the electrode arrays to the solutions having same or similar solutions or suspensions (reference solutions);

(2) with an impedance analyzer or impedance measurement circuit, measuring the resistance (serial resistance) for each electrode array, such resistance being the sum of the resistance of electronic switches, the resistance of the electrical connection traces between the connection pads and the electrode structures (for example, between the connection pads and the electrode buses, for the electrode structures in **Figure 1**) and the resistance of the electrode arrays with the reference solutions present;

(3) solving for the resistances of electrical connection traces using equation (15) and equations (9B), (9C) and (9D), noting in the calculation with equation (15), the geometrical relationships between the electrode arrays in **Figure 1** are used to determine the factor $\alpha_{A-D}$, $\alpha_{B-D}$ and $\alpha_{C-D}$;

(4) applying the cell suspensions of interest to each electrode array; and with an impedance analyzer or impedance measurement circuit, measuring the resistance (serial resistance) of each electrode array, such resistance being the sum of the resistance of electronic switches, the resistance of the electrical connection traces between the connection pads and the electrode structures, the resistance of the electrode arrays with the cell suspensions of the interest present,

(5) Calculating the resistance of the electrode arrays using equations (10A), (10B), (10C) and (10D) by subtracting the electronic switch resistances and the resistances of electrical connection traces from the measured resistances in step (4).

Note that in above method, the steps of exposing the electrode arrays to reference solution for the determination of the electrical resistance of electrically conductive traces (step (1), (2) and (3)) may be performed before or after the steps of applying the solutions of interest or cell suspensions of interest to the electrode arrays and measuring the total electrical resistance (step (4)). For example, step (4) may be performed first, followed by steps (1) and (2). In one approach, after step (4), the cell suspensions of the interest may be removed from the electrode array. Then reference solutions can be added to the electrode arrays. In another approach, after step (4), the cells are all lysed with some cell lysis solutions so that the electrodes are exposed to the same, reference solutions for the measurement and calculation of step (2) and (3). And then, step (5) is performed to determine the electrical resistance of electrode arrays with the cell suspensions of interest present.

The determination of the resistances of the electrical conductive traces for the electrode arrays that essentially identical electrode arrays may be, or may not be, part of the monitoring of cell-substrate impedance for cell-based assays. It depends on how the impedance data (measured at a single frequency or multiple frequencies, measured at multiple time points) of the electrode arrays is analyzed.

In some assays, one is interested in the relative change in the resistance or impedance of the electrode arrays with the cells present relative to the baseline resistance or impedance. For such cases, it is preferred to determine the resistance (or impedance) of the electrode arrays from the total, measures resistance (or impedance) by subtracting the resistance of the electrical conductive traces and the resistance of electronic switches. Thus, determination of the resistances or impedance of the electrically conductive traces may be required.

In some other assays, one is interested in the absolute changes in the resistance (or impedance) of the electrode arrays with cells present relative to the baseline resistance (or impedance). In these cases, one can directly subtract the measured resistance or impedance for the baseline condition from the measured resistance or impedance for the condition that the cells are present on the electrode arrays. The contribution of the resistance (or impedance) of the electronic switches and the resistance (or impedance) of the electrically conductive traces to the total measured resistance (or impedance) values is cancelled out in such subtractions. Thus, there is no need for determining the resistances of the electrically conductive traces.

In some assays, one is interested in calculating the Cell Index or Cell Number Index based on the monitored impedance values. Depending on which method is used for calculating the Cell Index, it may, or may not, be necessary to determine the resistances of the electrically conductive traces. For example, for the Cell Index calculation method (A) described above, the resistances of the electrically conductive traces are needed, in order to remove the effect of the resistance of the electrically conductive traces on the analysis of the relative change of the resistance or impedance. In another example, for the Cell Index calculation method (F) described above, there is no need to determine the resistances of the electrically conductive traces since the effect of the resistance of the electrically conductive traces is canceled out in the calculations.

The monitoring of the cell-substrate impedance may be or may not be based on the change with respect to the baseline impedance (or resistance). For example, a cell-based assay is performed to assess the effect of a test compound on the cells. One method in performing such an assay is by monitoring of the cell-substrate impedance and determining the change in the cell-substrate impedance before and after the addition of the test compound to the cells. The monitoring of cell-substrate impedance can be performed at a single frequency point or multiple frequency points, at a single time point or multiple time points after drug addition. For example, the impedance is first measured at a single frequency or multiple frequencies for the electrode arrays with the cells present just before addition of test compound. The test compound is then added to the cells. The impedance is then measured again at the same single frequency or multiple frequencies for the electrode arrays with the cells after the addition of test compound. Such post-compound addition measurement may be performed for many time points continuously in a regular or irregular time intervals. The change in the cell-substrate impedances can be determined or quantified by subtracting the impedance(s) (resistance and/or reactance) measured before addition of the test compound from the impedance(s) (resistance and/or reactance) measured after addition of the test compound. If the measurement is done at multiple frequencies, a single parameter or multiple parameters may be further derived for each time point after compound addition based on the calculated change in the cell-substrate impedances. Such parameters are used to quantify the cell changes after compound addition. Such approaches can be used further to analyze the responses of the cells to a test compound at multiple concentrations to derive dose-dependent response curves.

*Normalized Cell Index, Delta Cell Index*

A "Normalized Cell Index" at a given time point is calculated by dividing the Cell Index at the time point by the Cell Index at a reference time point. Thus, the Normalized Cell Index is 1 at the reference time point. Normalized cell index is cell index normalized against cell index at a particular time point. In most cases in the present applications, normalized cell index is derived as normalized relative to the time point immediately before a compound addition or treatment. Thus, normalized cell index at such time point (immediately before compound addition) is always unit one for all wells. One possible benefit for using such normalized cell index is to remove the effect from difference in cell number in different wells. A well having more cells may produce a larger impedance response following compound treatment. Using normalized cell index, it helps to remove such variations caused by different cell numbers.

A "delta cell index" at a given time point is calculated by subtracting the cell index at a standard time point from the cell index at the given time point. Thus, the delta cell index is the absolute change in the cell index from an initial time (the standard time point) to the measurement time.

*Cell Change Index*

The time-dependent cellular response (including cytotoxicity response) may be analyzed by deriving parameters that directly reflect the changes in cell status. For example, time dependent cellular response may be analyzed by calculating the slope of change in the measured impedance responses (that is equivalent to the first order derivative of the impedance response with respect to time, impedance response here can be measured impedance data or derived values such as cell index, normalized cell index or delta cell index). In another example, the time-dependent cellular responses (including cytotoxic responses) may be analyzed for their higher order derivatives with respect to time. Such high order derivatives may provide additional information as for how cells responding to different compounds and as for the mechanisms of compound action.

As an example, we describe how one can to derive a parameter, called Cell Change Index, based on the real time, quantitative information (i.e., cell index, CI) about biological status of cells in the wells provided from RT-CES system. This new parameter, Cell Change Index (CCI), can effectively link time dependent cell index I with cell status, is calculated as,

$$CCI\ (t) = \frac{dCI\ (t)}{CI\ (t) \cdot dt}. \tag{5}$$

Thus CCI is the normalized rate of change in cell index. CCI values can be used to quantify the cell status change. For cells in an exponential growth under regular cell culture condition, the cell index determined by a cell-substrate impedance monitoring system described herein is expected to be a proportionate measure of the cell number in the well since the cell morphology and average extent of cell adhesion to the electrode surfaces among the whole cell population do not exhibit significant changes over time. Thus, the cell index (CI) increase with time following an exponential function, such that

$$CI\ (t) = CI\ (0) * 2^{\frac{t}{DT}} \tag{6}$$

where DT is the cell doubling time. For such exponential growth culture, CCI(t) is a constant, giving

$$CCI\ (t) = \frac{0.693}{DT} \approx \frac{0.7}{DT}. \tag{7}$$

Thus, several types of CCI(t) can be classified as:

(1) If CCI is about 0.7/DT, cell index increases in the same rate as that expected for an exponential growth of the cells.
(2) If CCI >> 0.7/DT, cell index increases faster than that expected for an exponential growth (or log growth) of the cells. This indicates that cells may grow faster than regular exponential growth, or cells may exhibit some morphology change (e.g. cell spreading out or adhering better to the electrode surfaces), leading to large impedance signal, or both of above effects, or there may be other cell behaviors occurring particular to the assay or culture conditions.
(3) If CCI is more than zero but somewhat smaller than 0.7/DT, then cell index increases in the rate slowed than that expected for an exponential growth. This indicates that cell growth rate may be slowed down relative to exponential growth, or cell growth may be somewhat inhibited by chemical compounds added to the culture media or by other cell culture parameters, or that certain populations of cells are dying off and detaching from the electrode surfaces, or there may be other cell behaviors occurring particular to the assay or culture conditions.
(4) If CCI is about zero, then cell index shows a near constant value. This may indicate that the cell growth is nearly-completely inhibited. For example, all the cells are arrested at certain points of cell cycle and are not progressing further. Or, this may indicate that the number of cells dying off in the culture is nearly as the number of newly-divided cells. Alternatively this may indicate that cells reach stationary phase of cell culture. Alternatively this may indicate that number of cells are above the detection upper limit of the cell-substrate impedance monitoring system. There is also the possibility of other cell behaviors occurring particular to the assay or culture conditions.
(5) If CCI is negative, then the cell index is decreasing with time, showing the cells losing attachment to the electrode surface or changing their morphology.
(6) If CCI is very negative, then the cell index decreases rapidly with time, showing that either cells lose attachment to the electrode surfaces quickly or cells change their morphology very quickly.

**D. Methods for performing real-time cell-based assays**

Our cell-based assays can be performed in real time to assess cell proliferation, cell growth, cell death, cell morphology, cell membrane properties (for example, size, morphology, or composition of the cell membrane) cell adhesion, and cell motility. Thus the assays can be cytotoxicity assays, proliferation assays, apoptosis assays, cell adhesion assays, cell activation or stimulation assays, anti-cancer compound efficacy assays, receptor-ligand binding or signal transduction analysis, assays of cytoskeletal changes, assays of cell structural changes (including but not limited to, changes in cell membrane size, morphology, or composition), cell quantification, cell quality control, time-dependent cytotoxicity profiling, assays of cell differentiation or de-differentiation, detection or quantitation of neutralizing antibodies, specific T-cell mediated cytotoxic effect assays, assays of cell adhesivity, assays of cell-cell interactions, analysis of microbial, viral, or environmental toxins, etc.

The assays are real-time assays in the sense that cell behavior or cell status being assayed can be assessed continuously at regular or irregular intervals. Cell behaviors, cell responses, or cell status can be assayed and the results recorded or displayed within seconds to minutes of their occurrence. The cell response during an assay can be monitored essentially continuously over a selected time period. For example, a culture can be monitored every five to fifteen minutes for several hours to several days after addition of a reagent. The interval between impedance monitoring, whether impedance monitoring is performed at regular or irregular intervals, and the duration of the impedance monitoring assay can be determined by the experimenter.

Thus, the cell-based impedance assays avoid inadvertently biased or misleading evaluation of cell responses due to the time point or time points chosen for sampling or assaying the cells. In addition, the assays do not require sampling of cell cultures or addition of reagents and thus eliminate the inconvenience, delay in obtaining results, and error introduced by many assays.

Descriptions of cell-substrate monitoring and associated devices, systems and methods of use have been provided in WO 2004/010103; WO 2004/010102; United States patent 7,470,533; U.S. Patent 7,192,752; and United States patent 7,459,303. Additional details of cell-substrate impedance monitoring technology are further disclosed herein.

In brief, for measurement of cell-substrate or cell-electrode impedance, cell-substrate impedance monitoring devices are used that have microelectrode arrays with appropriate geometries fabricated onto the bottom surfaces of wells such as microtiter plate wells, or have a similar design of having multiple fluid containers (such as wells) having electrodes fabricated on their bottom surfaces facing into the fluid containers. Cells are introduced into the fluid containers of the devices, and make contact with and attach to the electrode surfaces. The presence, absence or change of properties of cells affects the electronic and ionic passage on the electrode sensor surfaces. Measuring the impedance between or among electrodes provides important information about biological status of cells present on the sensors. When there are changes to the biological status of the cells analogue electronic readout signals can be measured automatically and in real time, and can be converted to digital signals for processing and for analysis.

Preferably, cell-substrate impedance assays are performed using a system that comprises a device, an impedance monitor, a device station that comprises electronic circuitry and engages the device and the impedance analyzer, and a software program that controls the device station and records and analyzes impedance data.

Using our system, a cell index can be automatically derived and provided based on measured electrode impedance values. The cell index obtained for a given well reflects: 1) how many cells are attached to the electrode surfaces in this well, and 2) how well (tightly or extensively) cells are attached to the electrode surfaces in this well. Thus, the more the cells of same type in similar physiological conditions attach the electrode surfaces, the larger the cell index. And, the better the cells attach to the electrode surfaces (e.g., the cells spread-out more to have larger contact areas, or the cells attach tighter to electrode surfaces), the larger the cell index.

Our method can be used to assay cell status, where cell status includes, but is not limited to, cell attachment or adhesion status (e.g. the degree of cell spread, the attachment area of a cell, the degree of tightness of cell attachment, cell morphology) on the substrate including on the electrodes, cell growth or proliferation status; number of viable cells and/or dead cells in the well; cytoskeleton change and re-organization and number of cells going through apoptosis and/or necrosis. Our cell-based assays include, but are not limited to, cell adhesion, cell apoptosis, cell differentiation, cell proliferation, cell survival, cytotoxicity, cell morphology detection, cell quantification, cell quality control, time-dependent cytotoxicity profiling, IgE-mediated cell activation or stimulation, receptor-ligand binding, viral and bacterial toxin mediated cell pathologic changes and cell death, detection and quantification of neutralizing antibodies, specific T-cell mediated cytotoxic effect, and cell-based assays for screening and measuring ligand-receptor binding.

Cells are preferably added to at least two fluid containers of a device, each of which comprises an electrode array, and impedance is monitored from at least two wells that comprise cells and an electrode array.

The cells used in the assay can be primary cells isolated from any species or cells of cell lines. Primary cells can be from blood or tissue. The cells can be engineered cells into which nucleic acids or proteins have been introduced. In some embodiments, different cell types are added to different wells and the behavior of the cell types is compared.

An impedance monitoring assay can be from minutes to days or even weeks in duration. Preferably, impedance is monitored at three or more time points, although this is not a requirement. Impedance can be monitored at regular or irregular time intervals, or a combination of irregular and regular time intervals. Impedance can be monitored at irregular intervals and then at regular intervals during a particular time window of the assay. Impedance can be monitored at one frequency or at more than one frequency. For example impedance can be monitored over a range of frequencies for each time point at which impedance is monitored. Preferably, impedance is monitored at at least one frequency between about 1 Hz and about 100 MHz, more preferably at at least one frequency between about 100 Hz and about 2 MHz.

Figure 2 depicts results from monitoring cell proliferation. In this experiment, H460 cells were introduced into wells of a 16 well device of a cell-substrate impedance monitoring system, with different wells receiving different initial cell seeding numbers. The device was engaged with a device station of the system that was in a tissue culture incubator that kept a temperature of 37 degrees C and an atmosphere of 5% $CO_2$. Cell-substrate impedance was monitored at 15 minute intervals for 125 hours. The cell index was calculated by the system for each time point and displayed as a function of time to give cell growth (proliferation) curves for each cell seeding number. The cell growth curves were plotted on a log scale showing exponential growth phases and stationary phases.

Figure 3 depicts results of real-time monitoring of cell attachment and spreading ofNIH3T3 cells. The cells were seeded onto cell-substrate impedance monitoring devices that were coated with either poly-L-lysine or fibronectin. The device was connected to a device station that was in a tissue culture incubator that kept a temperature of 37 degrees C and an atmosphere of 5% $CO_2$. Cell attachment and cell spreading on the difference coating surfaces were monitored by measuring impedance on the cell-substrate monitoring system. Impedance was monitored in real time every 3 minutes for 3 hours. The cell index for each time point was calculated by the impedance monitoring system and plotted as a function of time.

Figure 4 shows the results of an experiment monitoring morphological changes in Cos-7 cells in response to stimulation with epidermal growth factor (EGF). Cells were seeded in wells of a 16 well monitoring device that engaged a device station of a cell-substrate monitoring system. The device station was positioned in an incubator held at 37 degrees C and 5% $CO_2$. The cells were serum starved for 8 hours and then stimulated with 50 nanograms/mL of EGF. Control cells did not receive EGF. Impedance was monitored at 3 minute intervals for 2 hours and then at 1 hour intervals for 14 hours. The cell index was calculated by the system and plotted as a function of time. An initial jump in cell index is seen in EGF-treated cells due to membrane ruffling and actin dynamics in response to EGF. The arrow indicates the point of EGF addition.

## Quantifying Cells Using Cell-Substrate Impedance Devices

We also provide methods of quantifying cells, comprising: providing our device having two or more electrode arrays, each of which is associated with a fluid container of the device; attaching the device to an impedance analyzer; adding cells to one or more fluid containers of the device; monitoring impedance from the one or more fluid containers to obtain impedance measurements at one or more time points after adding the cells to the one or more fluid containers; deriving a cell index for the one or more time points; and using the cell index to determine the number of cells in the one or more fluid containers at at least one of the one or more time points. The cell index is used to determine the number of cells using a formula that relates cell index to cell number, in which the formula is obtained by: providing a device for cell-substrate monitoring, attaching the device to an impedance monitor; adding cells to one or more fluid containers of the device; measuring impedance of the one or more fluid containers comprising cells; calculating a cell index from the impedance measurements; determining the number of cells of said at least one fluid container at the time of impedance monitoring by a means other than impedance monitoring; and deriving a formula that relates the number of cells of the one or more fluid containers at the two or more time points with the impedance measurements at the two or more time points.

Sometimes the number of cells introduced to the wells are pre-known or predetermined before cells are added in to the wells. Under such conditions, one assumes that there will be no change in cell number or little change in cell number when the impedance measurement for obtaining the formula is performed.

The number of cells determined by a method other than impedance monitoring can be determined by, for example, cell plating, hemacytometer counting, flow cytometry, or Coulter counting.

The method can also be practiced using an impedance monitoring system, where the system includes a multi-well cell-substrate impedance monitoring device, an impedance analyzer, a device station, and a software program. The method includes; providing a multi-well cell-substrate impedance measuring system; adding cells one or more wells of the system; monitoring impedance from the one or more wells comprising cells at one or more time points after adding the cells to the one or more wells; deriving a cell index for the one or more time points; and using the cell index to determine the number of cells in said at least well at at least one of said one or more time points.

The cell index is used to determine the number of cells using a formula that relates cell index to cell number, in which the formula is obtained by: providing a system for cell-substrate monitoring, where the system comprises at least one multi-well cell-substrate impedance monitoring device, adding cells to one or more wells of a device of the system; measuring impedance of the one or more wells comprising cells at two or more time points; calculating a cell index from the impedance measurement at the two or more time points; determining the number of cells of the one or more wells at the two or more time points by a means other than impedance monitoring; and deriving a formula that relates the number of cells of the one or more wells at the two or more time points with the impedance measurements at the two or more time points.

Sometimes the number of cells introduced to the wells are pre-known or predetermined before cells are added in to the wells. Under such conditions, one assumes that there will be no change in cell number or little change in cell number when the impedance measurement for obtaining the formula is performed.

The number of cells determined by a method other than impedance monitoring can be determined by, for example, cell plating, hemacytometer counting, flow cytometry, or Coulter counting.

Formulas relating cell index (including normalized cell index and delta cell index, which can also be used) to cell number for a given cell type can be used to quantitate cells of that type in assays using a cell-substrate impedance monitoring device, such as a device described herein. Generally, for a give cell type and for cells under similar physiological conditions, the derived formulas relating cell index to cell number can be used in subsequent assays. There is no need to obtain the formula each time when an assay is performed. However, it is worthwhile to point that the formula can only be valid as long as the cells are under same physiological conditions in the assays where the formula was derived and where the formula is used. If the cell condition is different, for example, the composition of culture media is changed, or the cell attachment surface is altered, then the formula will not hold. In another example, if cells are in log-growth phase in one assay and are in stationary phase in another assay, then the formula may not hold. Another point worth mentioning here is that relates the fact the derived cell index or impedance also depends on cell attachment quality on the surface as well as cell morphology. If cell morphology or cell attachment changes during an assay, then one need to distinguish between the changes caused by change in cell number or in cell morphology or in cell attachment.

As an example, we can derive the correlation formula between cell index and cell number for NIH3T3 cells under the experimental conditions. The formula for converting cell index to cell number for this particular case is: Cell number = 2000* Cell index-145. To test this formula, we found the error in estimating cell number based on the cell index data shown in Figure 8 as compared to the seeded cell number is less than 20% .

Cell-based assays to test the effects of compounds on cells

In our methods that investigate test compound effects on cells, impedance is preferably monitored from at least one test compound well at at least one time point before adding said at least one test compound to said at least one test compound well. Preferably, impedance is monitored at four or more time points, at least one of which is prior to the addition of one or more test compounds. Preferably, impedance is monitored at regular or irregular time intervals for an assay period of from minutes to days, for example, for a period of between several hours and several days. The cell-substrate impedance can be monitored at at least one time point prior to addition of the test compound, and at regular time intervals thereafter. For example, impedance can be measured at one or more intervals before adding the compound and at a regular 2 hour, 1 hour, 30 min or 15 min time intervals after adding the compound. Preferably, impedance is measured at three or more time points spaced at regular intervals. Our real-time assay allows one to perform the measurement on cell-substrate impedance with various time resolutions, for example, measurement taking place at a longer time interval such as every hour or every two hours, or at a shorter time interval every minute or a few minutes.

Impedance can be monitored at one frequency or at more than one frequency. For example, in some preferred embodiments, impedance is monitored over a range of frequencies for each time point at which impedance is monitored. Preferably, impedance is monitored at at least one frequency between about 1 Hz and about 100 MHz, more preferably at at least one frequency between about 100 Hz and about 2 MHz.

*Cell-based Assays with More Than One Cell Type*

Figures 10A and B show the responses of various cell types (listed in Table 1) to olomoucine treatment as monitored using a cell-substrate impedance monitoring system. The indicated cell lines were seeded onto microtiter devices fabricated with electronic sensor arrays shown in Figure 1. The cellular responses were continuously monitored at 15 or 30 or 60 minutes time interval before and after treatment with olomoucine. Comparison among these cell index curves showed that certain similarity does exist. Take the treatment of olomoucine at 100 uM as an example. For a significant number of cell types tested, olomoucine treatment resulted in a near-constant cell index for some length of time (for example: 10, 20 or 30 hrs) a long time. This relates to the fact olomoucine is a cell cycle resting compound and for some time period following compound addition, cells do not divide any more and so cell number does not change but cells remain "live". Thus, for such time period, cell index did not change with time. The "near-constant" cell index curves were also observed for cells treated with roscovitine, which is another compound causing cell cycle arrest. The cell index curves shown in Figures 10A and 10B are strikingly different from the cell index curves shown in Figure 9A and 9B, and Figure 11A and 11B, where compounds follow different mechanism of compound action.

The CI derived from impedance data from wells comprising different cell types and a test compound can be used to derive cell change index (CCI) values for assay time points. CCI values of particular cell types at assay time points can be compared. Such comparisons can indicate whether different cell types are responding similarly to a compound. CCI can also be plotted versus time, and CCI curves of cells of different types assayed with one or more test compounds can be compared to determine the similarities or differences in cellular responses of different cell types to a test compound.

*Evaluating the Effect of Different Concentrations of a Compound on Cells*

Figures 9A and B shows the responses of various cell types (listed in Table 1) to doxorubicin treatment as monitored using our cell-substrate impedance monitoring system. The indicated cell lines were seeded onto microtiter devices fabricated with electronic sensor arrays shown in Figure 1. The cellular responses were continuously monitored at 15 or 30 or 60 minutes time interval before and after treatment with doxorubicin. Comparison among these cell index curves showed that certain similarity does exist. Take the treatment of doxoombincin at 3.13 uM as an example. For most of cell types tested, initially after the treatment, cell index increased with time in similar way to the cell index from DMSO control wells. After 10-20 hrs, depending on cell type, the cell index reached a peak and started decreasing with time. From that time on, the cell index monotonically decreases. Such cell index curves - characterized by "going up first and then going down" -were also observed for the cells treated with 5-Fluorouracil. Both Doxorubicin and 5-Fluorouacil act on cells through effects on DNA replication or topology.

Furthermore, such cell index curves are strikingly different from the cell index curves shown in Figure 10A and 10B, where 100 uM of olomoucine resulted in a nearly constant cell index value for 10, 20 even 30 hrs after compound addition. The cell index curves shown in Figure 9 are also strikingly different from the cell index curves in Figure 11, where nM concentration of paclitaxel caused an intial cell index decrease for about 15 hrs (it varies between cell types) and then a cell index increase. These dynamic changes in cell index curves reflect the fact that these different compounds interacts with the cells differently. Compounds that interact with cells in similar way or following same mechanism would result in a similar cell index response curves. One application of this is to investigate the mechanism of compound action based on the observed cell index curves. If cell index responses follow a certain pattern, then one may be able to deduce the mechanism of compound action. Alternatively, if two compounds showed similar, dynamic cell index response curves, then these two compounds may act on the cells with similar or same mechanism of compound action.

**Figure 11A and 11B** shows the responses of various cell types (listed in Table 1) to paclitaxel treatment as monitored using our cell-substrate impedance monitoring system. The indicated cell lines were seeded onto microtiter devices fabricated with electronic sensor arrays shown in Figure 1. The cellular responses were continuously monitored at 15 or 30 or 60 minutes time interval before and after treatment with paclitaxel. Comparison among these cell index curves showed that certain similarity does exist. Take the treatment of palitaxel at 0.78 - 12.5 nM range as examples.

Typically, such nM paclitaxel treatment resulted in an initial decrease in cell index for about 15 - 20 hrs. For one particular cell index curve, after the cell index reached a minimum, it then reversed its decreasing trend and started to increase. Such "going down and then going up" feature in cell index curves was also observed in cell index curves for cells treated with vinblastin or colcemid. Examples of cell index curve for vinblastin-treated cells are shown in Figure 16A and Figure 22. All these compounds - i.e., paclitaxel, vinblastin and colcemid, are so called mitotic poisons and follow similar mechanism of drug action. For example, both vinblastin and paclitaxel act on microtubule dynamics within a cell.

In addition, for a given assay time point, cell index (including normalized cell index or delta cell index), can be plotted versus compound concentration. Such dose response relationships can be used to derive a time-dependent IC5, IC10, IC20, IC30, IC40, IC50, IC60, IC70, IC80, IC90, or IC95. In some preferred embodiments, a time-dependent IC50 is calculated for a compound. Determining a range of time-dependent IC50s for a compound provides information on when the effect of the compound on cells is maximal.

The CI derived from impedance data from wells comprising different cell types and a test compound can be used to derive cell change index (CCI) values for assay time points. CCI values of particular cell types at assay time points can be compared. Such comparisons can indicate whether different cell types are responding similarly to a compound. CCI can also be plotted versus time, and CCI curves of cells of different types assayed with one or more test compounds can be compared to determine the similarities or differences in cellular responses of different cell types to a test compound.

For example, the time frame, magnitude, and duration of a difference in response as evidenced by the curves can indicate a difference in efficacy or mechanism of compounds. The impedance differences can reflect differences in, for example, cell attachment or adhesion, cell growth or proliferation; the number of viable cells or dead cells; cytoskeleton organization or function; or the number of cells going through apoptosis or necrosis in response to a test compound.

Preferably, data from impedance monitoring of wells that comprise different cell types are compared. In one preferred embodiment impedance monitoring is performed for different cell types exposed to multiple dose concentrations of a compound. In some embodiments, multiple compounds can be tested with multiple cell types. In some embodiments, multiple compounds at multiple concentrations can be tested with multiple cell types.

Cytotoxicity Profiling

Various approaches may be used in comparing the cytotoxic responses of compounds. A cell index (or cell number index) can optionally be calculated using the impedance values obtained. Time dependent IC50 may be derived for the compounds and comparison between their cytotoxic responses is done by comparing their time dependent IC50 curves based on cell index values. If the IC50 curves follow a similar time-dependent trend, the two compounds may follow a similar mechanism for inducing cytotoxicty effects. In another embodiment of the method described, direct comparison of time-dependent cytotoxic responses of two compounds are done where the concentrations for the two compounds may be the same or may be different. Direct comparison between time-dependent cytotoxic responses may be done by analyzing the slope of change in the measured responses (that is equivalent to the first order derivative of the response with respect to time) and comparing the time-dependent slopes for the two compounds. In another approach, the time-dependent cytotoxic responses may be analyzed for their higher order derivatives with respect to time. Comparing such high order derivatives may provide additional information as for the mechanisms of compound-induced cytotoxicity.

Analyzing real-time cytotoxicity response may include the derivation of time-dependent IC50 values for the compound on the multiple cell types. In another method, analyzing real-time cytotoxicity response may include derivation of the slope of change in the time dependent cytotoxicity response at a given compound concentration. In yet another method, analyzing real-time cytotoxicity response may include derivation of high-order derivatives of the time dependent cytotoxicity response with respect to time at a given compound concentration.

In yet another method cytotoxicity profiling of multiple compounds is performed on multiple cell types.

Analyzing real-time cytotoxicity response may include derivation of the slope of change in the time dependent cytotoxicity response at a given compound concentration. In yet another method, analyzing real-time cytotoxicity response may include derivation of high-order derivatives of the time dependent cytotoxicity response with respect to time at a given compound concentration.

Some examples of compound assays that can be performed using a cell-substrate impedance system are provided by way of illustration with reference to the figures. In these examples, cell index is calculated using the same method as the Cell Index calculation method (A) as described in Section C. In some of the figures, Normalized Cell Index was plotted. The Normalized Cell Index at a given time point is calculated by dividing the Cell Index at the time point by the Cell Index at a reference time point. Thus, the Normalized Cell Index is 1 at the reference time point.

If cell attachment conditions remain unchanged or exhibit little change over the course of an assay that uses impedance monitoring, then the larger the cell index, the larger the number of the cells in the wells. A decrease in cell index suggests that some cells are detaching from the substrate surface or dying under the influence of the compound. An increase in cell index suggests that more cells are attaching to the substrate surfaces, indicating an increase in overall cell number.

Figure 5 shows curves that represent the time-dependent cell index for H460 cells treated with different concentrations of the anticancer drug paclitaxel. In this experiment, H460 cells were introduced into wells of a 16X cell-substrate impedance monitoring device. The device was positioned on a device station that was located in an incubator maintaining conditions of 37 degrees C and 5% $CO_2$. The cells were cultured and treated at their exponential growth phase with different concentrations of paclitaxel. The dynamic response of the cells to different doses of paclitaxel was monitored by monitoring cell-substrate impedance in real time every 15 minutes for 50 hours after treatment using a cell-substrate impedance monitoring system. The cell-substrate impedance monitoring system calculated the cell index at each time point monitored and plotted the cell index as a function of time. For paclitaxel concentrations between 67 nanomolar and 500 nanomolar, H460 cells exhibited a gradual decrease in cell index after compound addition. However, the cell index reached a minimum at a time dependent on the compound concentration, between about 15 hours and 20 hours after compound addition. After that point, there was a gradual increase in cell index in these wells. The cell index for compound concentration of 33 nanomolar exhibited a near-constant value for up to about 15 hours after compound addition. After 15 hours following compound addition, the cell index exhibited a gradual increase.

Information about cell responses to the compound includes, but is not limited to, information about cell attachment or adhesion status (e.g. the degree of cell spread, the attachment area of a cell, the degree of tightness of cell attachment, cell morphology) on the substrate including on the electrodes, cell growth or proliferation status; number of viable cells and/or dead cells in the well; cytoskeleton change and re-organization and number of cells going through apoptosis and/or necrosis. Information about cell status may also include any compound-cell interaction leading to any change to one or more of above cell status indicators. For example, if the compound binds to a receptor on the cell surface and such binding leads to a change in cell morphology, then the binding of compound to the receptor can be assayed by the monitored cell-substrate impedance. The cell-based assays that be performed with above methods include, but not limited to, cell adhesion, cell apoptosis, cell differentiation, cell proliferation, cell survival, cytotoxicity, cell morphology detection, cell quantification, cell quality control, time-dependent cytotoxicity profiling, IgE-mediated cell activation or stimulation, receptor-ligand binding, viral and bacterial toxin mediated cell pathologic changes and cell death, detection and quantification of neutralizing antibodies, specific T-cell mediated cytotoxic effect, cell-based assay for screening and measuring ligand-receptor binding.

Figure 6 shows curves that represent the time-dependent cell index for H460 cells treated with anticancer drug AC 101103. H460 cells were introduced into wells of a 16X cell-substrate impedance monitoring device. The device was positioned on a device station that was located in an incubator maintaining conditions of 37 degrees C and 5% $CO_2$. The cells were cultured and treated at their exponential growth phase with different concentrations of AC 101103. The dynamic response of the cells to different doses of AC101103 was monitored by measuring impedance in real time every 30 minutes for about 20 hours after treatment on the cell-substrate monitoring system.

Notably, the time-dependent cell index in Figure 6 is significantly different from those shown in Figure 5. For compound concentrations at 3.125 microgram/ml, 6.25 microgram /ml and 12.5 microgram /ml, the cell index exhibited a near-constant value for about 5 hrs, about 15 hrs and > 20 hrs respectively. For compound concentrations at 3.125 microgram /ml and 6.25 microgram/ml, the cell index started to increase after about 5 hrs and about 15 hrs following compound addition. For the compound concentration of 25 microgram/ml, there was a gradual, yet slow decrease in the cell index after compound addition. For the compound concentration of 50 microgram /ml, there was an about 10 hr time period over which the cell index remained near-constant, and after that, the cell index decreased steadily.

Figure 7 shows dynamic drug response curves of A549 cells treated with doxorubicin. 10,000 A549 cells were seeded into each well of a 16 X device. The device was positioned on a device station that was located in an incubator maintaining conditions of 37 degrees C and 5% $CO_2$. Cell attachment and cell growth were monitored on a cell-substrate impedance system in real time before treatment by monitoring impedance at regular intervals. When the cells were in exponential growth phase, doxorubicin at different concentrations was added to the wells. The same volume of the solvent used to dissolve the drug was added to some wells as a control. The time, and drug dose dependent cell response (calculated as cell index) to doxorubicin was recorded in real time on the cell-substrate impedance monitoring system as shown in this figure.

Real-time cell based assays to identify a compound capable of interacting with a G-Protein coupled receptor (GPCR)

The methods may be applied to identifying one or more compounds that are capable of interacting with a G-protein coupled receptor. More specifically the methods may be applied to identify compounds that affect a GPCR such as but not limited to stimulation or inhibition of the GPCR. The methods may identify GPCR agonists, inverse agonists, antagonists and the like. The methods may be used to identify compounds that affect known GPCRs and orphan GPCRs.

The methods include cell-impedance technology to assess and quantify the morphological changes that occur in cells or cell lines that are expressing appropriate GPCR in response to their cognate ligand or agonist using cell-substrate impedance technology. It has been shown that multiple GPCRs and GPCR-mediated signaling pathways converge upon and induce rearrangement of the actin cytoskeleton at the periphery or an increase in stress fiber formation. GPCR-mediated actin cytoskeleton rearrangement has been shown to be mediated by activation of tyrosine kinases and members of the rho family of GTPases (Luttrel, LM. Mol Cell (2002) 9:1152-4.). We have utilized the cell shape changes (the changes in cell morphology) that occur as a result of GPCR-mediated rearrangement of the actin cytoskeleton as readout for GPCR function using cell-substrate impedance technology.

The methods can screen for natural ligands or agonists of the appropriate GPCR utilizing electronic measurement and sensing of cells. One approach for electronic measurement of cells is based on the measurement of cell-substrate or cell-electrode impedances. The approach features in the integration of cell biology with microelectronics and is based on the electronic detection of biological assay process. The details of this cell electronic sensing technology, called real-time cell electronic sensing (RT-CES), and associated devices, systems and methods of use have been provided inWO 2004/010103; United States patent 7,470,533; WO 2005/047482; U.S. Patent 7,192,752; WO 2005/077104; U.S. Patent7,560,269.

For measurement of cell-substrate or cell-electrode impedance using RT-CES technology, microelectrodes having appropriate geometries are fabricated onto the bottom surfaces of microtiter plate or similar device, facing into the wells. Cells are introduced into the wells of the devices, and make contact to and attach to the electrode surfaces. The presence, absence or change of properties of cells affects the electronic and ionic passage on the electrode sensor surfaces. Measuring the impedance between or among electrodes provides important information about biological status of cells present on the sensors. When there are changes to the biological status of the cells analogue electronic readout signals are measured automatically and in real time, and are converted to digital signals for processing and for analysis. In a RT-CES system, a cell index is automatically derived and provided based on measured electrode impedance values. The cell index obtained for a given well reflects : 1) how many cells are attached to the electrode surfaces in this well, 2) how well cells are attached to the electrode surfaces in this well. Thus, the more the cells of same type in similar physiological conditions attach the electrode surfaces, the larger the cell index. And, the better the cells attach to the electrode surfaces (e.g., the cells spread-out more to have larger contact areas, or the cells attach tighter to electrode surfaces), the larger the cell index.

The method can screen for ligands or agonists of GPCR being expressed by appropriate cell lines using measurement of cell-substrate impendence. The method is based on quantification in real time of the cytoskeletal changes and/or morphological changes that arise as a response to ligand or agonist binding to the exogenous or endogenous GPCR being expressed on the surface of an appropriate cell line or primary cells. Because the electronic assay readout relies on cytoskeletal dynamics and/or cell morphology and/or cell adhesion property, all of which are intrinsic cell responses to the activated GPCR, it precludes the need for establishing reporter cell lines or using any other reagent. Furthermore, since the assay is performed in real time, multiple treatments can be performed within the same experiment and receptor desensitization can be assessed.

The invention provides a method of identifying a compound capable of interacting with a G-Protein Coupled Receptor (GPCR) comprising: a) providing a device capable of measuring cell-substrate impedance; wherein said device is in the format of a multi-well plate, each well comprising a nonconductive substrate over which is fabricated a conductive electrode array that is capable of attachment by a cell, further wherein said device is operably connected to an impedance analyzer; b) adding test cells to at least two wells for attachment to said electrode array, wherein said test cells express a GPCR; c) determining first cell indices from first impedances, wherein said first impedances are cell-substrate impedances measured from said at least two wells immediately preceding step d), wherein the cell indices positively correlate with the number of viable cells in the wells, how well they are attached to the electrode surface, and the cell/electrode contact area due to the cell morphology status; d) adding a compound to at least one well containing said test cells to form at least one compound well and adding a vehicle control to at least another well containing test cells to form at least one control well; e) determining second cell indices from said second impedances, wherein said second impedances are cell-substrate impedances measured from said at least one compound well and said at least one control well after step d), wherein the cell indices positively correlate with the number of viable cells in the wells, how well they are attached to the electrode surface, and the cell/electrode contact area due to the cell morphology status; f) determining the change in cell index for said at least one compound well by comparing said second cell index of said at least one compound well to said first cell index of said at least one compound well, and determining the change in cell index of said at least one control well by comparing said second cell index of said at least one control well to said first cell index of said at least one control well; h) comparing said change in cell index between said at least one compound well and said at least one control well; and g) identifying said compound interacts with said GPCR if said comparison demonstrates a significant difference between said change in cell index of said at least one compound well and said change in cell index of said at least one control well.

*Devices for measuring cell-substrate impedance*

The devices used with the methods include devices capable of measuring or monitoring cell-substrate impedance. The cell-substrate (or cell-electrode) impedance is related to not only to the number of the cells but also to the morphology and adhesion quality of the cells on the electrode surfaces. The higher the cell number on the electrode surface is, the larger the cell-substrate impedance (resistance). Similarly, the more spread out the cells are on the electrode surfaces, the larger the cell-substrate impedance (resistance). Likewise, the tighter the cell adhesion to the electrode surfaces, the larger the cell-substrate impedances (resistance). The methods provide for monitoring the activation of GPCR in living cells based on a change in cell morphology and/or cell adhesion occurred when GPCR is activated.

Examples of suitable devices for monitoring or measuring cell-substrate impedance are previously described in the present application. In preferred embodiments the devices frequently contain at least two wells, at least one well forming a test well or a compound well and at least one well forming a control well or a confirmation well. One skilled in the art would recognize that multiple wells may be utilized for one or more assays. For example the device may be in the format of a multi-well plate. In this embodiment multiple assays may be performed simultaneously. Each of the wells may contain a conductive electrode array. As a non-limiting example, a multi-well plate may be a 16 well plate, a 24 well plate, a 96 well plate, a 384 well plate, and a 1536 well plate.

As another nonlimiting example, a device capable of monitoring or measuring substrate impedance may include but is not limited to a nonconducting substrate, two or more electrode arrays fabricated on the substrate, wherein each of the two or more electrode arrays includes two electrode structures; the two or more wells on the substrate, wherein each of the two or more arrays is associated with one of the two or more wells, and at least two connection pads, each of which is located on an edge of the substrate, wherein for each of the two or more electrode arrays, each of the two electrode structures includes multiple electrode elements and the first of the two electrode structures of each of the at least two electrode arrays is connected to one of the at least two connection pads, and the second of the two electrode structures of each of the at least two electrode arrays is connected to another of the at least two connection pads, further wherein at least two of the two or more electrode arrays share one common connection pad, further wherein each electrode array has an approximately uniform electrode resistance distribution across the entire array, and further wherein said substrate has a surface suitable for cell attachment or growth, wherein the cell attachment or growth on the substrate can result in a detectable change in impedance between or among the electrode structures within each electrode array.

*The GPCR superfamily*

The methods may be used to identify compounds that affect a variety of GPCRs. The human genome project has identified a number of proteins which can be categorized into GPCRs based on sequence. The number of GPCRs encoded by the human genome is estimated to be between 800-1000 and thus far approximately 650 GPCR have been identified from the effort of the human genome project, 200 of which are classified as known GPCRs because the activating ligands for these receptors are known (Nambi, P and Nambi, A. Assay and Drug Development Technologies (2003) 1, 305-310). The remaining receptors for which the ligands are not known are considered "orphan receptors" and they are the subject of intense scrutiny as potential medically relevant targets. There are a number of *in vitro* and cell-based assays available which are used to screen for potential agonist or antagonist of GPCRs. The *in vitro* assays are based on binding studies with labeled ligand and receptor. (Nambi, P and Nambi, A. Assay and Drug Development Technologies (2003) 1, 305-310)The cell-based assays are based on engineering cell lines to express exogenous GPCRs alone or together with a reporter plasmid. Calcium sensitive dyes have been used extensively to screen for GPCRs that increase intracellular calcium levels in response to agonists challenge. Alternatively, a fluorescent or luminescent-based reporter assay co-transfected with the appropriate GPCR and G-protein has also been used to identify potential agonists or antagonists of the transfected GPCR (Nambi, P and Nambi, A. Assay and Drug Development Technologies (2003) 1, 305-310). While these assays are extremely useful in high throughput screening to identify potential agonists and antagonists, they do involve pre-labeling the cells with fluorescent dyes in the case of calcium-based assays or lysing the cells to measure the activity of reporter genes.

The method addresses a variety of difficulties in identifying compounds that affect GPCRs or the GPCR pathway. As a nonlimiting example, the method is useable with a recombinant GPCR, an endogenous GPCR, an orphan GPCR, a constitutively active GPCR, a chimeric GPCR, or other chimeric receptors containing a GPCR property.

*Test cells*

The methods refer to adding test cells to at least one well of a device capable of monitoring or measuring cell-substrate impedance. Test cells refer to cells that express the receptor of interest. In the instance of the methods of identifying a compound that affects a GPCR, the test cells would express the GPCR of interest. Test cells may express a variety of receptors such as but not limited to a recombinant GPCR, an endogenous GPCR, an orphan GPCR, a constitutively active GPCR, a chimeric GPCR, or other chimeric receptors containing a GPCR property.

Test cells may be added to the wells using any suitable method known in the biological arts for cell transfer or high throughput cell transfer such as but not limited to aliquoting, pipeting or transferring cells. Test cells may be added at a predetermined concentration or not.

*Compounds added to test wells*

The methods include adding one or more compounds to test cells, control cells and the like to determine the effect of the compound on a GPCR or GPCR pathway. A well containing a compound may be referred to as a compound well for clarity. The compound may be a particular compound of interest or may be a compound from a library of compounds. As nonlimiting examples, the compound may be believed to be a potential ligand, an agonist, an inverse agonist or an antagonist or inhibitor for a GPCR. The compound may be added at a single concentration or at a variety of concentrations. In some embodiment dose-response curves are obtained by adding a compound in more than one concentration and correlating the response in cell-substrate impedance or a cell index (as obtained from the cell-substrate impedance measurement) to the compound concentration. Cell responses to a compound may result in a change (for example, increase or decrease) in cell-substrate impedance or in cell index. Furthermore, the EC50 or IC50 of a compound may be determined by identifying the concentration of compound that results in approximately 50% of maximum responses in cell-substrate impedance or cell index.

*Measuring or monitoring impedance (cell-substrate impedance)*

The methods monitor impedance over time while adding one or more compounds to test cells, control cells, confirmation cells and the like. As previously described impedance may be monitored continually or at desired time intervals or time points. The methods describe an impedance as a first impedance, a second impedance, a third impedance and the like. Each of the referenced impedance measurements may be a single impedance measurement or a series of impedance measurements. For example a first impedance measurement may be taken after adding test cells to at least two wells and before adding a compound or a vehicle control to the wells. In this instance the first impedance measurement may function as a type of control to determine the change in impedance of a single well upon the addition of a compound or vehicle control. Thus, multiple impedance measurements may be taken prior to adding a compound, and may be referred to as a first impedance measurement. Since a cell index may be determined for an impedance measurement, a series of cell indexes (i.e., cell indices) may be determined corresponding to the series of impedance measurements, where each of the first cell indexes corresponds to a distinct impedance measurement. Similarly, a second impedance may be measured or monitored after adding a compound to the at least one well containing a test cell (frequently referred to as forming a compound well). In this instance, the second impedance measures the cellular response or detects changes corresponding to the addition of a compound. The second impedance measurement may therefore reflect an impedance measurement at a time point after addition of a compound. Depending on the desired assay, the second impedance measurement may contain one or more measurements. A series of three or more impedance measurements at three or more time points may be performed to provide a time-dependent, impedance curve or cell index curve that may depict the time dependent changes to the test cells or control cells due to the addition of the compound (or maybe due to the interaction of the compound with a GPCR). When multiple measurements at multiple time-points are taken or the impedance is routinely monitored, a peak in cell-substrate impedance (cell-substrate resistance) or a peak in cell index or a peak in normalized cell index or a peak in delta cell index may be identified. The peak may be a maximum or a minimum impedance (e.g. resistance) out of all the impedance values measured after addition of the compound. Or, the peak may be a maximum change (e.g, relative change or absolute change) in cell-substrate impedance with respect to the impedance before addition of the compound. Or the peak may be a maximum change in cell index or normalized cell index or delta cell index out of all the cell indexes or normalized cell indexes determined from the measurement of cell-substrate impedance after addition of the compound. For each concentration of the compound, such a peak may be identified and may be plotted versus the compound concentration to determine a dose-response curve. Thus, the second impedance measurement may be a series of impedance measurements optionally taken to identify a dose curve. Alternatively, the area under the cell-index or impedance curve for each concentration of the compound can also be determined and used in generating a dose-response curve. Here cell-index curve or impedance curve refers to time-dependent cell-index (or time-dependent impedance) plotted versus time after addition of the compound. In a non-limiting example, the second impedance may be taken at a time point including but not limited to a time point such as more than 1 minute, more than 5 minute, more than 30 minute, more than 1 hour, more than 2 hours, more than 5 hours, more than 10 hours, and more than 24 hours after adding the compound or vehicle control.

Measuring or monitoring impedance at a given time point does not mean that the impedance measurement took place at a single time moment. Indeed, measuring an impedance may take a short period of time, for example, 1 second, or 100 milliseconds, or one millisecond, or a one microsecond, or even less than one microsecond. Thus, such a measurement time is not taken into account and measuring impedance at a given time point may refer to a time point just before the starting moment of the impedance measurement, or just after the end of the impedance measurement, or any time moment in between.

Monitoring impedance may include performing one or more impedance measurements at one or more time points or a series of measurements at a series of time points. Monitoring impedance may include performing impedance measurements at multiple frequencies. Impedance measurement includes the measurement of resistance and/or reactance. Depending on the desired assay, impedance may be monitored or measured at regular or irregular time intervals during an assay period.

In some embodiments an impedance measurement is performed at a time point or time points before adding a compound or a vehicle control or immediately prior to adding a compound or a vehicle control to the cells. In one exemplary embodiment, impedance measurement may be performed at a time point of less than less than 30 minutes, less than 1 hour, less than 2 hours, less than 4 hours, or less than 10 hours, or less than 24 hours, or other time length prior to adding a compound or vehicle control to the cells. In another exemplary embodiment, impedance measurement may be performed at a time point immediately preceding or prior to adding a compound or a vehicle control, such as less than 20 minutes, less than 10 minutes, less than 5 minutes, less than 2 minutes, or less than 1 minute prior to adding the compound or vehicle control. A vehicle control in this context refers to a medium or solution or solvent that has the same composition as the medium or solution or solvent in which a test compound is placed, but without the test compound.

In still another exemplary embodiment, impedance measurement may be performed at regular or irregular time intervals prior to addition of a test compound or a vehicle control. As can be envisioned, a series of two, three, four or more measurements may be desired after adding a compound. In one exemplary embodiment, impedance measurement may be performed at regular time intervals (for example, every 15 seconds, or every minute, or every 15 minutes) after adding a compound. In another exemplary embodiment, impedance measurement may be performed at irregular time intervals (for example, initially every 2 minutes up to 1 hour and followed by every 30 minutes up to 24 hours) after adding a compound. In still another exemplary embodiment, impedance measurement may be performed at a time point of more than 15 seconds, more than 1 minute, more than 5 minute, more than 30 minutes, more than 1 hour, more than 2 hours, more than 5 hours, more than 10 hours after adding a compound.

The methods identify a compound capable of interacting with a G-Protein Coupled Receptor (GPCR) in test cells using measurement of cell-substrate impedance. It is based on impedance-based quantification of change in cell morphology (and/or cell adhesion) which arise as a response to compound interacting with (for example, binding to) a GPCR, causing cellular changes, including but not limited cytoskeleton rearrangement. Because the impedance readout relies on the interaction of the test cells with the sensor electrodes, the change in cell morphology and or cell adhesion due to the compound interacting with a GPCR leads to a time-dependent change (for example, increase or decrease) in the cell-substrate impedance which correlates with the interaction of the compound with the GPCR, or with GPCR activation.

*Determining cell index*

The methods include comparing one or more cell indices (i.e., cell indexes) or cell index values. In one embodiment, a cell index is determined by calculating, for each measurement frequency, the relative-change in resistance (a component of impedance) of a well when the cells are present in the well with respect to that of the well when no cell is present, and then finding the maximum relative-change in resistance for all frequencies measured. The maximum relative-change in resistance is used as cell index (see equation (4) in Section **C. Methods for Calculating Cell Index (CI) and Cell Change Index** (**CCI**)). If impedance is measured at a single frequency, then the relative change in resistance (a component of impedance) of a well when compounds are present in the well with respect to that of the well when no compound is present, or when only test cells or only control cells are present. Other methods for calculating cell index have been disclosed in a previous Section **C. Methods for Calculating Cell Index (CI) and Cell Change Index (CCI).** Since the cell index or cell indexes correspond to an impedance measurement or impedance measurements, the methods envision comparing cell indexes for the same well measured at different time points to determine a change in cell index. For example, one may compare cell indexes derived at time points before and after adding a compound that interacts with or is suspected to interact with a GPCR in the cells to determine the change in cell index as a result of adding the compound. The methods may also compare changes in cell indexes between different wells, for example, a compound well and a control well. As with impedance measurements, a first cell index may be one or more cell indexes, a second cell index may be one or more cell indexes, a third cell index may be one or more cell indexes and the like. When determining a series of cell indexes, a single cell index may be utilized for a distinct cell impedance or impedance measurement.

As previously demonstrated, cell indexes may be normalized for comparison. Normalization may take into account the number of cells in a particular well, variables in detecting impedance. Although methods of normalization may vary, in some embodiments, the normalization is performed at a time point a short time before doing a treatment on cells, for example, adding compound or a vehicle control. As nonlimiting examples, a short time may be less than 1 minute, less than 2 minutes, less than 5 minutes, less than 10 minutes, less than 30 minutes, less than 1 hour, less than 2 hours, less than 5 hours, less than 10 hours and less than 24 hours.

*Determining changes in impedance or cell index*

The methods determine a cell index before and after adding a compound suspected of being capable of affecting a GPCR. In preferred embodiments the change in cell index of a single well is determined. As nonlimiting examples, a change in impedance may be determined for a compound well, a test well, a control well, a confirmation well and the like. Determining the change in cell indes may be performed by comparing a second cell index of a particular well measured or derived at a second time point to the first cell index of the same well measured or derived at a first time point.

The change in cell index may be derived by subtracting the first cell index of a particular well measured at a first time point from the second cell index of the same well measured at a second time point. The change in cell index derived this way is referred as "absolute change" in cell indices (cell indexes). In one example of this embodiment, a particular well is a compound well, the first time point is a time point immediately preceding adding a compound to the cells in the compound well and the second time point is a time point after adding the compound. For this example, the "absolute" change in cell index for the compound well reflects the cell responses to the addition of the compound. In another example of this embodiment, a particular well is a control well, the first time point is a time point immediately preceding adding a vehicle control to the cells in the control well and the second time point is a time point after adding the vehicle control. For this example, the "absolute" change in cell index for the control well reflects the cell responses to the addition of the vehicle control. The absolute change in cell index may also be referred as "delta cell index" if the first time point is a standard time point and the cell index at the standard time point is used for calculating "absolute change in cell index" for multiple second time points. Furthermore, cell index in embodiment here may be a normalized cell index.

In another embodiment the change in or cell index may be derived by two subsequent steps: (1) determining "absolute change" in cell indexes by subtracting the first cell index of a particular well measured at a first time point from the second cell index of the same well measured at a second time point; (2) dividing the "absolute change" in cell indexes by the first cell index of the same well measured at the first time point. The change in cell indices (cell indexes) derived this way is referred as "relative change" in cell indexes. In one example of this embodiment, a particular well is a compound well, the first time point is a time point immediately preceding adding a compound to the cells in the compound well and the second time point is a time point after adding the compound. For this example, the "relative" change in cell index for the compound well reflects the cell responses to the addition of the compound. In another example of this embodiment, a particular well is a control well, the first time point is a time point immediately preceding adding a vehicle control to the cells in the control well and the second time point is a time point after adding the vehicle control. For this example, the "relative" change in cell index for the control well reflects the cell responses to the addition of the vehicle control.

As described previously, monitoring impedance may include performing impedance measurements at multiple frequencies. Impedance measurement includes the measurement of resistance and/or reactance. Thus, a change in impedance may refer to a change in impedance at a particular measurement frequency, or may refer to a maximum change in impedance for multiple measurement frequencies, or may refer other parameters that may be derived from the change in the frequency spectrum of the impedance. Furthermore, a change in impedance may be a change in resistance, or reactance, or resistance and reactance.

*Comparing changes in impendance or cell index*

Changes in cell index are compared between test samples and control samples. For example, a change in cell index for a compound well as a result of adding a compound to the cells in the compound well may be compared to the change in cell index for a control well as a result of adding a vehicle control to the cells in the control well. If such a comparison demonstrates a significant difference (for example, more than 0.5%, or more than 1%, or more than 10%) between the compound well and the control well, then change in cell index for the compound well is significantly different from the change in cell index for the control well. We may identify that the compound may interact with the cells. If the cells express a GPCR, we may identify that the compound may interact with the GPCR in the cells.

In another example, a change in cell index for a test well as a result of adding a compound to the test cells in the test well may be compared to the change in cell index for a control well as a result of adding the same compound to the control cells in the control well. If such a comparison demonstrates a significant difference (for example, more than 0.5%, or more than 1%, or more than 10%) between the test well and the control well, then change in cell index for the test well is significantly different from the change in cell index for the control well. We may identify that the compound may interact with the test cells differently from the interaction of the compound with the control cells. If the test cells express a GPCR whilst the control cells do not express the GPCR or express the GPCR at a significantly lesser level than the test cells, we may identify that the compound may interact with the GPCR in the test cells.

In another example, a change in cell index for a test well as a result of adding a compound to the test cells in the test well may be compared to the change in cell index for a confirmation well as a result of adding a vehicle control to the test cells in the confirmation well. In still another example, a change in cell index may be compared between multiple compound wells such as multiple compound wells containing the compound at a variety of dilutions or concentrations.

*Identifying compounds of interest*

Compounds that interact with GPCRs are identified by determining which compound(s) cause significant changes in cell index. For example, when screening or identifying a compound capable of affecting a GPCR, a compound may be identified if a significant change is observed between the change in cell index of the compound well in comparison to the change in cell index of a control well. Here both compound well and control well have the same test cells expressing a GPCR. The compound is added to the compound well whilst a vehicle control is added to the control well. For the compound well, the change in cell index refer to the change occurred after adding the compound. For the control well, the change in cell index refer to the change occurred after adding the vehicle control. As a nonlimiting example, a compound may be identified as an agonist to a GPCR if the comparison indicates a significant change including an increase or a decrease in cell index for the compound well after the compound is added to the test cells that express the GPCR, as relative to the control well. A compound may be identified as an inverse agonist if the comparison indicates a significant change including a decrease or increase in cell index for the compound well after the compound is added as relative to the control well, and the significant change in cell index for the compound well is in the OPPOSITE direction compared to the change of a known agonist. For example, a known agonist of a GPCR may result in a significant increase in cell index after addition of the agonist to the cells expressing the GPCR. For this GPCR in the same cells, a compound is identified as an inverse agonist if the compound results in a significant decrease in cell index after addition of the compound. In another example, an agonist may result in a significant decrease in cell index and an inverse agonist may result in a significant increase in cell index.

Compounds may be added to multiple wells to determine a dose curve or dose response curve. Each concentration of a compound results in a change in cell index after addition of the compound. Such a change in cell index may be determined or derived at multiple time points. Thus, a time-dependent change of cell index (a trace in cell index change) may be derived for each concentration of the compound. To provide a dose-response curve, one may define or derive a single parameter to reflect the cellular response or cellular change for each concentration of the compound. In one preferred embodiment a single parameter for reflecting the cellular response or cellular changes is the maximum change in cell index after addition of the compound and a dose-response curve may be constructed by plotting a maximum change in cell index for each concentration of the compound versus the concentration of the compound. In another embodiment, the area under the cell-index or impedance curve for each concentration of the compound may also be determined and used in generating a dose-response curve. Here cell-index curve or impedance curve refers to time dependent cell-index (or time dependent impedance) plotted versus time after addition of the compound.

In another preferred embodiment, the single parameter for reflecting the cellular response or cellular change is a change in cell index at one particular time point after the compound addition. This particular time point may correspond to the time point at which a maximum change in cell index for the highest concentration of the compound occurs.

With a dose-response curve or a dose curve, one may be able to derive EC50 or IC50 of a compound. In one embodiment, an EC50 is a concentration of a compound capable of inducing 50% of the maximum response in cell index. The maximum response in cell index refers to the maximum change in cell index for all concentrations of the compound, as derived from the dose-response curve or dose curve. Various formula or methods may be used to derive EC50 or IC50 from a dose response curve for a compound. Those skilled in biostatistics or in the analysis of biological data may readily choose appropriate formula or methods to calculate an EC50 or IC50 from a dose curve. For example, experimental dose curve may be fitted into a sigmoid curve and an EC50 or IC50 may be derived from the curve fitting.

*Alternative methods of identifying compounds that affect the GPCR*

The invention also provides a method of identifying a compound capable of interacting with a G-Protein Coupled Receptor (GPCR) comprising: a) providing a device capable of measuring cell-substrate impedance; wherein said device is in the format of a multi-well plate, each well comprising a nonconductive substrate over which is fabricated a conductive array that is capable of attachment by a cell, further wherein said device is operably connected to an impedance analyzer; b) adding test cells to at least one of said at least two wells for attachment to said conductive array to form at least one test well, and adding control cells to at least another well for attachment to said conductive array to form at least one control well, wherein said test cells express a GPCR and said control cells do not express said GPCR or express said GPCR at a significantly lesser level that said test cells; c) determining first cell indices from first impedances, wherein said first impedances are cell-substrate impedances measured from said at least one test well and from said at least one control well immediately preceding step d), wherein the cell indices positively correlate with the number of viable cells in the wells, how well they are attached to the electrode surface, and the cell/electrode contact area due to the cell morphology status; d) adding a compound to said at least one test well and to said at least one control well; e) determining second cell indices from said second impedances, wherein said second impedances are cell-substrate impedances measured from said at least one test well and from said at least one control well after step d), wherein the cell indices positively correlate with the number of viable cells in the wells, how well they are attached to the electrode surface, and the cell/electrode contact area due to the cell morphology status; f) determining the change in cell index of said at least one test well by comparing said second cell index of said at least one test well to said first cell index of the said at least one test well, and determining the change in cell index of said at least one control well by comparing said second cell index of said at least one control well to said first cell index of the said at least one control well; g) comparing said changes in cell index between said at least one test well and said at least one control well; and h) identifying said compound interacts with said GPCR if said comparison demonstrates a significant difference between said change cell index for said at least one test well and said change in cell index for said at least one control well.

Compounds that interact with GPCRs are identified by determining which compound(s) cause significant changes in cell index. For example, when screening or identifying a compound capable of affecting a GPCR, a compound may be identified if a significant change is observed between the change in cell index of the test well in comparison to the change in cell index of a control well. Here test well has the test cells expressing a GPCR and control well has control cells not expressing the GPCR or expressing GPCR at a significantly low level. The compound is added to both the test well and the control well. For the test well, the change in cell index refers to the change occurred after adding the compound to the test cells. For the control well, the change in cell index refers to the change occurred after adding the compound to the control cells. As a nonlimiting example, a compound may be identified as an agonist to a GPCR if the comparison indicates a significant change including an increase or a decrease in cell index of the test well after the compound is added to the test cells that express the GPCR, as relative to the control well. A compound may be identified as an inverse agonist if the comparison indicates a significant change including a decrease or increase in cell index of the test well after the compound is added as relative to the control well, and the significant change in cell index for the test well is in the OPPOSITE direction compared to the change of a known agonist. For example, a known agonist of a GPCR may result in a significant increase in cell index after addition of the agonist to the cells expressing the GPCR.

For this GPCR in the same cells, a compound is identified as an inverse agonist if the compound results in a significant decrease in impedance or cell index after addition of the compound. In another example, an agonist may result in a significant decrease in cell index and an inverse agonist may result in a significant increase in cell index.

The provided methods have particular utility to identifying agonists or antagonists for orphan GPCRs. As provided earlier it is predicted that there are between 800-1000 potential GPCR that are coded by the genome and only 200 of which have known ligands. Therefore, in order to identify potentially relevant pharmaceutical targets the pharmaceutical industry has invested extensively in trying to identify the ligands for the orphan GPCR. The methods screen for potential ligands or agonist of an orphan GPCR using-substrate impedance technology. The methods may include test cells engineered to express the appropriate orphan GPCR seeded in either ACEA's 16X or 96X microtiter plates (E-Plates) at a pre-determined cell density, treating the test cells with appropriate ligand concentration from a ligand library or with potential agonist using a compound library. As a control cells that are not expressing the receptor may also be treated with the ligands or potential agonist library. The transient morphological changes of the cells due to ligand or agonist stimulation may be electronically monitored using the RT-CES system. Plotting the ligand concentration against the maximal change in cell index at each ligand concentration will result in a dose-response curve. The maximal change in this context refers to the maximum change in cell index after addition of the compound with respect to the cell index before adding the compound. Alternatively, the area under the cell-index curve for each concentration of the ligand or agonist can also be determined and used in generating a dose-response curve. The cell-index curve refers to time dependent cell-index plotted versus time after addition of the ligand or agonist. The dose-response curve can be used to calculate the EC50 value, which is the molar concentration of the ligand or agonist that produces half of the maximum biological response or half of the maximum response in cell index. The maximum response in cell index refers to the maximum change in cell index for all concentrations of the compound, as derived from the dose-response curve or dose curve. If the actual ligand for the receptor is known, it may be used as a positive control in the assay and the EC50 value of the potential agonists may be compared to the EC50 of the natural ligand. Compounds that do not induce any cell-electrode impedance response (with respect to the response of the control cells) will not be considered as agonists. Compounds that induce a response at the concentration being screened may be further evaluated at different doses to determine their relative EC50 value. Those compounds that illicit an EC50 response which is equal to or less than that of the natural ligand are considered as agonists. Also, compounds that do reach maximum response even at much higher EC50 values may still be considered as potential agonists. Compounds that only induce a partial response (a fraction of the maximum response) may be considered as partial agonists.

*Methods of screening for an antagonist for a G-protein coupled receptor (GPCR) with a known ligand*

The methods may be adapted to screen for an antagonist for a GPCR with a known ligand. For example impedance can be measured before and after stimulation of the GPCR, where a potential antagonist is added prior to stimulation of the GPCR. More specifically a method for screening for an antagonist, includes (a) providing a device capable of measuring cell-substrate impedance, wherein the device comprises at least two wells, further wherein the device is operably connected to an impedance analyzer; (b) adding test cells to each of at least two of the at least two wells, wherein the test cells express a GPCR; (c) adding a compound suspected of being a GPCR antagonist to at least one of the at least two wells to form at least one compound well, adding a vehicle control to at least another well of the at least two wells to form at least one control well; (d) measuring first impedances of the at least one compound well and the at least one control well immediately preceding step (e), and determining first cell indices (cell indexes) from the first impedances; (e) adding a GPCR ligand to the at least one compound well and to the at least one control well; (f) measuring second impedances of the at least one compound well and the at least one control well after step e) and determining second cell indices from the second impedances; (g) determining the change in the cell index for the at least one compound well by comparing the second cell index of the at least one compound well to the first cell index of the at least one compound well, and determining the change in the cell index of the at least one control well by comparing the second cell index of the at least one control well to the first cell index of the the at least one control well; (h) comparing the change in cell index between the at least one compound well and the at least one control well; and (i) identifying the compound is an antagonist for the GPCR if the comparison demonstrates a significant difference between the change in cell index of the at least one compound well and the change in cell index of the at least one control well.

The method can also comprise identifying the compound as an antagonist for the GPCR if the change in cell index of the at least one compound well is significantly smaller than the change in cell index of the at least one control well. In another word, a compound is identified as an antagonist for the GPCR if the compound inhibits or reduces the impedance response of the cells to the stimulation of an agonist or a GPCR ligand.

A method for screening for a potential antagonist of GPCRs with known ligands using cell-substrate impedance technology may include the following steps. (1) Cells expressing the appropriate GPCR will be obtained or engineered to express the appropriate GPCR at adequate levels of expression. (2) The cells will be seeded on ACEA's 16X or 96X microtiter plates (E-Plates) at a predetermined concentration. The attachment and proliferation of the cells will be monitored continuously on the RT-CES system. (3) The cells will be pre-incubated with a pre-determined concentration of potential antagonists or compounds from a library and then stimulated with an appropriate amount of ligand or agonist that will result in optimal response on the RT-CES. As a control, the cells will be pre-incubated with vehicle alone and then stimulated with the appropriate ligand and also the cells will be treated with the compounds, but not stimulated with the ligand or agonist. The cell response will be continuously monitored on the RT-CES. (4) An antagonist may be identified if (a) the cells pre-incubated with vehicle alone and then stimulated with the appropriate ligand give a significant change in impedance or cell index as a result of ligand stimulation; (b) the cells pre-incubated with a potential antagonist and then stimulated with the appropriate ligand gives a significantly smaller change in impedance or cell index as a result of ligand stimulation (i.e., the antagonoist reduces or inhibits the ligand-induced cell responses).

Once potential antagonists that block the agonist or ligand response has been identified, the IC50 values of the potential antagonists, which are the molar concentration of the antagonist which results in 50% inhibition of the maximal cell-electrode impedance response (i.e., leading to that the maximal cell electrode impedance response is reduced by 50%), will be determined. The IC50 will be determined by preincubating the cells which express the appropriate receptors with increasing concentration of the potential antagonist and then stimulating the cells with the appropriate ligand or agonist. As a control, the cells will only be incubated with media or vehicle alone. In addition, if there are any known antagonists for the receptor of interest, those may also be included in the assay as a positive control. Those compounds that block the agonist or ligand maximal response in a dose dependent manner and cause the cells to return to baseline will be considered as potential antagonists. A dose response curve may be constructed by plotting the ligand-induced or agonist-induced cell-impedance response for cells pre-incubated with each concentration of the potential antagonist versus concentration of the potential antagonist. Alternatively, the area under the cell-index curve or impedance curve for each concentration of the potential antagonist can also be determined and used in generating a dose-response curve. Here cell-index curve or impedance curve refers to time dependent cell-index (or time dependent impedance) plotted versus time after addition of the compound. The dose response curves may show that the potential antagonist inhibits or reduces the ligand-induced (or agonist-induced) cell-substrate impedance responses in a dose-dependent manner. From such a dose-response curve, IC50 may be derived or calculated. Those who are skilled in the biostatistics or in the analysis of biological data may readily choose appropriate formula or methods to calculate an IC50 from a dose-response curve of an antagonist. For example, experimental dose curve may be fitted into a sigmoid curve and an IC50 may be derived from the curve fitting.

*Methods of Identifying a Compound that affects a G-protein coupled receptor (GPCR) pathway*

The methods may be adapted to screen or identify a compound that affects a GPCR pathway. For example, the a method of identifying a compound that affects a GPCR pathway includes: (a) providing a device capable of measuring cell-substrate impedance, wherein the device comprises at least two wells, further wherein the device is operably connected to an impedance analyzer; (b) adding test cells to at least two of the at least two wells, wherein the test cells express a GPCR; (c) adding a compound suspected of being capable of affecting a GPCR pathway to at least one of the at least two wells containing the test cells to form at least one compound well, adding a vehicle control to at least another well of the at least two wells containing the test cells to form at least one control well; (d) measuring first impedances of the at least one compound well and the at least one control well immediately preceding step (e), and optionally determining first cell indices from the first impedances; (e) adding a GPCR activating compound to the at least one compound well and to the at least one control well; (f) measuring second impedances of the at least one compound well and the at least one control well after step (e) and optionally determining second cell indices from the second impedances; (g) determining the change in the impedance or cell index for the at least one compound well by comparing the second impedance or the second cell index of the at least one compound well to the first impedance or the first cell index of the at least one compound well, and determining the change in the impedance or cell index of the at least one control well by comparing the second impedance or the second cell index of the at least one control well to the first impedance or the first cell index of the at least one control well; (h) comparing the change in impedance or cell index between the at least one compound well and the at least one control well; and (i) identifying the compound effects the GPCR pathway if the comparison demonstrates a significant difference between the change in impedance or cell index of the at least one compound well and the change in impedance or cell index of the at least one control well.

The method can also further comprise identifying the compound as an inhibitor for the GPCR pathway if the change in impedance or cell index of the at least one compound well is significantly smaller than the change in impedance or cell index of the at least one control well. In another word, a compound is identified as an inhibitor for the GPCR pathway if the compound inhibits or reduces the impedance response of the cells to the stimulation of a GPRC activating compound (for example, an agonist or a GPCR ligand). One may determine a dose-response curve for such a compound that is capable of inhibiting GPCR pathway and determine the IC50 values using the approach similar to that used for deriving IC50 for an antagonist for a G-protein coupled receptor (GPCR) with a known ligand described previously.

**EXAMPLES**

**Example 1. Profiling of dynamic cell responses to anti-cancer drugs using ACEA RT-CES system.**

In this study, we used the RT-CES system to dynamically monitor cancer cell responses to chemotherapeutic compounds with characterized mechanisms, and to profile the specific cell response patterns. Thirteen cancer cell lines including cancers of breast, prostate, lung, colon, ovary, kidney, fibroblast, and central nervous system were tested (Table 1). Each cancer cell type was treated with 11 chemotherapeutic compounds, classified as DNA damaging agents, protein kinase inhibitors, anti-mitotic drugs, cell cycle specific inhibitors, protein synthesis inhibitors plus a compound of unknown category (Table 2). Dynamic and dose dependent cell-compound interaction patterns were characterized and summarized for all the tested cell lines and compounds. The profiles for three drugs, doxorubicin, olomoucine and paclitaxel against a panel of 12 different cell lines are presented in Figures 9, 10 and 11, respectively. In addition, we characterized the biological effect of these compounds on cells by monitoring cell cycle progression, cell viability and morphological status of the cells in an attempt to correlate specific cellular response to the shape of the cell index trace (Figures 12, 13 and 14). Furthermore we calculated the time-dependent IC-50 values for each compound against the various cell lines (Figure 15) and developed an algorithm to calculate Cell Change Index to profile the dynamic cell response of the different chemotherapeutic agents across the different cell lines. Cell Change Index was calculated for the dynamic RT-CES responses of different cell lines to different chemotherapeutic agents using the definitions described above. Based on the time-dependent values of CCI, each CCI value region across the time scale is represented by black-white shading-based coding. For example, if after compound addition, the CCI value (for a particular cell line under a specific compound treatment at the concentration of IC50 value) is nearly zero for certain period of time and then becomes positive, attaining a value about 0.7/DT (DT is doubling). Then the cell response to this compound is represented as a rectangle followed by a rectangle. Examples of such analysis is shown in Figures 16. The overall black-white shading-based coding map representing the cell dynamic responses to various compounds is shown in Figure 17.

In summary of this study, we note that using the RT-CES system to screen chemotherapeutic agents results in unique activity patterns that is dependent on the compound itself, the concentration, length of incubation and the cell type. The "signature" patterns of each drug correlates with specific biological phenomenon such as log growth, cell cycle rest, morphology change and cell death. Cell Change Index was a good parameter derived from RT-CES data to mathematically describe cell changes. Cell response profiling based on CCI value indicates that drugs with similar mechanism of action displays similar patterns. Thus, the similarity in the dynamic cell-compound interaction patterns may indicate similarity in mechanism of action, mode of resistance and possibly molecular targets. The RT-CES system can be readily adapted to high throughput dynamic screening and analysis of anti-cancer compounds and the information-intensive approach presented in this study can be applied to profile existing cancer chemotherapeutic agents, screen new compounds and provide insight into the mechanism of action of anti-cancer agents.

Table I. List of cancer cell lines tested against a number of chemical compounds.

| Cancer Cell Line | Organ or Tissue Origin |
|---|---|
| MDA.MB231 | Breast Cancer |
| MCF7 | Breast Cancer |
|  |  |
| NCI-H460 | Non-Small Cell Lung Cancer |
| MV522 SW | Non-Small Cell Lung Cancer |
| A549 | Non-Small Cell Lung Cancer |
|  |  |
| HT29 | Colon cancer |
| HCC2998 | Colon cancer |
|  |  |
| A2780 | Ovarian Cancer |
| OVCAR4 | Ovarian Cancer |
|  |  |

(continued)

| Cancer Cell Line | Organ or Tissue Origin |
|---|---|
| PC-3 | Prostate Cancer |
| HepG2 | Human Hepatosarcoma |
| A431 | Epidermoid Cancer |
| HT1080 | Fibrosarcoma |

Table II. List of chemical compounds used in the study of profiling cell dynamic responses to a number of anti-cancer compounds.

| Machanisms of Compound Action | Chemical Compounds | concentration |
|---|---|---|
| Effect on DNA replication or Topology | | From High to Low (dilution factor:2) |
| | Doxorubincin | 6.25 uM - 0.098 uM |
| | 5-Fluorouracil | 50 uM - 0.78 uM |
| Mitotic Poisons | | |
| | Colcemid | 3.125 uM - 0.049 uM |
| | Paclitaxol | 0.0125 uM- 0.00019 uM |
| | Vinblastin | 1.56 uM - 0.024 uM |
| Cell Cycle Arrest | | |
| | Olomoucine | 100 uM - 1.56 uM |
| | Roscovitine | 50 uM - 0.78 uM |
| Kinase Inhibitors | | |
| | Staurosporine | 5 uM - 0.078 uM |
| | Tamoxifan | 50 uM - 0.78 uM |
| Protein synthesis Inhibitor | | |
| | Rifampicin | 100 uM - 1.56 uM |
| Unknown type | | |
| | ACEA-1 | |

## Example 2. Cytotoxicity Profiling

### Methods

*Cells.* All the cells used in this study were obtained from ATCC and maintained at 37°C incubator with 5% $CO_2$ saturation. H460, HepG2 and HT1080 cells were maintained in RPMI media containing 5% FBS and 1% penicillin and streptomycin. NIH3T3 cells were maintained in DMEM media containing 10% FBS and 1% penicillin and streptomycin.

*Cell Proliferation Assays.* For each of the cell type, the indicated number of cells was seeded per well in 96X microtiter plates (e-plate™) with incorporated electrode structures in individual wells device in 100 $\mu$L of media. The attachment, spreading and proliferation of the cells were continuously monitored every 30 minutes using the RT-CES™ system (a cell-substrate impedance monitoring system. Cell proliferation was monitored for a period of 48-72 hours depending on the experiment. The electronic readout, cell-sensor impedance is displayed as a parameter called Cell Index.

*Drug Treatment and Cytotoxicity Assessment*. For each cell type the optimum cell concentration was chosen based on their respective proliferation pattern (Figure 18). The indicated cell numbers were seeded per well of ACEA's 16X or 96X e-plate™ in 100 $\mu$L final volume. The attachment, spreading and proliferation of the cells were continuously monitored every 30 minutes using the RT-CES system. Approximately 24 hours after seeding, when the cells were in the log growth phase, the cells were treated with 100 $\mu$L of the indicated compounds dissolved in cell culture media. The cells were also treated with DMSO, which served as vehicle control. Depending on the experiment, the final DMSO concentration in the media was in the range of 0.25%-0.5%.

*MTT Assay.* Increasing numbers of NIH3T3 cells were seeded in 16X e-plate and monitored by RT-CES to obtain the corresponding Cell Index. The media was immediately aspirated and the cells were then assayed by using the standard MTT assay according to the manufacturer's protocol.

*Flow Cytometry.* A549 cells were seeded at a density of 500,000 cells/well in 60 mm tissue culture dishes. Approximately, 24 hours after seeding, the cells were treated with the indicated final concentration of Olomoucine and 16 hours later the cells were washed with PBS, trypsinized, washed twice with PBS and fixed in 70% methanol and stored at 4°C until the staining step. The cells were stained with propidium iodide and analyzed by FACS using a wavelength of 488nm.

**Monitoring Dynamic Cell Proliferation in Real-Time Using the RT-CES**

In order to assess dynamic cell proliferation using the RT-CES system, H460 human lung cancer cells, H1080 fibrosarcoma cells, HepG2 human hepatosarcoma cells and NIH3T3 mouse fibroblasts were seeded at 2500 and 10,000 cells per well in triplicate in ACEA's 96X e-plate™. The cells were continuously monitored every 30 minutes using the RT-CES system for the indicated period of time (Figure 18). As shown in Figure 18, each cell type has its own characteristic kinetic trace, based on the number of cells seeded, the overall size and morphology of the cells and the degree to which the cells interact with the sensor surface. Also, the adhesion and spreading kinetics as well as time when the cells enter the log growth phase is characteristic of each of the indicated cell lines and therefore offers an excellent internal control and a way to standardize and validate stock cultures during different phases of the manufacturing process.

To ascertain that the RT-CES units of Cell Index correlates with the number of the cells in the well, increasing numbers ofNIH3T3 cells were seeded in ACEA 16X e-plate™ and were monitored for up to 10 hours, at which time the Cell Index was acquired. Figure 19A shows a plot of Cell number seeded versus the Cell Index obtained and indicates that for this particular cell type the RT-CES system could detect as little as 100 cells and the readout is linear by two orders of magnitude all the way up to 10000 cells. In addition, at the end of the experiment described in Figure 19A, the cells were also assayed by the MTT assay. As shown in Figure 19B, even at up to 1000 cells the MTT assay is not appreciably different than background values and for cell numbers exceeding 1000, then the MTT units correlates with the number of cells seeded in a linear fashion. However, it is important to remember that while the RT-CES system is capable of dynamic and continuous measurements, for comparative reasons the experiment described in Figure 19 was only conducted at a single point, since MTT is a single point assay.

**Assessment of Drug Interaction with Target Cells Using the RT-CES™ System**

To assess drug potency using the RT-CES system, the IC-50 value of Tamoxifen was determined for different cell lines and compared with MTT assay at 48 hours after Tamoxifen addition. According to Table III, the IC-50 values obtained for Tamoxifen for the different cell lines using the RT-CES system is very consistent with the values obtained by the MTT assay, indicating that the RT-CES system can be used to assess the potency of various drugs against different adherent cell lines.

In order to observe the kinetics of drug interaction with target cells, A549 non-small lung cancer cells were seeded in ACEA 96X e-plate™ and continuously monitored until the cells reached the log growth phase at which point different concentrations of paclitaxel were added to the cells at the indicated final concentration. As shown in Figure 20A, paclitaxel at the highest concentration initially induces a cytotoxic effect which is mainly due to cell death as judged by Annexin V staining (Figure 20B). Remarkably, the cells recover from the initial cytotoxic effect of the drug and start to re-proliferate. While it remains to be determined if this phenomenon is due to metabolism and inactivation of paclitaxel or due to the emergence of paclitaxel-resistant subpopulation, this experiment clearly exemplifies the tremendous advantage of real-time measurement which is offered by the RT-CES system and allows the user to the opportunity to observe and assess the entire history of drug interaction with the target cells which provides further information in addition to cell viability or cytotoxicity. The phenomenon observed in Figure 20A would've been easily missed by traditional single-point assays such as MTT.

Yet another major advantage of using the RT-CES system to continually monitor the interaction of drugs with target cells is that the user can obtain insight into the mechanism of action of the drug of interest. To demonstrate this point, A549 cells were seeded in ACEA 96X microtiter device and continually monitored by the RT-CES. The cells were treated with either DMSO as the vehicle control or with 100 $\mu$M Olomoucine which is a CDK inhibitor and induces cell cycle arrest either at G1$\rightarrow$S transition or at the G2$\rightarrow$M transition, depending on the cell line. As shown in Figure 21A addition of Olomoucine to exponentially growing A549 cells causes the trace of the Cell Index recordings of the cells to level off and remain in a steady state that is reminiscent of cell cycle block, where the cells are neither proliferating nor dying off. The control cells treated with DMSO continue to proliferate until they reach confluence, at which time they are contact inhibited and the Cell Index recording levels off. To demonstrate that the effect of Olomoucine on A549 cells as monitored by the RT-CES was indeed due to an arrest of the cell cycle, A549 cells growing on tissue culture dish were treated with the same concentrations of DMSO and Olomoucine and subjected to flow cytometry analysis. As shown in Figure 21B, the flow cytometry analysis indicates that treatment of A549 cells with the indicated concentration of Olomoucine induces cell cycle arrest at the G2→M transition, where CDKs such as CDK2 is active. Taken together, using the RT-CES system to dynamically monitor drug interaction with the target cells offers the user the opportunity to understand the mechanism of drug action and its mode of interaction with the target cell.

In order to assess the RT-CES system for analysis of cytotoxicity, the interaction of A549 cells was examined with cytotoxic agents with different mechanism of action. Figure 22 shows the characteristic trace of A549 cells monitored by RT-CES™ and treated with different concentrations of 5-fluorouracil, vinblastine and staurosporine. According to Figure 22, dynamic monitoring of the interaction of the indicated cytotoxic agents leads to the generation of characteristic kinetic patterns that is dependent on the cellular background, the concentration of the drug, the duration of exposure and the mechanism of drug action. Since each compound has its own characteristic pattern, these kinetic traces could potentially be used to determine the mechanism of action of compounds with unknown targets by comparing the kinetic profile to the profile of compounds with known mechanism of action.

Label-free and dynamic monitoring of cell proliferation, viability and cytotoxicity using the RT-CES system offers very distinct and important advantages over traditional endpoint assays. It allows for built in internal quality control to assure consistency and reproducibility between the different assays. Dynamic monitoring allows for observation of the entire episode of drug interaction with target cells and the user can therefore have a better understanding of the mode and mechanism of drug interaction. Furthermore, the actual kinetic trace of the drug interaction with the target cell is very significant because it can offer clues as to the mechanism of drug interaction with the target cell. Finally, since each compound or drug has its own characteristic profile with respect to its interaction with target cells, the RT-CES system can be used as a way to determine the mechanism of action of drugs with unknown targets.

**Table III.** Comparison of IC-50 values for Tamoxifen treatment of different cancer cell lines using the RT-CES system versus MTT assay. The indicated cell lines were seeded in ACEA 16X devices and monitored by RT-CES. Approximately 24 hours later, the cells were treated with increasing concentrations of Tamoxifen and then continually monitored by RT-CES. The experiment was stopped about 48 hours later and the cells in the 16X devices were assayed by using MTT. The IC-50 values derived from RT-CES system are time-dependent. In the table, the IC-50 values at about 48 hrs after compound treatment are shown for RT-CES system determination and MTT assay.

| Cell Type | RT-CES™ | MTT Assay |
| --- | --- | --- |
| HT1080 | 22.4 $\mu$M | 30.0 $\mu$M |
| NIH3T3 | 16.0 $\mu$M | 19.0$\mu$M |
| HepG2 | 15.2 $\mu$M | 16.2 $\mu$M |
| HUEVEC | 7.5 $\mu$M | 8.0 $\mu$M |

**Example 3: Measuring and monitoring the morphological changes occurring in Chinese hamster ovary (CHO) cells upon stimulation of the muscarinic M1 and M3 receptors**

As an example, we describe here the use of the ACEA RT-CES™ system to measure and monitor the morphological changes that occur as a result of carbacol treatment of Chinese hamster ovary (CHO) cells which express the muscarinic M1 and M3 receptor.

CHO cells expressing the muscarinic M1 receptor (CHO-M1) or the muscarinic M3 receptor (CHO-M3) were seeded in ACEA's 16X device (E-Plate) at 20,000 cells/well and the attachment and growth of the cells in the 37°C tissue culture incubator were monitored in real-time using RT-CES™ system (which includes, for example, a 16X device station or 16X E-Plate station, RT-CES impedance analyzer and integrated software for controlling the impedance analyzer and device station). Cell index curves measured on the RT-CES system are shown in Figure 23 and 24. After 22 hours the cells were treated with different doses of carbacol which is an agonist for both the M1 and M3 receptors. As a control, the cells were treated with media alone. The device chambers were returned to the incubator and recording was resumed. In addition, as a measure of specificity, the cells were also treated with carbacol in the presence of excess amount of pirenzipine, which is an antagonist of the M1 and M3 receptors.

The effect of carbacol on CHO-M1 cells and CHO-M3 cells in the presence or absence of pirenzipine is shown in Figure 23 and 24. The effect of carbacol is seen as a transient and abrupt shift in the trace of the cell index number at 22 hours, immediately after treatment with different doses of carbacol. The duration of the signal for the carbacol-mediated affect lasts approximately 4 hours and then returns to baseline. The vehicle control did not have any effect on the recording. However, as expected, pirenzipine blocked the effect of carbacol on both CHO-M1 and CHO-M3 cells indicating that the effect of carbacol is indeed mediated through the M1 and M3 receptors, respectively. Furthermore, carbacol and pirenzipine did not have any effect on CHO-K cells which do not express the M1 and M3 muscarinic receptors (Figure 25), indicating that the response is dependent on the expression of the muscarinic M1 or M3 receptors.

In addition CHO-M1 cells were stimulated with increasing concentration of carbacol and the cell-electrode impedance response was continually monitored by RT-CES system. As shown in Figure 26, the cell-electrode impedance response is dependent on the concentration of carbacol which is maximal at 31.25 nM. This experiment shows that the RT-CES system can be used to assess the effect of GPCR stimulation on cells and furthermore, it illustrates that this assay can be used to screen for antagonist of specific GPCRs. The assay can be performed in high-throughput format, in real time and without the need for any other reagents or cellular manipulation.

**Example 4: Monitoring dose-dependent functional activation of the Muscarinic M1 and M3 receptors in Chinese hamster ovary (CHO) cells and pharmacological characterization by a selective M1 agonist**

M1 and M3 muscarinic receptors are linked to a modulation of cell function via Gq class of heterotrimeric G-proteins. We used CHO cells stably expressing the M1 and M3 muscarinic receptors to test their responses to carbachol, a non-hydrolysable analogue of acetylcholine. The cells were continuously monitored using the RT-CES system. At the indicated time point, increasing concentrations of carbachol were added to the cells and the cell response was monitored every 3 min. As shown in Figure 27, carbachol ranging from 15 nM to 11 uM leads to a concentration-dependent increase in cell index in M 1 cells (A) and M3 cells (C). The dynamic of the cell responses demonstrates that maximal cell responses were achieved one hour after the application of carbachol and the cell index gradually decrease to lower levels. Plotting the peak cell index response versus the corresponding log concentration allows for calculation of the $EC_{50}$ values of carbachol for M1 cells (B) and M3 cells (D). Both the M1 and M3 muscarinic receptors are coupled to Gq subclass of $G\alpha$ proteins so that their response patters on the RT-CES system are highly similar. To examine the pharmacology of carbachol on M1 cells, we started with a selective M1 antagonist, pirenzepine, to confirm the specificity of carbachol. As shown in Figure 27 (D), pirenzepine attenuates carbachol-induced cell responses in a concentration-dependent manner with IC50 of 0.7 uM. These results indicate that the effects of carbachol observed are caused by M1 receptors overexpressed in the host cell line.

*B. Monitoring Dose-Dependent Functional Activation Of The Muscarinic M3 And M2 Receptors Expressed In RBL-2H3 Cell Line*

Parallely, we compared two distinct muscarinic receptors which preferentially interact with specific G proteins. In general, odd numbered muscarinic receptors interact with $\alpha$ subunit of Gq class while even numbered muscarinic receptors are coupled to $\alpha$ subunit of Gi class. In turn, activation of Gq stimulates phospolipase C and activation Gi decreases cAMP production. Clearly as indicated in Figure 28, activation of M3 (A) and M2 (C) by carbachol reveals two unique profiles of cell index dynamics. Similar but not identical to M3 expressed in CHO cell line (Figure 27C.), M3 expressed in RBL-2H3 cell line demonstrates that maximal cell responses were achieved one hour after the application of carbachol and the cell index stay high for a relative longer period of time. On the contrary, M2 expressed in RBL-2H3 cell line reveals a complete different pattern to carbachol application. Cell Index increase sharply and reached the maximal levels with 1-2 hours and then quickly tapered off and the cell index stay lower close to basal levels afterwards.

*C. Monitoring Dose-Dependent Functional Activation Of The Human Histamine H1 Receptor (hH1]-C1) Stably Expresses In CHO Cell Line*

Another category of GPCR coupled to Gq has been investigated. We choose cell line overexpress human recombinant H1 receptors (hH1-C1) to substantiate what have been found in our previous described results. hH1-C1 cells were tested by various concentrations of histamine. As shown in Figure 29, histamine produce concentration-dependent increases in cell index and the $EC_{50}$ of histamine is 1.7 nM (B). Brompheniramine (a selective H1 antagonist) completely blocks histamine-induced CI increases with IC50 of 12 nM indicating that histamine-induced CI increases in hH1-C1 cells were specifically mediated by H1 receptors.

*D. Monitoring Dose-Dependent Functional Activation Of The Human Vasopressin Receptor 1a (V1a-C1) Stably Expressed In 1321-N1 Cells And Characterization Of Pharmacology*

Vasopressin 1a receptor (V1a) together with V1b and V2, are a member of a family of related GPCRs which are activated by neurohypophysial peptide hormones including vasopressin (AVP). V1a is coupled to Gq and activation of the receptors leads to PLC activation and mediates a plethora response to AVP. We tested the V1a-C1 cell lines in RT-CSE system for the purpose of characterization of pharmacology. We used the natural ligand of V1a, namely AVP, to monitor the change of CI as the read-outs of functional activation of the V1a receptors. We tested various concentrations AVP ranging from 1 pM to 10 uM. The traces for concentrations between 30 pM and 0.3 uM are shown in Figure 30A. AVP-induced CI increases were transient in nature and CI gradually approached to basal levels after 5-6 hour of AVP application. Maximal effects of each tested-concentration were use to generate a concentration-dependent curve shown in Figure 30B and $EC_{50}$ of AVP is 155 pM. [β-Mercapto- β, β -cyclopentamethylene-propionyl1, O-Et-Tyr$^2$, Val$^4$, Arg$^8$] Vasopressin (βM AVP) was used a blocker for AVP. The IC50 of βM AVP is 761 nM.

**Example 5: Monitoring the functional activation of GPCRs expressed endogenously in a variety of cell lines**

*A. Using The RT-CES System To Monitor The Functional Activation Of Endogenous Histamine Receptor In HeLa Cells And Characterization Of Pharmacology.*

HeLa cells were tested by application of histamine to monitor the activation of the endogenous histamine receptors. As demonstrated in Figure 31A, B, histamine produced concentration-dependent increases of CI and the EC50 of histamine is 146 nM. The effects of histamine can be attenuated by brompheniramine with $IC_{50}$ of 406 nM (Figure 31C, D).

*B. Using The RT-CES System To Monitor The Functional Activation Of Endogenous Endothelin Receptor In Hela Cells*

HeLa cells were tested by application of endothelin 1 to monitor the activation of the endogenous endothelin receptors. As demonstrated in Figure 32, endothelin 1 produced concentration-dependent increases of CI and the EC50 of endothelin 1 is 0.9 nM.

*C. Using The RT-CES System To Monitor The Functional Activation Of Endogenous B-Adrenergic Receptors In C6 Glioma*

C6 cells were tested by application of isoproterenol, a β-adrenergic agonist. Unlike activation of other category of G protein subunits, activation of β-adrenergic receptors produced the dynamics of decreased CI. Isoproterenol produced a concentration-dependent and transient decrease of CI (Figure 33A) with EC50 of 24 nM (Figure 33B). The effects of isoproterenol can be blocked by β-adrenergic receptors antagonist alprenolol (Figure 33C) with an IC50 of 120 nM (Figure 33D) indicating the involvement of β-adrenergic receptors for this phenomenon. To substantiate the involvement of Gs, we take the advantage of dibutyl cAMP, a cell membrane permeable analogue of cAMP (Figure 33E, F), and forskolin (Figure 33G, H), an adenylate cyclase activator; both mimic the increase of intracellular cAMP as the consequence of Gs activation. Both compounds demonstrated the profiles of decreased CI supporting the results of isoproterenol.

*D. Using The RT-CES System To Monitor The Functional Activation Of The Calcitonin Receptor In CHO-K1 Cell*

Another pair of ligand-receptor coupled to Gs, calcitonin on CHO cells, has been tested by RT-CES system. In contrast to the activation of Gs in C6 cells, activation of Gs in CHO by calcitonin generated the increased CI. As shown (Figure 34 A, B), the effects of calcitonin is concentration-dependent with $EC_{50}$ of 0.385 nM.

**Example 6: Monitoring the GPCR activation pathway using the RT-CES™ system**

*A. Activation Of GPCRs in hH1-C1 Cells And V1a-C1 Cells By Their Respective Ligands Cause Membrane Ruffling And Focal Adhesion Assembly*

As the cell-electrode impedance reading is primarily associated with number, shape and strength of the adhesion of the cells on the surface of electrodes, we utilized there are indeed a morphological changes of the cells after the application of the ligand, which are associated with the changes of CI we observed in RT-CES system. We choose hH1-C1 and V1a-C1 cells stimulated by their respective ligands. After the addition of the ligand, f-actin was visualized by FTIC-phalloidin and a focal adhesion protein paxillin was co-stained by its antibody. As shown in Figure 35, after a brief exposure to histamine (100 nM, 5 min), membrane ruffling as indicated by phalloidin and increased focal adhesion assembly reflected by paxillin become apparent (arrows in lower panels of Figure 35). The similar phenomenon was found in V1a-C1 cells stimulated by AVP (arrows in lower panels of Figure 36).

*B. Utilization Of Various Signal Transduction Blockers To Dissect The Mechanisms Of Histamine-Induced CI Changes In hH1-C1 Cells.*

In order to fully understand the intracellular events after activation of H1 receptors, various pathway blockers or toxins were applied to the hH1-C1 cells before the addition of histamine. The CI was continuously monitored by RT-CES system. We demonstrated that protein kinas C inhibitor, bisindolylmalemide (10 uM) completely inhibits the effects of histamine (Figure 37 A). Src inhibitor, PP2 delays and attenuates the effects of histamine the effects histamine (Figure 37 B) implying that Src pathway might be involved in GPCRs mediated intracellular events. Cell adhesion blocker latrunculin completely eliminate the effects of histamine stressed the importance of cell adhesion in response to H1 activation by histamine (Figure 37 C). Rho kinase inhibitor ROCK partially reduced the magnitude of CI increases by histamine and slightly delayed the effects of histamine. histamine (Figure 37 D). MEK inhibitor PD98059 also delayed the effects of histamine but has little inhibition for the magnitude of histamine. histamine (Figure 37 E). PLC inhibitor U73122 significantly delayed onset and reduced the magnitude by histamine (Figure 37 F).

**Example 7: Application Of RT-CES System For Ranking The Potency Of Antagonists And For Screening Inverse Agonists**

*A. Using RT-CES System To Rank The Potencies Of Antagonists: Potential Application Of Compound Screening*

In order to further explore the possibilities of compound screening by RT-CES system, we used the hH1-C1/histamine pair to compare the potencies of a panel of selective H1 antagonists. As shown in Figure 38, RT-CES system can readily rank the potencies of the antagonists. The order of potency ranking is loratidine>mirtazepine>triprolidine= mepyramine. Tiotidine, a selective H2 antagonist was used a negative control.

*B. Using RT-CES System To Discovery Potential Inverse Agonists*

One of the new applications of RT-CES system described in this application is its potential use for inverse agonist characterization. Recent literatures have revealed that some of the antagonist has the intrinsic activities of inverse agonist. We demonstrated here that application of selective H1 antagonist alone produced a CI profile opposite to what agonist generated; namely, agonist (histamine) increases the CI while inverse agonist decreases the CI. The RT-CES system can also rank the potencies of a panel of inverse agonists. As shown in Figure 39, the order of potency ranking as the inverse agonists is loratidine>mirtazepine>triprolidine> mepyramine. Tiotidine, a selective H2 antagonist does not have the activities of inverse agonist.

**Example 8: Dynamic monitoring of G-protein couple receptor (GPCR) in living cells using the RT-CES system**

This example further expands on the utilization of the RT-CES technology to develop a label-free and real-time cell-based assay for monitoring functional activation of GPCRs in living cells. We demonstrate that this assay can be used with both engineered cell lines expressing recombinant GPCRs coupled to different G-proteins and more importantly with cell lines expressing endogenous levels of GPCRs. In addition, the non-invasive nature of the RT-CES readout makes it especially attractive as a tool for GPCR screening and research because multiple stimulations with the same agonist/antagonist or different agonist/antagonist in different combinations can be carried out in the same well. Also, the dynamic nature of the cell response to a particular agonist provides high content information regarding the signaling pathways being activated and can also be used to screen for antagonists and inverse agonists.

*A. Materials and Methods*

*Cell Culture*. All the cells used in this study were purchased from ATCC unless indicated otherwise. The cells are cultured in a standard humidified incubator at 37° C with 5% $CO_2$. C6 cells and HeLa cells were maintained in Dulbecco's modified Eagle's medium (DMEM) supplemented with 10% fetal calf serum, 2mM glutamine and 1% penicillin/ streptomycin. Chinese hamster ovary (CHO) cells were maintain in Ham's F12 medium supplemented with 10% fetal calf serum, 2mM glutamine and 1% penicillin/streptomycin. Cell line stably expressing human recombinant histamine receptor 1 (H1), human vasopressin 1a ($V_{1a}$) and human recombinant dopamine receptor 1 (D1) were obtained form Euroscreen and maintained in Ham's F12 medium supplemented with 10% fetal calf serum, 2mM glutamine, 1% penicillin/streptomycin and 400 ug/mL G418. Cell line stably expressing human recombinant 5-hydroxytryptamine receptor 1A (5-HT1A) was obtained form Euroscreen and maintained in Ultra-CHO medium supplemented with 1% penicillin/streptomycin and 400 ug/mL G418.

*Reagents.* All the reagents were purchased form Sigma (St. Louis, MO) unless indicated otherwise Loratidine, mirtazepine, mepyramine, triprolidine, tiotidine were purchased form Tocris (Ellisville, MO)

*RT-CES Measurement of Cell-Electrode Impedance.* The detailed experimental procedures have been described previously (Abassi et al, . Label-free, real-time monitoring of IgE-mediated mast cell activation on microelectronic cell sensor arrays, in J. of Immunological Methods. Vol: 292, pp 195-205, 2004; Solly et al., Application of real-time cell electronic sensing (RT-CES) technology to cell-based assays, in Assay and Drug Development Technologies. Vol: 2, pp 363-372, 2004.) Briefly, 50 $\mu$L of selective medium was added to wells of ACEA's 16x E-plates to obtain background readings followed by the addition of 150 $\mu$L of cell suspension containing the indicated number of cells. The E-plate containing the cells were allowed to incubate at room temperature for 10 minutes prior to being placed on the device station in the 37°C $CO_2$ incubator for continuous recording of impedance as reflected by Cell Index (CI). The cells were allowed to attach and spread typically for 6-8 h to reach a stable base line before the addition of agonists. Typically, 5 $\mu$L of 40X stock solution of agonist was gently added to the well and recording was resumed. For pharmacological and mechanistic studies, antagonists or inhibitors were added to the cells 5-10 min prior to the agonist application. The results were expressed by normalized CI, unless indicated otherwise, which are derived from the ratio of CIs before and after the addition of the compounds. For concentration-dependent study, maximal response (usually 1-2 h after the ligand application) of a given concentration of the compound was used to plot the concentration-dependent curve and $EC_{50}$ or $IC_{50}$ were calculated by Prism (San Diego, CA). *Fluorescence Microscopy*. H1 cells and V1a cells were seeded in 16-well Lab-tec chamber slides and allowed to attach and spread for 24 h. The cells were stimulated by 100 nM histamine for 5 min and washed 3 times with PBS before fixation. The cells were fixed in 4% paraformaldehyde and permeabilized in PBS containing 0.2% Triton X 100. After washing, the fixed cells were blocked by PBS containing 0.5% BSA. The cells were stained with phalloidin conjugated with FTIC and a monoclonal anti-paxillin antibody from Sigma (St. Louis, MO). The cells were washed 3 times with PBS and visualized and imaged by using a Nikon E400 epifluorescence microscope and Nikon ACT software.

*B. Dynamic Monitoring of GPCR Activation in Living Cells by RT-CES System.*

The RT-CES system is composed of a 16X or 96X device station which fits inside the tissue culture incubator, an electronic analyzer and a computer which runs the software and operates the entire system. At the core of the system are 16X and 96X electronic plates (E-plates™) with integrated microelectrodes in the bottom of the wells. Adherent cells are cultured on the surface of the sensors and the presence or absence of cells sensitively and precisely affects the electronic and ionic passage between cell culture media and the microelectrodes. Thus, interrogating the electrode impedance provides succinct information about the biological status of the cells such as proliferation, morphological changes and cell death.

In order to determine if the RT-CES system can be used to dynamically monitor the functional activation of GPCRs, CHO-K1 cells expressing the human H1 histamine receptor (H1) and 1321-N1 cells expressing the human vasopressin receptor (V1a) were seeded on the E-plates and stimulated with histamine and vasopressin, respectively (Figure 40). Both histamine and vasopressin induced an immediate and transient increase in CI (Figure 40A and 40C). The maximal response for H1 cells was at 40 min after histamine addition while for V1a cells it was at 90 min (Figure 40A and C). Both histamine and vasopressin act through Gq and have also been shown to modulate the actin cytoskeleton and its regulatory proteins such as focal adhesion kinase (FAK) and paxillin. In order to determine if histamine and vasopressin lead to modulation of the actin cytoskeleton and its signaling proteins, H1 cells and V1a cells were stimulated with histamine and vasopressin, respectively, fixed, stained with FITC-Phalloidin and anti-paxillin mAb (Figure 40B). Histamine treatment of H1 cells lead to an immediate (5 min) induction of membrane ruffles and translocation of paxillin to the site of membrane ruffles which is indicative of active actin remodeling (Figure 40B). Similarly, vasopressin also induced formation of membrane ruffles and translocation of paxillin to these sites (Figure 40D). In summary, the RT-CES system can detect the functional activation of GPCR based on its ability to modulate the actin cytoskeleton and cell adhesion.

C. *Dynamic and Quantitative Monitoring of Recombinant GPCRs Coupled to Different Signaling Pathways.*

In order to further extend the findings discussed above, H1 cells were seeded in E-plates, treated with increasing concentration of histamine and continually monitored by RT-CES system. As depicted in Figure 41A, histamine leads to a transient and dose-dependent increase in CI. To obtain a dose-response curve the log concentration of histamine was plotted against the normalized CI and the approximate $EC_{50}$ value obtained from this plot was 1.7nM. Similarly, vasopressin causes a transient and dose-dependent increase in CI (Figure 41C) with $EC_{50}$ of 155pM (Figure 41D). To determine if the functional activation of GPCRs coupled to other signaling pathways such as Gs or Gi can also be monitored on RT-CES system, CHO-K1 cells expressing the human dopamine 1 receptor (D1) coupled to Gs, or the human 5HT1A receptor (5-HT1A) coupled to Gi, were seeded on E-plates and stimulated with increasing concentrations of their respective ligands. As shown in Figure 42A and 42C, both cell types are activated in a dose-dependent manner. To generate a dose-response curve, the peak normalized CI value was plotted versus the log concentration of the agonist. Stimulation of D1 receptor by dopamine gave an $EC_{50}$ value of 0.79pM (Figure 42B), while 5-HT1A receptor by 5-HT stimulation gave an $EC_{50}$ value of 12.4nM (Figure 42D). In summary, the RT-CES system can detect the functional activation of GPCRs coupled to the different signaling pathways, which traditionally requires different detection technologies and instrumentation such as calcium or IP3 measurement (Gq) and cAMP measurements (Gs and Gi).

D. *Dynamic Monitoring of Endogenously Expressed GPCR in Living Cells.*

One of the challenges encountered by current label-based assays such as calcium measurements is that the cells have to be engineered to express promiscuous G proteins coupled to the calcium pathway. Alternatively, receptors may need to be overexpressed in order to obtain sufficient measurable signal. Once again all these artificial manipulations may ease the burden of screening large sets of compounds; however, physiological relevance of the findings will need to be supplemented with other experiments. The number of assays available to measure the functional activation of endogenous GPCRs in their natural and physiologically relevant settings is limited. We wanted to determine if RT-CES system can monitor the functional activation of endogenous GPCRs coupled to different signaling pathways. HeLa cells were seeded in E-plates and stimulated with increasing concentrations of histamine. As shown in Figure 43A, histamine stimulation of HeLa cells leads to a dose-dependent and transient increase in cell index. The kinetics and amplitude of histamine-mediated response in HeLa cells are significantly different from those of H1 cells. Primarily, this can be explained by cell type specificity in terms of histamine signaling. Also, other histamine receptors in addition to H1 may be present in HeLa cells which can contribute to the amplitude and duration of the response. The calcitonin receptor is coupled to the Gs pathway. To determine if endogenous activation of calcitonin receptor can be detected by the RT-CES system, CHO-K1 cells were seeded on E-plates and stimulated with the indicated concentrations of calcitonin (Figure 43C). As shown in Figure 43C, calcitonin leads to a robust and transient increase in CI and plotting normalized CI versus log concentration of the ligand gives a dose-response curve with an $EC_{50}$ of 0.385nM (Figure 43D). On the contrary, $\beta 1$ adrenergic receptor stimulation in C6 cells by isoproterenol reveals an opposite changes of CI. As shown in Figure 43E, isoproterenol generated a dose-dependent decrease of CI with an $EC_{50}$ of 24nM (Figure 43F). In summary, it has been demonstrated that the RT-CES system can monitor the functional activation of GPCRs coupled to different signaling pathways for both endogenous receptors and recombinant receptors.

E. *Quantitative Analysis and Ranking of Histamine Receptor Antagonists and Inverse Agonists.*

In order to validate the GPCR assay using the RT-CES system we chose a panel of well-characterized and selective histamine antagonists to test and rank their potency in H1 cells stimulated with histamine. H1 cells were seeded in E-plates, pre-incubated with the indicated concentrations of selective H1 antagonists or H2 receptor antagonists as a control and then stimulated with a fixed concentration of histamine (20nM). The peak normalized CI values were plotted versus the log concentrations of the antagonists. H1 receptor antagonist triprolidine, loratidine, mirtazepine and mepyramine led to a dose-dependent inhibition of histamine-mediated cellular response (previously shown as Figure 38) with $IC_{50s}$ of 9nM, 24uM, 343nM and 9nM respectively while H2 selective antagonist tiotidine did not inhibit histamine-induced cell response at all the indicated concentrations tested. These results strongly indicate that RT-CES system can be used for the purpose of potency ranking of antagonists in a receptor subfamily specific manner. Interestingly, the H1 receptor antagonists alone gave rise to dose-dependent decreases of CI levels (previously shown as Figure 39). This observation can be explained by the fact that all these H1 receptor antagonists have certain degrees of activities as inverse agonist (Fitzsimons et al., Mepyramine, a histamine H1 receptor agonist, binds preferentially to a G protein-coupled form of the receptor and sequesters G protein, in The Journal of Biochemical Chemistry, Vol: 279, pp 34431-34439, 2004), which impacts the basal activities of H1 receptor. Furthermore, selective H2 receptor antagonist tiotidine did not seem to affect histamine-mediated activation of the H1 cells nor did it have activity as inverse agonist indicating that the responses are specific. In summary, these results demonstrate that the RT-CES system can be used to screen selective antagonists or inverse agonists of GPCRs. Furthermore, the basal CI displayed prior to agonist addition is an actual reflection of the basal and cumulative signaling taking place inside the cell and any agent such as inverse agonists that perturb the basal signaling activity can be detected by the RT-CES system.

We have shown in this example that the RT-CES system can be used for functional screening of GPCR activity in cell-based assays. The main features which distinguish the RT-CES based assay described here from other cell-based functional assays for GPCRS are: (i) no pre- or post-labeling of the cells are necessary, saving expensive reagents and time; (ii) the readout is non-invasive and therefore cellular destruction is not required, allowing for multiple manipulations on the same cells in the same well, (iii) real-time kinetic readout which provides succinct and high content information regarding the pathways being activated, (iv) no compound interference with the detection method which can be a major problem in most optical-based assays and (v) since the readout monitors cell attachment and cell morphology which are integral components of cell viability, any compound that may be potentially cytotoxic or may have other adverse effects can be detected. Finally, the RT-CES system provides novel readouts for GPCR functions in living cells and provides another vantage point to increase our understanding of GPCR functions.

## Example 9: Dynamic monitoring of COS-7 cells during stimulation or inhibiton of a RTK

The RT-CES™ system was used to dynamically monitor the inhibition and/or stimulation of a Receptor Tyrosine Kinase (RTK). Furthermore the RT-CES™ system allowed the determination of the EC50 and IC50 of the RTK.

*Cell Culture and Reagents.* COS7 cells were acquired from ATCC. They were maintained in DMEM supplemented with 10% fetal bovine serum and incubated at 37 deg with 5% $CO_2$. From the time the cells were plated through the experimental process, cells were continually monitored with the RT-CES system. Cells were plated on sensor plates at $1X10^4$ cells per well and incubated overnight. During the day of assay, cells were serum starved in DMEM supplemented with 0.25% BSA for a total of 4 hours. If treated with inhibitors, cells were preincubated with inhibitors during the last hour of serum starvation and stimulated with growth factors. Inhibitors (Calbiochem) and LOPAC enzyme inhibitor ligand set (Sigma) were resuspended and stored according to manufacturers instructions.

*ELISA.* Cells were plated on sensor plates at $1X10^4$ cells per well and incubated overnight. During the day of assay, cells were serum starved in DMEM supplemented with 0.25% BSA for a total of 4 hours. If pretreated with inhibitors, cells were preincubated with inhibitors during the last hour of serum starvation and then stimulated with growth factor for 15 minutes. After growth factor stimulation, cells were washed 2 times with cold PBS and lysed. ELISA (Biosource) assay was performed to detect total EGFR and phospho-EGFR (1068) and read at 450 nm.

*Statistical and Data Analysis.* All dose response curves were generated by plotting the average of % control +/- standard deviation *versus* ligand or inhibitor concentrations. The average % control was calculated relative to samples treated with growth factor alone minus inhibitor of quadriplicate samples. The $EC_{50}$ for ligands and $IC_{50}$ for inhibitors were determined from fitted curve generated by XL*fit* 4.0.

*Result and Discussion.* Cells plated on the ACEA RT-CES sensor plates (E-plates™) were continuously monitored from the time of plating to the end of the experiment. This allows for continuous monitoring of cells and assay conditions for quality control before and during the time of experimentation. $1X10^4$ COS7 cells plated on ACEA RT-CES sensor plates were serum starved for a total of 4 hours and stimulated with 25 $\eta$g/mL EGF or insulin and monitored every minute from the time of ligand addition. Ligand addition results in a rapid and transient increase in cell index for both EGF and insulin treated cells (Figure 44A). This increase was immediately followed by a decrease in cell index, with EGF showing a faster rate of decrease in cell index over time compared to insulin. The transient increase in cell index is a result of cytoskeletal rearrangements as a result of growth factor treatment. It is well documented that one of the intracellular signals RTKs activate are cytoskeletal changes resulting in membrane ruffling, lamellipodia and filopodia formation. To characterize the specificity of these responses to ligand treatment, cells were pretreated for one hour with 10 $\mu$M of the EGFR inhibitor (EGFRI), 4557W, prior to addition of EGF or insulin. Since the inhibitor is specific to EGFR, application of the EGFRI should only affect cellular changes induced by EGF treatment. Indeed, after ligand addition, the transient increase in cell index was only detected on cells treated with insulin and not EGF (Figure 44B). The absence of cell response in EGF treated cells was a result of the specific inhibition of EGFR and its signaling pathways by the EGFRI. The specificity of this inhibitor and ligand response is demonstrated by the lack of effect on the transient increase in cell index of insulin treated cells.

To further characterize this cellular response, a wide range of EGF and insulin concentrations were used to determine the ligand $EC_{50}$ (Figure 45A and 45B). The cell index was measured for each concentration every minute over several hours. Cells treated with low concentration of the ligand showed transient small changes in peak cell index, while an increasing concentration of ligand resulted in an increase in the amplitude of the peak of the cell index. The magnitude of the cell index was directly related to the concentration of ligand used and reached a saturable response. From these trace the maximum cell index for each ligand concentration were determined and the % control relative to the response of the sample treated with the maximum ligand concentration calculated and plotted *versus* ligand concentrations. From the fitted curves the EGF and insulin $EC_{50}$ were calculated to be 0.95 ng/mL and 8.5 ng/mL, respectively. An important consideration of the assay is to show that these results are consistent with other RTK assays. To compare the $EC_{50}$ values derived from ACEA RT-CES with a well-established assay used to monitor RTK activity, ELISA assays were performed to detect phosphorylated EGFR on COS7 cells treated with varying concentrations of EGF (Figure 45C). From the fitted curve an $EC_{50}$ value of 2.6 ng/mL was calculated. This value was comparable to $EC_{50}$ values determined using the ACEA RT-CES demonstrating the use of this system as an alternative or complementary assay to existing RTK assays.

To determine the appropriate conditions to initiate a screen for EGFR inhibitors using the ACEA RT-CES system, a few parameters were optimized to maximize the signal to noise and the quality of the data. First, the optimum concentration of cells needed to achieve the maximum amount of signal was determined (Figure 46A). A range of COS7 cells were plated and tested for response to EGF. The maximum peak in cell index as a result of EGF treatment was observed to increase with increasing number of cells plated. However, this response reached a critical cell density wherein further increase in cell number resulted in a decrease in cell index. This decrease is thought to be due to the absence of available space between the cells preventing the lateral expansion cell membrane over the sensors during ligand mediated cytoskeletal rearrangement. Second, it is also necessary to titrate the ligand concentration to determine the maximum amount of ligand needed to produce the highest signal and appropriate ligand concentration for the type of assay used (Figure 46A). Having maximized these conditions, the statistical parameter in terms of Z' factor and S/N value of the assay was determined and evaluated to assess the quality of the assay (Figure 46B). The Z'factor is a statistical characteristic for evaluating the assay quality. The Z' factor calculated showed a value of 0.6, which is above the acceptable limit for a robust and consistent assay, and the S/N value of 38.

In order to validate this assay a screen was conducted of a diverse collection of small molecule inhibitors from Sigma (Figure 47A). This library was supplemented to include a specific EGFRI. The library was arrayed in a 96-well format and contains several wells of full activity (positive) and zero activity (negative) reference controls. Sigma's inhibitor ligand set was screened at a single concentration between 5-10 $\mu$M. Maximum cell index as a result of EGF treatment was determined for each inhibitor treated samples and the % control relative to the positive reference, EGF treated cell without inhibitor, was calculated. Using 60 % (or 40% inhibition) as the cutoff criteria for hit consideration, the screening study identified a single potent inhibitor. This inhibitor was the EGFRI, 4557W, which was added to the library. The assay was also tested against a collection of kinase inhibitors and similarly identified only the EGFRI to produce the most significant inhibition (Figure 47B). The inhibitor was further characterized and a dose response curve generated (Figure 47C). From the fitted curve an IC50 of 161 $\eta$M was calculated. This set of experiments demonstrates the ability of ACEA RT-CES system to identify a potent and selective inhibitor from a diverse library and chemically focused kinase library, and also to further characterize identified hits.

In addition to insulin and EGF, this cellular response can also be detected with treatment of HGF, FGF and PDGF in different mammalian cancer cells (Figure 48). To explore the responses to these growth factors, several human cell lines were plated and treated with different growth factors. Cell lines such as A431 showed a robust response to EGF and HGF, while minimal response was observed to other growth factors.

In conclusion, these data demonstrate a facile and novel cell based assay for RTK activity and function. This assay quantifies morphological changes in response to growth factor treatment and therefore mimics proximal events in kinase activation. Unlike other RTK assay, this assay is cell-based, label-free and monitors cellular changes in real-time, therefore acquiring high content information regarding the state of the cell and the signaling pathways being activated. In addition, the RTK assay described here does not require expensive reagents nor suffer from assay component interference. Since the readout is non-invasive multiple treatments can be carried out in the same well and can also be used in conjunction with other existing cell-based assays for RTK. It requires very little optimization and user training, making this assay amenable for use in both primary and secondary screens.

**Example 10: Method to assess the functional contribution of receptor tyrosine kinase activity in cancer cell proliferation**

In addition to morphological changes, stimulation of receptor tyrosine kinases via their cognate ligand can induce cell growth and proliferation. It has been shown that stimulation of cells via growth factors such as EGF can induce signaling pathways which induces the entry of the cells into the cell cycle and proliferation. Therefore, cellular proliferation is another method to assess the functional role of growth factor receptor tyrosine kinases. The RT-CES system can be readily used to monitor cellular proliferation, especially those of breast and lung cancer, where receptor tyrosine kinases have been shown to play an important role.

As an example we describe the monitoring of MCF-7 cancer cell proliferation and its inhibition by a general tyrosine kinase inhibitor, staurosporine. MCF-7 cells were seeded at a density of 8000 cells per well of ACEA e-plates. Cell proliferation was monitored using the RT-CES system for 48 hours at which point the cells were treated with either DMSO alone as a control or staurosporine at a final concentration of 2.5 $\mu$M (Figure 49). The figure illustrates that the RT-CES system can be used to monitor the proliferation of cancer cells and furthermore, it can be used to assess the effect of tyrosine kinase inhibitors, which does affect EGF receptor as well as other tyrosine kinases on the proliferation of cancer cells. This method allows real-time monitoring of the effect of receptor tyrosine kinase inhibitors using the ACEA RT-CES system.

References

1. Nambi, P and Aiyar N, 2003, G-protein coupled receptors in drug discovery, Assay and Drug Development Technologies Vol: 1, pp 305-310

2. Roberto E. Favoni and Alessandra De Cupis 2000, The role of polypeptide growth factors in human carcinomas: new targets for a novel pharmacological approach, Pharmacological Reviews, Vol:52, pp 179-205.

3. El-Rayes BF and LoRusso PM, 2004, Targeting the epidermal growth factor receptor, British Journal of Cancer, Vol: 91, pp: 418-424.

4. Abassi Y.A., Jackson J.A., Zhu J., O'Connell J., Wang X., Xu X 2004, Label-free, real-time monitoring of IgE-mediated mast cell activation on microelectronic cell sensor arrays. Journal of Immunological Methods. Vol: 292, pp195-205.

5. Solly K., Wang X., Xu X., Struloviki B., Zheng W. 2004. Application of real-time cell electronic sensing (RT-CES) technology to cell-based assays, Assay and Drug Development Technologies. Vol: 2, pp 363-372.

6. Fitzsimons C.P., Monczor F., Fernández N., Shayo C., Davio C. 2004, Mepyramine, a histamine H1 receptor agonist, binds preferentially to a G protein-coupled form of the receptor and sequesters G protein, The Journal of Biochemical Chemisty. Vol: 279, pp 34431-34439.

**Claims**

1. A method of identifying a compound capable of interacting with a G-Protein Coupled Receptor (GPCR) comprising:

   a) providing a device capable of measuring cell-substrate impedance; wherein said device is in the format of a multi-well plate, each well comprising a nonconductive substrate over which is fabricated a conductive electrode array, further wherein said device is operably connected to an impedance analyzer;
   b) adding test cells to at least two wells for attachment to said electrode array, wherein said test cells express a GPCR;
   c) determining first cell indices from first impedances, wherein said first impedances are cell-substrate impedances measured from said at least two wells immediately preceding step d), wherein the cell indices positively correlate with the number of viable cells in the wells, how well they are attached to the electrode surfaces, and the cell/electrode contact area due to the cell morphology status;
   d) adding a compound to at least one well containing said test cells to form at least one compound well and adding a vehicle control to at least another well containing test cells to form at least one control well;
   e) determining second cell indices from said second impedances, wherein said second impedances are cell-substrate impedances measured from said at least one compound well and said at least one control well after step d), wherein the cell indices positively correlate with the number of viable cells in the wells, how well they are attached to the electrode surfaces, and the cell/electrode contact area due to the cell morphology status;
   f) determining the change in cell index for said at least one compound well by comparing said second cell index of said at least one compound well to said first cell index of said at least one compound well , and determining the change in the impedance or cell index of said at least one control well by comparing said second cell index of said at least one control well to said first cell index of said at least one control well,
   g) comparing said change in cell index between said at least one compound well and said at least one control well; and
   h) identifying said compound interacts with said GPCR if said comparison demonstrates a significant difference between said change in cell index of said at least one compound well and said change in cell index of said at least one control well.

2. The method according to claim 1, wherein said compound is added to at least two wells and in at least two different concentrations.

3. The method according to claim 1, wherein said compound is added to at least two wells in at least two different concentrations forming at least two compound wells, further wherein a dose curve is determined by plotting a maximum change in cell index for each concentration of said compound versus the corresponding concentration or determining the area under the cell-index curve for the different concentrations of the compound and plotting the area under the cell-index curve or impedance curve for each of the compound concentrations versus the corresponding concentration.

4. A method of identifying a compound capable of interacting with a G-Protein Coupled Receptor (GPCR) comprising:

   a) providing a device capable of measuring cell-substrate impedance; wherein said device is in the format of a multi-well plate, each well comprising a nonconductive substrate over which is fabricated a conductive array, further wherein said device is operably connected to an impedance analyzer;
   b) adding test cells to at least one of said at least two wells for attachment to said conductive array to form at least one test well, and adding control cells to at least another well for attachment to said conductive array to form at least one control well, wherein said test cells express a GPCR and said control cells do not express said GPCR or express said GPCR at a significantly lesser level that said test cells;
   c) determining first cell indices from first impedances, wherein said first impedances are cell-substrate impedances measured from said at least one test well and from said at least one control well immediately preceding step d), wherein the cell indices positively correlate with the number of viable cells in the wells, how well they are attached to the electrode surfaces, and the cell/electrode contact area due to the cell morphology status;

d) adding a compound to said at least one test well and to said at least one control well;

e) determining second cell indices from said second impedances, wherein said second impedances are cell-substrate impedances measured from said at least one test well and from said at least one control well after step d), wherein the cell indices positively correlate with the number of viable cells in the wells, how well they are attached to the electrode surfaces, and the cell/electrode contact area due to the cell morphology status;

f) determining the change in cell index of said at least one test well by comparing said second cell index of said at least one test well to said first cell index of the said at least one test well, and determining the change in cell index of said at least one control well by comparing said second cell index of said at least one control well to said first cell index of the said at least one control well;

g) comparing said changes in cell index between said at least one test well and said at least one control well; and

h) identifying said compound interacts with said GPCR if said comparison demonstrates a significant difference between said change in cell index for said at least one test well and said change in cell index for said at least one control well.

5. The method accordig to claim 1 or 4, wherein said multi-well plate is selected from the group consisting of a 16 well plate, a 24 well plate, a 96 well plate, a 384 well plate, and a 1536 well plate.

6. The method according to claim 1 or 4, wherein said cell substrate impedance measures a characteristic of the cytoskeleton of a cell.

7. The method according to claim 1 or 4, wherein said GPCR is selected from the group consisting of a recombinant GPCR, an endogenous GPCR, an orphan GPCR, a constitutively active GPCR, and chimeric GPCR, or other chimeric receptor containing GPCR property.

8. The method according to claim 1 or 4, wherein said cell index is a normalized cell index wherein normalization is at a time point a short time before adding said compound or said vehicle control, wherein the said short time is selected from the group consisting of less than 1 minute, less than 2 minutes, less than 5 minutes, less than 10 minutes, less than 30 minutes, less than 1 hour, less than 2 hours, less than 5 hours, less than 10 hours and less than 24 hours.

9. The method according to claim 1 or 4, wherein each of said first impedances are a series of impedance measurements prior to said adding said compound or said vehicle control and each of said first cell indices are a series of cell indices, wherein each of said first cell indices corresponds to a distinct impedance measurement within said series of impedance measurements.

10. A method according to claim 1 or 4, wherein said measuring said first impedances occurs at a time selected from the group consisting of less than 1 minute, less than 5 minutes, less than 30 minutes, less than 1 hour, less 2 hours, less than 5 hours, less than 10 hours, and less than 24 hours prior to said adding said compound or said vehicle control.

11. The method according to claim 1 or 4, wherein said compound is obtained from a library of compounds.

12. The method according to claim 1 or 4, wherein said compound is selected from a group consisting of a ligand, an agonist, and an inverse agonist.

13. The method according to claim 1 or 4, wherein each of said second impedances are a series of impedance measurements after said adding said compound or said vehicle control and each of said second cell indices are a series of cell indices, wherein each of said second cell indices correspond to a distinct impedance measurement within said series of impedance measurements.

14. A method according to claim 1 or 4, wherein said second impedances are measured at a time point selected from the group consisting of more than 1 minute, more than 5 minutes, more than 30 minutes, more than 1 hour, more than 2 hours, more than 5 hours, more than 10 hours, and more than 24 hours after said adding said compound or said vehicle control.

15. The method according to claim 1 or 4, wherein said changes in cell indices are absolute changes in cell indices.

16. The method according to claim 1 or 4, wherein said changes in impedances or cell indices are relative changes in impedances or cell indices.

**17.** The method according to claim 1 or 4, wherein said compound is identified as an agonist if said comparison indicates a significant change comprising an increase or a decrease cell index for said compound well after said compound is added, or as an inverse agonist if said comparison indicates a significant change in impedance comprising a decrease or increase in cell index for said compound well after said compound is added and the significant change in impedance or cell index for the compound well is in the opposite direction compared to the change of a known agonist.

**18.** The method according to claim 17, further comprising determining an EC50 of said compound, wherein said EC50 is a concentration of a compound capable of inducing 50% of maximum response in cell index or 50% of maximum change in area under the cell-index curve.

**Patentansprüche**

**1.** Verfahren zur Identifizierung einer Verbindung, die mit einem G-Protein gekoppeltem Rezeptor (GPCR) interagieren kann, umfassend :

a) Bereitstellen eines Geräts, das Zell-Substrat-Impedanz messen kann; wobei das Gerät die Form einer Platte mit mehreren Vertiefungen aufweist, wobei jede Vertiefung ein nicht leitendes Substrat umfasst, über dem ein Bündel leitender Elektroden gefertigt ist, wobei das Gerät ferner betriebsmäßig mit einem Impedanz-Analanalysegerät verbunden ist,
b) Zufügen von Testzellen in mindestens zwei Vertiefungen zur Anbindung an das Elektronenbündel, wobei die Testzellen einen GPCR ausdrücken;
c) Bestimmung erster Zellindizes von ersten Impedanzen, wobei die ersten Impedanzen Zell-Substrat-Impedanzen sind, die von den mindestens zwei Vertiefungen direkt vor Schritt d) gemessen werden, wobei die Zellindizes positiv mit der Zahl der lebensfähigen Zellen in den Vertiefungen, wie gut sie an die Elektrodenoberflächen angebunden sind, und dem Zellen/Elektroden-Kontaktbereich abhängig vom Zellmorphologiezustand korrelieren;
d) Zufügen einer Verbindung in zumindest eine Vertiefung, die die Testzellen enthält, um mindestens eine Verbindungsvertiefung zu bilden, und Zufügen eines Kontrollträgers in mindestens eine andere Vertiefung, die die Testzellen enthält, um mindestens eine Kontrollvertiefung zu bilden;
e) Bestimmung zweiter Zellindizes von zweiten Impedanzen, wobei die zweiten Impedanzen Zell-Substrat-Impedanzen sind, die von der mindestens einen Verbindungsvertiefung und der mindestens einen Kontrollvertiefung nach Schritt d) gemessen werden, wobei die Zellindizes positiv mit der Zahl der lebensfähigen Zellen in den Vertiefungen, wie gut sie an die Elektrodenoberflächen angebunden sind, und dem Zellen/Elektroden-Kontaktbereich abhängig vom Zellmorphologiezustand korrelieren;
f) Bestimmung der Veränderung des Zellindexes für die mindestens eine Zusammensetzungsvertiefung durch Vergleich des zweiten Zellindexes der mindestens einen Verbindungsvertiefung mit dem ersten Zellindex der mindestens einen Zusammensetzungsvertiefung und Bestimmung der Veränderung der Impedanz oder des Zellindexes der mindestens einen Kontrollvertiefung durch Vergleich mit dem zweiten Zellindex der mindestens einen Kontrollvertiefung mit dem ersten Zellindex der mindestens einen Kontrollvertiefung;
g) Vergleich der Veränderung des Zellindexes zwischen der mindestens einen Verbindungsvertiefung und der mindestens einen Kontrollvertiefung; und
h) Identifizierung der Interaktion der Verbindung mit dem GPCR, wenn der Vergleich einen signifikanten Unterschied zwischen der Veränderung des Zellindexes der mindestens einen Verbindungsvertiefung und der Veränderung des Zellindexes der mindestens einen Kontrollvertiefung ergibt.

**2.** Verfahren nach Anspruch 1, wobei die Verbindung zu mindestens zwei Vertiefungen und in mindestens zwei unterschiedlichen Konzentrationen zugegeben wird.

**3.** Verfahren nach Anspruch 1, wobei die Verbindung in mindestens zwei Vertiefungen in mindestens zwei unterschiedlichen Konzentrationen, die mindestens zwei Verbindungsvertiefungen bilden, zugegeben wird, wobei ferner eine Dosiskurve durch Auswertung einer Maximalveränderung des Zellindexes für jede Konzentration der Zusammensetzung im Vergleich zur entsprechenden Konzentration bestimmt wird oder Bestimmen des Bereichs unter der Zellindexkurve für die unterschiedlichen Konzentrationen der Zusammensetzung und Auswerten des Bereichs unter der Zellindexkurve oder der Impedanzkurve für jede der Zusammensetzungskonzentrationen im Vergleich zur entsprechenden Konzentration.

4. Verfahren zum Identifizieren einer Verbindung, die mit einem G-Protein gekuppelten Rezeptor (GPCR) interagiert, umfassend :

a) Bereitstellen eines Geräts, das die Impedanz eines Zellsubstrats messen kann; wobei das Gerät die Form einer Platte mit mehreren Vertiefungen aufweist, wobei jede Vertiefung ein nicht leitendes Substrat umfasst, über dem ein Bündel leitender Elektroden gefertigt ist, wobei das Gerät ferner betriebsmäßig mit einem Impedanz-Analanalysegerät verbunden ist,

b) Zufügen von Testzellen in mindestens zwei Vertiefungen zur Anbindung an das leitende Bündel, um mindestens eine Testvertiefung zu bilden, und Zufügen von Kontrollzellen in die mindestens eine andere Vertiefung zur Anbindung an das leitende Bündel, um mindestens eine Kontrollvertiefung zu bilden, wobei die Testzellen einen GPCR ausdrücken und die Kontrollzellen keinen GPCR ausdrücken, bzw. ihn auf einer signifikant niedrigeren Ebene als die Testzellen ausdrücken;

c) Bestimmung erster Zellindizes von ersten Impedanzen, wobei die ersten Impedanzen Zell-Substrat-Impedanzen sind, die von den mindestens einer Testvertiefung und mindestens einer Kontrollvertiefung direkt vor Schritt d) gemessen werden, wobei die Zellindizes positiv mit der Zahl der lebensfähigen Zellen in den Vertiefungen, wie gut sie an die Elektrodenoberflächen angebunden sind, und dem Zellen/Elektroden-Kontaktbereich abhängig vom Zellmorphologiezustand korrelieren;

d) Zufügen der Verbindung in die mindestens eine Testvertiefung und die mindestens eine Kontrollvertiefung;

e) Bestimmung zweiter Zellindizes von zweiten Impedanzen, wobei die zweiten Impedanzen Zell-Substrat-Impedanzen sind, die von der mindestens einen Testvertiefung und von der mindestens einen Kontrollvertiefung nach Schritt d) gemessen werden, wobei die Zellindizes positiv mit der Zahl der lebensfähigen Zellen in den Vertiefungen, wie gut sie an die Elektrodenoberflächen angebunden sind, und dem Zell//Elektroden-Kontaktbereich abhängig vom Zellmorphologiezustand korrelieren;

f) Vergleich der Veränderung des Zellindexes in der mindestens einen Testvertiefung durch Vergleich des zweiten Zellindexes der mindestens einen Testvertiefung mit dem ersten Zellindex der mindestens einen Testvertiefung und Bestimmen der Veränderung des Zellindexes der mindestens einen Kontrollvertiefung durch Vergleich des zweiten Zellindexes der mindestens einen Kontrollvertiefung mit dem ersten Zellindex der mindestens einen Kontrollvertiefung;

g) Vergleich der Veränderungen des Zellindexes zwischen der mindestens einen Testvertiefung und der mindestens einen Kontrollvertiefung; und

h) Identifizierung der Interaktion der Verbindung mit dem GPCR, wenn der Vergleich einen signifikanten Unterschied zwischen der Veränderung des Zellindexes der mindestens einen Testvertiefung und der Veränderung des Zellindexes der mindestens einen Kontrollvertiefung ergibt.

5. Verfahren nach Anspruch 1 oder 4, wobei die Vertiefungsplatte au seiner Gruppe gewählt wird, die aus einer Platten mit 16 Vertiefungen, einer Platte mit 24 Vertiefungen, einer Platte mit 96 Vertiefungen, einer Platte mit 384 Vertiefungen und einer Platte mit 1536 Vertiefungen besteht.

6. Verfahren nach Anspruch 1 oder 4, wobei die Zell-Substrat-Impedanz ein Merkmal des Zytoskeletts einer Zelle misst.

7. Verfahren nach Anspruch 1 oder 4, wobei GPCR au seiner Gruppe gewählt wird, die aus einem rekombinanten GPCR, einem endogenen GPCR, einem seltenen GPCR, einem konstitutionell aktiven GPCR, einem chimären GPCR, oder einem anderen chimären Rezeptor, der GPCR-Merkmale enthält, besteht.

8. Verfahren nach Anspruch 1 oder 4, wobei der Zellindex ein normierter Zellindex ist, wobei die Normierung zu einem Zeitpunkt stattfindet, der kurz vor dem Zufügen der Verbindung oder des Kontrollträgers liegt, wobei ein kurzer Zeitpunkt aus der Gruppe gewählt wird, die aus weniger als seiner Minute, weniger als zwei Minuten, weniger als 10 Minuten, weniger als 30 Minuten, weniger als 1 Stunde, weniger als 2 Stunden, weniger als 5 Stunden, weniger als 10 Stunden und weniger als 24 Stunden besteht.

9. Verfahren nach Anspruch 1 oder 4, wobei jede der ersten Impedanzen eine Reihe von Impedanzmessungen vor dem Zufügen der Verbindung oder des Kontrollträgers und jeder der ersten Zellindexe eine Reihe von Zellindexen sind, wobei jeder der ersten Zellindexe einer bestimmten Impedanzmessung innerhalb einer Reihe von Impedanzmessungen entspricht.

10. Verfahren nach Anspruch 1 oder 4, wobei das Messen der ersten Impedanzen zu einem Zeitpunkt stattfindet, die aus der Gruppe gewählt ist, die aus weniger als 1 Minute, weniger als 5 Minuten, weniger als 30 Minuten, weniger als 1 Stunde, weniger als 2 Stunden, weniger als 5 Stunden, weniger als 10 Stunden und weniger als 24 Stunden vor dem Zufügen der Verbindung oder des Kontrollträgers besteht.

11. Verfahren nach Anspruch 1 oder 4, wobei die Verbindung aus einer Verbindungsbibliothek stammt.

12. Verfahren nach Anspruch 1 oder 4, wobei die Verbindung au seiner Gruppe gewählt wird, die aus einem Liganden, einem Agonisten und einem inversen Agonisten besteht.

13. Verfahren nach Anspruch 1 oder 4, wobei jede der zweiten Impedanzen eine Serie von Impedanzmessungen nach dem Zufügen der Verbindung oder des Kontrollträgers ist und jeder der zweiten Zellindexe eine Serie von Zellindexen sind, wobei jeder der zweiten Zellindexe einer bestimmten Impedanzmessung innerhalb einer Reihe von Impedanzmessungen entspricht.

14. Verfahren nach Anspruch 1 oder 4, wobei die zweiten Impedanzen zu einem Zeitpunkt gemessen werden, der aus der Gruppe gewählt ist, die aus mehr als 1 Minute, mehr als 5 Minuten, mehr als 30 Minuten, mehr als 1 Stunde, mehr als 2 Stunden, mehr als 5 Stunden, mehr als 10 Stunden und mehr als 24 Stunden nach dem Zufügen der Verbindung oder des Kontrollträgers besteht.

15. Verfahren nach Anspruch 1 oder 4, wobei die Veränderungen der Zellindexe absolute Veränderungen der Zellindexe sind.

16. Verfahren nach Anspruch 1 oder 4, wobei die Veränderungen der Impedanzen oder der Zellindexe relative Veränderungen der Impedanzen oder der Zellindexe sind.

17. Verfahren nach Anspruch 1 oder 4, wobei die Verbindung als ein Agonist identifiziert ist, wenn der Vergleich eine signifikante Veränderung umfassend eine Erhöhung oder Verminderung des Zellindexes für die Verbindungsvertiefung nach dem Zufügen der Verbindung ergibt, oder als ein inverser Agonist, wenn der Vergleich eine signifikante Veränderung umfassend eine Erhöhung oder Verminderung der Impedanz des Zellindexes für die Verbindungsvertiefung nach dem Zufügen der Verbindung ergibt und die signifikante Veränderung der Impedanz oder des Zellindexes der Verbindungsvertiefung in die umgekehrte Richtung verläuft, wie die Veränderung eines bekannten Agonisten.

18. Verfahren nach Anspruch 17, ferner umfassend die Bestimmung eines EC50 der Verbindung, wobei EC50 eine Konzentration einer Verbindung ist, die eine Maximalantwort des Zellindexes oder 50% der Maximalveränderung in dem Bereich unter der Zellindexkurve induzieren kann.

**Revendications**

1. Un procédé d'identification d'un composé capable d'interagir avec un récepteur couplé aux protéines G (GPCR) comprenant :

   a) la fourniture d'un dispositif capable de mesurer l'impédance cellule-substrat ; dans lequel ledit dispositif est sous la forme d'une plaque multi-puits, chaque puits comprenant un substrat non conducteur sur lequel est fabriqué un réseau d'électrodes conductrices, en outre dans lequel ledit dispositif est connecté de manière opérationnelle à l'analyseur d'impédance ;
   b) l'ajout de cellule test à au moins deux puits pour la fixation audit réseau d'électrodes, dans lequel lesdites cellules test expriment un GPCR ;
   c) la détermination de premiers indices de cellule à partir des premières impédances, dans lequel lesdites premières impédances sont des impédances cellule-substrat mesurées à partir desdits au moins deux puits immédiatement avant l'étape d), dans lequel les indices de cellule corrèlent positivement avec le nombre de cellule viables dans les puits, dans quelle mesure elles sont fixées aux surfaces d'électrodes, et de la surface de contact cellule/électrode en raison du statut de la morphologie de cellule ;
   d) l'ajout d'un composé à au moins un puits contenant lesdites cellules test pour former au moins un puits de composé et l'ajout d'un véhicule contrôle à au moins un autre puits contenant les cellules test pour former au moins un puits contrôle ;
   e) la détermination des seconds indices de cellule à partir desdites secondes impédances, dans lequel lesdites secondes impédances sont des impédances cellule-substrat mesurées à partir dudit au moins un puits de composé et dudit au moins un puits control après l'étape d), dans lequel les indices de cellule corrèlent positivement avec le nombre de cellule viables dans les puits, dans quelle mesure elles sont fixées aux surfaces d'électrodes, et de la surface de contact cellule/électrode en raison du statut de la morphologie de cellule ;

f) la détermination de la modification d'indice de cellule dudit au moins un puits de composé en comparant ledit second indice de cellule dudit au moins un puits de composé audit premier indice de cellule dudit au moins un puits de composé, et la détermination de la modification d'impédance ou d'indice de cellule dudit au moins un puits contrôle en comparant ledit second indice de cellule dudit au moins un puits contrôle audit premier indice de cellule dudit au moins un puits contrôle ;

g) la comparaison desdites modifications d'indice de cellule entre ledit au moins un puits de composé et ledit au moins un puits contrôle ; et

h) l'identification dudit composé interagissant avec ledit GPCR si ladite comparaison démontre une différence significative entre ladite modification d'indice de cellule dudit au moins un puits de composé et ladite modification d'indice de cellule dudit au moins un puits contrôle.

2. Le procédé selon la revendication 1, dans lequel ledit composé est ajouté à au moins deux puits et dans au moins deux concentrations différentes.

3. Le procédé selon la revendication 1, dans lequel ledit composé est ajouté à au moins deux puits dans au moins deux concentrations différentes formant au moins deux puits de composé, en outre dans lequel la courbe de dose est déterminée en représentant une modification maximale d'indice de cellule pour chaque concentration dudit composé par rapport à la concentration correspondante ou la détermination de l'aire sous la courbe cellule-substrat pour les différentes concentrations du composé et en représentant l'aire sous la courbe cellule-substrat ou la courbe d'impédance pour chacune des concentrations de composé par rapport à la concentration correspondante.

4. Un procédé d'identification d'un composé capable d'interagir avec un récepteur couplé aux protéines G (GPCR) comprenant :

a) la fourniture d'un dispositif capable de mesurer l'impédance cellule-substrat ; dans lequel ledit dispositif est sous la forme d'une plaque multi-puits, chaque puits comprenant un substrat non conducteur sur lequel est fabriqué un réseau conducteur, en outre dans lequel ledit dispositif est connecté de manière opérationnelle à l'analyseur d'impédance ;

b) l'ajout de cellule test à au moins l'un desdits au moins deux puits pour la fixation audit réseau conducteur pour former au moins un puits test, et l'ajout de cellule contrôle à au moins un autre puits pour la fixation audit réseau conducteur pour former au moins un puits contrôle, dans lequel ladite cellule test exprime un GPCR et lesdites cellules contrôle n'expriment pas ledit GPCR ou expriment ledit GPCR à un niveau significativement moindre que lesdites cellules test ;

c) la détermination de premiers indices de cellule à partir des premières impédances, dans lequel lesdites première impédances sont des impédances cellule-substrat mesurées à partir dudit au moins un puits test et à partir dudit au moins un puits contrôle immédiatement avant l'étape d), dans lequel les indices de cellule corrèlent positivement avec le nombre de cellule viables dans les puits, dans quelle mesure elles sont fixées aux surfaces d'électrodes, et de la surface de contact cellule/électrode en raison du statut de la morphologie de cellule ;

d) l'ajout d'un composé audit au moins un puits test et audit au moins un puits contrôle ;

e) la détermination des seconds indices de cellule à partir desdites secondes impédances, dans lequel lesdites secondes impédances sont des impédances cellule-substrat mesurées à partir dudit au moins un puits test et à partir dudit au moins un puits control après l'étape d), dans lequel les indices de cellule corrèlent positivement avec le nombre de cellule viables dans les puits, dans quelle mesure elles sont fixées aux surfaces d'électrodes, et de la surface de contact cellule/électrode en raison du statut de la morphologie de cellule ;

f) la détermination de la modification d'indice de cellule dudit au moins un puits test en comparant ledit second indice de cellule dudit au moins un puits test audit premier indice de cellule dudit au moins un puits test, et la détermination de la modification d'indice de cellule dudit au moins un puits contrôle en comparant ledit second indice de cellule dudit au moins un puits contrôle audit premier indice de cellule dudit au moins un puits contrôle ;

g) la comparaison desdites modifications d'indice de cellule entre ledit au moins un puits test et ledit au moins un puits contrôle ; et

h) l'identification dudit composé interagissant avec ledit GPCR si ladite comparaison démontre une différence significative entre ladite modification d'indice de cellule pour ledit au moins un puits test et la modification d'indice de cellule pour ledit au moins un puits contrôle.

5. Le procédé selon la revendication 1 ou 4, dans lequel ladite plaque multi-puits est choisie dans le groupe consistant en une plaque 16 puits, une plaque 24 puits, une plaque 96 puits, une plaque 384 puits, et une plaque 1536 puits.

6. Le procédé selon la revendication 1 ou 4, dans lequel ladite impédance cellule-substrat mesure une caractéristique du cytosquelette d'une cellule.

7. Le procédé selon la revendication 1 ou 4, dans lequel ledit GPCR est choisi dans le groupe consistant en un GPCR recombinant, un GPCR endogène, un GPCR orphelin, un GPCR constitutivement actif, et un GPCR chimérique, ou un autre récepteur chimérique contenant la propriété de GPCR.

8. Le procédé selon la revendication 1 ou 4, dans lequel ledit indice de cellule est un indice de cellule normalisé dans lequel la normalisation est à un point de temps un court instant avant l'ajout dudit composé ou dudit véhicule contrôle, dans lequel ledit court instant est choisi dans le groupe consistant en moins de 1 minute, moins de 2 minutes, moins de 5 minutes, moins de 10 minutes, moins de 30 minutes, moins d'1 heure, moins de 2 heures, moins de 5 heures, moins de 10 heures et moins de 24 heures.

9. Le procédé selon la revendication 1 ou 4, dans lequel chacune desdites premières impédances est une série de mesures d'impédances avant ledit ajout dudit composé ou dudit véhicule contrôle et chacun desdits premiers indices de cellule est une série d'indices de cellule, dans lequel chacun desdits premiers indices de cellule correspond à une mesure d'impédance distincte à l'intérieur de ladite série de mesures d'impédances.

10. Un procédé selon la revendication 1 ou 4, dans lequel ladite mesure desdites impédances intervient à un moment choisi dans le groupe consistant en moins de 1 minute, moins de 5 minutes, moins de 30 minutes, moins d'1 heure, moins de 2 heures, moins de 5 heures, moins de 10 heures et moins de 24 heures avant ledit ajout dudit composé ou dudit véhicule contrôle.

11. Le procédé selon la revendication 1 ou 4, dans lequel ledit composé est obtenu à partir d'une librairie de composés.

12. Le procédé selon la revendication 1 ou 4, dans lequel ledit composé est choisi dans le groupe consistant en un ligand, un agoniste, et un agoniste inverse.

13. Le procédé selon la revendication 1 ou 4, dans lequel chacune desdites secondes impédances est une série de mesures d'impédance après ledit ajout dudit composé ou dudit véhicule contrôle et chacun desdits seconds indices de cellule est une série d'indices de cellule, dans lequel chacun desdits seconds indices de cellule correspond à une mesure d'impédance distincte à l'intérieur de ladite série de mesures d'impédance.

14. Un procédé selon la revendication 1 ou 4, dans lequel lesdites secondes impédances sont mesurées à un point de temps choisi dans le groupe consistant en plus de 1 minute, plus de 5 minutes, plus de 30 minutes, plus d'1 heure, plus de 2 heures, plus de 5 heures, plus de 10 heures et plus de 24 heures après ledit ajout dudit composé ou dudit véhicule contrôle.

15. Le procédé selon la revendication 1 ou 4, dans lequel lesdites modifications d'indices de cellule sont des modifications absolues d'indices de cellule.

16. Le procédé selon la revendication 1 ou 4, dans lequel lesdites modifications d'impédances et d'indices de cellule sont des modifications relatives d'impédances ou d'indices de cellule.

17. Le procédé selon la revendication 1 ou 4, dans lequel ledit composé est identifié comme un agoniste si ladite comparaison indique une modification significative comprenant une augmentation ou une diminution d'indice de cellule pour ledit puits de composé après l'ajout dudit composé, ou comme un agoniste inverse si ladite comparaison indique une modification significative d'impédance comprenant une diminution ou une augmentation d'indice de cellule pour ledit puits de composé après l'ajout dudit composé et que la modification significative d'impédance ou d'indice de cellule pour le puits de composé soit dans la direction opposée par rapport à la modification d'un agoniste connu.

18. Le procédé selon la revendication 17, comprenant en outre la détermination d'un EC50 dudit composé, dans lequel ledit EC50 est une concentration d'un composé capable d'induire 50% de réponse maximale de l'indice de cellule ou 50% de modification maximale dans l'aire sous la courbe cellule-indice.

Figure 1A

106
105

106

105

Figure 1B

Connection pad 1

Electrical trace 1

*1*

*B1*

*A1*

*A*

*B*

*A2*

*B2*

*2*

Electrical trace 2

Connection pad 2

*Figure 1C*

EP 1 773 979 B1

Figure 2.

EP 1 773 979 B1

**Figure 3.**

Figure 4.

**Figure 5.**

**Figure 6.**

Figure 7.

| Cell Number | 16000 | 8000 | 4000 | 2000 |
| Cell Index | 8.3 | 4.6 | 1.9 | 0.9 |

| Cell Number | 1000 | 500 | 250 | 125 |
| Cell Index | 0.51 | 0.3 | 0.22 | 0.13 |

**Figure 8.**

EP 1 773 979 B1

Figure 9A.

**Figure 9B.**

Figure 10A.

Figure 10B.

Figure 11A

Figure 11B.

Figure 12A

Figure 12.B

Figure 13A

Figure 13B.

Figure 14A

Figure 14 B.

Figure 15A

Figure 15B

Figure 15C

Figure 15D

Figure 16A.

EP 1 773 979 B1

Figure 16B.

# Shade-coding Scheme

CCI >> 0.7/DT:

CCI ~ 0.7/DT:

0< CCI < 0.7/DT:

CCI ~ 0:

CCI < 0:

CCI << 0:

Figure 16C

EP 1 773 979 B1

Figure 17.

Figure 18

A

B

Figure 19

95

A

B

**Figure 20**

EP 1 773 979 B1

A

B

Figure 21

Figure 22

Figure 23

Figure 24

**A** CHO-K Cells

treatment

- medium control
- 500 uM carbacol
- 125 uM carbacol
- 31.25 uM carbacol

**B** CHO-K Cells

treatment

- control
- 500 uM carbacol+pirenzepin
- 125 uM carbacol+pirenzepin
- 31.25 uM carbacol+pirenzepin

# Figure 25

Figure 26

Figure 27

Figure 28

Figure 29

**Figure 32**

**Figure 33 A-B**

Figure 33 C-H

**Figure 34**

**Figure 35**

Phalloidin          anti-Paxillin

Control

+ Vasopressin

Figure 36

Figure 37

Figure 38

Figure 39

Figure 40

Figure 41

Figure 42

Figure 43

Figure 44

EP 1 773 979 B1

Figure 45

EP 1 773 979 B1

**A**

**B**

| Z' | S/N | S/B | CV% |
|---|---|---|---|
| 0.6 | 38 | 1.5 | 3.8 |

**Figure 46**

Figure 47 A-B

C

D

**Figure 47C**

EP 1 773 979 B1

Figure 48A

Figure 48B

Figure 48C

126

Figure 49

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004010103 A
- WO 2004010102 A
- WO 2005077104 A
- WO 2005005979 A
- US 7470533 B
- US 7195752 B
- US 7192752 B
- US 7459303 B
- WO 2005047482 A
- US 7560269 B

### Non-patent literature cited in the description

- **NAMBI, P ; AIYAR N.** G-protein coupled receptors in drug discovery. *Assay and Drug Development Technologies,* 2003, vol. 1, 305-310
- **LUTTREL, LM.** *Mol Cell,* 2002, vol. 9, 1152-4
- **NAMBI, P ; NAMBI, A.** *Assay and Drug Development Technologies,* 2003, vol. 1, 305-310
- **ABASSI et al.** Label-free, real-time monitoring of IgE-mediated mast cell activation on microelectronic cell sensor arrays. *J. of Immunological Methods,* 2004, vol. 292, 195-205
- **SOLLY et al.** Application of real-time cell electronic sensing (RT-CES) technology to cell-based assays. *Assay and Drug Development Technologies,* 2004, vol. 2, 363-372
- **FITZSIMONS et al.** Mepyramine, a histamine H1 receptor agonist, binds preferentially to a G protein-coupled form of the receptor and sequesters G protein. *The Journal of Biochemical Chemistry,* 2004, vol. 279, 34431-34439
- **ROBERTO E. FAVONI ; ALESSANDRA DE CUPIS.** The role of polypeptide growth factors in human carcinomas: new targets for a novel pharmacological approach. *Pharmacological Reviews,* 2000, vol. 52, 179-205
- **EL-RAYES BF ; LORUSSO PM.** Targeting the epidermal growth factor receptor. *British Journal of Cancer,* 2004, vol. 91, 418-424
- **ABASSI Y.A. ; JACKSON J.A. ; ZHU J. ; O'CONNELL J. ; WANG X. ; XU X.** Label-free, real-time monitoring of IgE-mediated mast cell activation on microelectronic cell sensor arrays. *Journal of Immunological Methods,* 2004, vol. 292, 195-205
- **SOLLY K. ; WANG X ; XU X. ; STRULOVIKI B. ; ZHENG W.** Application of real-time cell electronic sensing (RT-CES) technology to cell-based assays. *Assay and Drug Development Technologies,* 2004, vol. 2, 363-372
- **FITZSIMONS C.P. ; MONCZOR F. ; FERNÁNDEZ N. ; SHAYO C. ; DAVIO C.** Mepyramine, a histamine H1 receptor agonist, binds preferentially to a G protein-coupled form of the receptor and sequesters G protein. *The Journal of Biochemical Chemisty,* 2004, vol. 279, 34431-34439